# EUROPEAN PATENT APPLICATION

(11) **EP 2 913 341 A1**
(43) Date of publication of application: **02.09.2015**
(21) Application number: 15155019.1
(22) Date of filing: 21.12.2007
(51) Int. Cl.: C07K 16/00, C12Q 1/68, A61K 39/395, G01N 33/53

(54) **Method of detecting ocular diseases and pathologic conditions and treatment of same**

(30) Priority: 22.12.2006 US 876709 P
(62) Divisional of application: 07872362.4
(71) Applicant: University of Utah Research Foundation, Salt Lake City, UT 84108 (US)
(72) Inventor: Zhang, Kang, Salt Lake City, UT 84124 (US); Yang, Zenglin, Salt Lake City, UT 84108 (US); Chen, Haoyu, Salt Lake city, UT 84108 (US); Li, Dean, Salt Lake City, UT 84108 (US)
(74) Representative: Denison, Christopher Marcus

(57) **Abstract**

Ocular diseases affecting the macula or the vasculature of the eye affect a wide variety of individuals. In particular, Age-related macular degeneration (AMD) is the most common cause of irreversible vision loss in the developed world and has a significant genetic predisposition. Methods of analyzing one or more mutations in the HtrA1 gene in order to identify individuals with a presusceptability to development of an ocular disease and a pathologic condition of the eye and diagnose those currently suffering from an ocular disease or a pathologic condition of the eye are provided. The methods of the present invention may further include analysis of the CFH gene in order to identify individuals with a presusceptability to development of an ocular disease and a pathologic condition of the eye and diagnose those currently suffering from an ocular disease or a pathologic condition of the eye.

Compositions and methods for treating ocular disease and pathologic conditions of the eye are also provided.

## Description

### STATEMENT OF FEDERAL SUPPORT

This invention was made with government support under grant number RO1EY14448 awarded by the National Institutes of Health. The Government has certain rights in this invention.

### SUMMARY

Variation in the human genome is correlated to a predisposition to ocular disease. In a particular embodiment, a variant of the HtrA1 gene is correlated herein with a predisposition to ocular disease. In one such embodiment, a genetic variation that leads to increased expression or upregulation of the HtrA1 protein is correlated to a predisposition to develop ocular disease. In another such embodiment, a variant of the HtrA1 gene is correlated to a predisposition to develop ocular disease, such as, for example, diabetic retinopathy (DR), retinopathy of prematurity (ROP), macular edema, and age related macular degeneration (AMD) in either or both of its dry or wet forms. In a particular embodiment, the variant of the HtrA1 gene is selected from single nucleotide polymorphism (SNP) rs11200638, rs1061170, rs10490924, rs3750848, rs3793917, rs932275, and combinations thereof.

In yet another embodiment, a variant of the complement factor H (CFH) gene is correlated herein with a predisposition to ocular disease. In one such embodiment, a genetic variation that leads to increased expression or upregulation of the CFH protein is correlated to a predisposition to develop ocular disease. In another such embodiment, a variant of the CFH gene is correlated to a predisposition to develop ocular disease, such as, for example, DR, ROP, macular edema, and AMD in either or both of its dry or wet forms. In a particular embodiment, the variant of the CFH gene is selected from rs1061170 (Y402H variant at 1q31 chromosome locus), rs529825, rs800292, rs1061147, rs203674, rs2274700, and combinations thereof.

Also described herein are methods for diagnosing or aiding in the diagnosis of ocular disease, and methods for identifying or aiding in the identification of subjects at risk for developing ocular disease. In one embodiment, such method includes taking a sample from a subject and identifying at least one genetic variation associated with ocular disease. In one such embodiment, identifying at least one genetic variation includes identifying a genetic variation selected from at least one variation in the HrtA1 gene, at least one variation in the CFH gene, and combinations thereof.

Methods of detecting, in a sample obtained from an subject, a genetic variant that is associated with ocular disease are also provided herein. In one such embodiment, the method includes detecting at least one genetic variant that is correlated with the occurrence and ocular disease, wherein the at least one genetic variant is selected from at least one variation in the HrtA1 gene, at least one variation in the CFH gene, and combinations thereof. In another embodiment, the method includes detecting a genetic variant selected from a variant of the HtrA1 gene, a variant of the CFH gene, and combinations thereof, that results in increased or upregulated protein expression. In yet another embodiment, the method includes detecting the presence of at least one SNP in a subject, wherein the SNP is selected from rs11200638, rs1061170, rs10490924, rs3750848, rs3793917, rs932275, rs529825, rs800292, rs1061147, rs203674, rs2274700 and combinations thereof and the presence of the at least one SNP is correlated with an ocular disease selected from DR, ROP, macular edema, and AMD in either or both of its dry or wet forms.

By way of example, a method for detecting a genetic variant associated with ocular disease is provided herein. In one embodiment, such a method may be a method for detecting at least one genetic variant associated with DR, ROP, macular edema, or AMD in either or both of its dry or wet forms, wherein the method includes, for example, (a) combining a sample obtained from a subject with one or more polynucleotide probes that hybridize, under stringent conditions, to the at least one genetic variation; and (b) determining whether hybridization occurs, wherein the occurrence of hybridization indicates that at least one genetic variant associated with AMD is present in the sample.

Isolated polynucleotides for the detection of genetic variants associated with ocular disease are also described herein. In one embodiment, an isolated polynucleotide for the detection of a variant HtrA1 gene is provided, wherein the isolated polynucleotide comprises a nucleic acid molecule that specifically detects a variation in the HtrA1 gene correlated with the occurrence of DR, ROP, macular edema, or AMD in either or both of its dry or wet forms. In one such embodiment, the isolated polynucleotide is useful for detecting a variant of the HtrA1 gene in a sample taken from a subject, wherein the variant is correlated with DR, ROP, macular edema, or AMD in either or both of its dry or wet forms. In another embodiment, the polynucleotide detects a SNP associated with increased expression or upregulation of HtrA1. In yet another embodiment, the polynucleotide probe detects SNP re11200638 near the promoter region of the HtrA1 gene. In another embodiment, an isolated polynucleotide for the detection of a variant CFH gene is provided, wherein the isolated polynucleotide comprises a nucleic acid molecule that specifically detects a variation in the CFH gene correlated with the occurrence of DR, ROP, macular edema, or AMD in either or both of its dry or wet forms. In one such embodiment, the isolated polynucleotide is useful for detecting a variant of the CFH gene in a sample taken from a subject, wherein the variant is correlated with DR, ROP, macular edema, or AMD in either or both of its dry or wet forms. In another embodiment, the polynucleotide detects a SNP associated with increased expression or upregulation of CFH. In yet another embodiment, the polynucleotide probe detects the SNP rs1061170 of the CFH gene at the 1q31 chromosome locus.

The polynucleotides described herein may further be used in allele-specific assays (e.g., allele-specific hybridization, primer extension, or ligation assays known in the art) to detect at least one variation in either or both of the HtrA1 gene and the CFH gene, wherein such at least one variation is correlated with the occurrence of DR, ROP, macular edema, or AMD in either or both of its dry or wet forms. Allele-specific probes and primers are able to specifically hybridize to one or more alleles of a gene and will not hybridize to other alleles of the same gene. For example, an allele-specific polynucleotide probe according to the present description may hybridize to a variant HtrA1 gene but will not hybridize to a wild type HtrA1 gene. Similarly, an allele-specific polynucleotide probe according to the present description may hybridize to a variant CFH gene but will not hybridize to a wild type CFH gene. In certain embodiments, an isolated polynucleotide according to the present description is a probe that hybridizes, under stringent conditions, to a variation selected from a variation in the HtrA1 gene, and a variation in the CFH gene, wherein the variation is correlated with the occurrence of ocular disease.

Pharmaceutical compositions for the treatment of ocular disease are also provided herein. In one embodiment, a pharmaceutical composition as provided herein includes at least one biologically active constituent capable of inhibiting or blocking one or more activities of a protein selected from HtrA1 and CFH. In another such embodiment, the pharmaceutical composition is for use in treating AMD in either or both of its wet or dry forms, DR, ROP or macular edema. In another such embodiment, the pharmaceutical composition is for use in treating choroidal or retinal neovascularization associated with or resulting from disease or injury. In another such embodiment, the pharmaceutical composition is for use in treating ischemia-induced retinal neovascularization resulting from vascular damage, occlusion or leak. In yet another such embodiment, the pharmaceutical composition is for use in treating a pathological condition associated with vascular leak or edema in the eye. In yet another such embodiment, the pharmaceutical composition is for use in treating a condition selected from retinal vein occlusion, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, and juxtafoveal telangiectas.

Methods for treating ocular disease are also provided herein. In one embodiment, the method includes inhibiting or blocking one or more activities of HtrA1, CFH, or both HtrA1 and CFH. In one such embodiment, a therapeutically effective amount of one or more biologically active agents capable of inhibiting or blocking one or more activities of HtrA1, CFH, or both HtrA1 and CFH is administered to a subject. For example, in one embodiment, one or more biologically active agents capable of inhibiting or blocking one or more activities of HtrA1, CFH, or both HtrA1 and CFH may be used to treat AMD in either or both of its wet or dry forms, DR, ROP or macular edema. In another embodiment, one or more biologically active agents capable of inhibiting or blocking one or more activities of HtrA1, CFH, or both HtrA1 and CFH may be used to treat choroidal or retinal neovascularization associated with or resulting from disease or injury. In another embodiment, one or more biologically active agents capable of inhibiting or blocking one or more activities of HtrA1, CFH, or both HtrA1 and CFH may be used to treat ischemia-induced retinal neovascularization resulting from vascular damage, occlusion or leak. In yet another embodiment, one or more biologically active agents capable of inhibiting or blocking one or more activities of HtrA1, CFH, or both HtrA1 and CFH may be used to treat a pathological condition associated with vascular leak or edema in the eye. In yet another such embodiment, the pharmaceutical composition is for use in treating a condition selected from retinal vein occlusion, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, and juxtafoveal telangiectas.

The methods described herein include administering a therapeutically-effective amount of one or more biologically active agents capable of inhibiting or blocking one or more activities of HtrA1, CFH, or both HtrA1 and CFH to a subject. Biologically active agents as contemplated herein include agents that reduce or inhibit transcription or translation of RNA material associated with expression of HtrA1, CFH, or both HtrA1. By way of example, and not limitation, biologically active agents suitable for use in the methods described herein may be selected from antibodies, serine protease inhibitors, nucleic acid molecules, such as RNAi compounds, molecules that inhibit or block HtrA1 protein binding to TGF-ß, a molecule that stimulates TGF-ß, small molecule compounds, and any combination thereof.

Antibodies to HtrA1 and methods of using such antibodies are described herein. The antibodies described herein are raised against HtrA1 and variants of HtrA1 as defined herein. The antibodies may be, for example, monoclonal antibodies, polyclonal antibodies, human antibodies, humanized antibodies, or affinity matured antibodies. In addition, where desired the antibodies described herein may be modified, such as by amino acid substitution, glycosylation modification, or by specific conjugation or formulation techniques as desired to achieve a targeted biological activity or functional characteristic. In particular examples, the antibodies described herein exhibit a minimum threshold reactivity with HtrA1 molecule, inhibit one or more activities of HtrA1, inhibit HtrA1 induced neovascularization, or exhibit any combination of such characteristics.

The antibodies disclosed herein are useful in treating disease or injury of the eye. In one embodiment, the antibodies disclosed herein may be used to treat retinal disease or injury. For example, in one embodiment, HtrA1 antibodies may be used to treat DR, ROP, macular edema, or AMD in either or both of its dry or wet forms. In another embodiment, HtrA1 antibodies may be used to treat choroidal or retinal neovascularization associated with or resulting from disease or injury. In another embodiment, the methods described herein include use of HtrA1 antibodies to treat ischemia-induced retinal neovascularization resulting from vascular damage, occlusion or leak. In yet another embodiment, the methods described herein include use of HtrA1 antibodies to treat a pathological condition associated with vascular leak or edema in the eye. In yet another embodiment, the methods described herein include use of HtrA1 antibodies to treat a condition selected from retinal vein occlusion, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, and juxtafoveal telangiectas. The methods described herein include administering a therapeutically-effective amount of one or more HtrA1 antibodies to a subject.

Pharmaceutical compositions of antibodies to HtrA1 are also described herein. The pharmaceutical compositions described herein include one or more biologically active agent in amounts sufficient to allow delivery of therapeutically-effective doses of the one or more biologically active agent to a subject. The pharmaceutical compositions described herein may include pharmaceutically acceptable excipients and may be formulated for delivery via any suitable route and means of administration. In one embodiment, a pharmaceutical composition for injection or infusion delivery of HtrA1 antibodies is provided.

Additional aspects and advantages will be apparent from the following detailed description, which proceeds with reference to the accompanying drawings.

### BRIEF DESCRIPTION OF THE DRAWINGS

FIG. 1 shows a fragment of human HtrA1 (or "hHtrA1) (SEQ. ID. NO: 1). The fragment is designated as "HU-142aa" and includes amino acid residues 142-480 of a full-length human HtrA1 (SEQ. ID. NO.: 2), which is designated as "HU-FULL."
FIG. 2 illustrates the results of a zymography analysis of purified HtrA1 proteins (left) and of an immunoblot analysis (right) of purified HtrA1 under nonreducing (-DTT) and reducing (+DTT) conditions.
FIG. 3 illustrates the results of an analysis of HtrA1 mRNA expression in AMD. Real-time RT-PCR semiquantitative analysis of HtrA1 RNA levels in blood lymphocytes from three AMD subjects with the AA genotype and three normal controls with the GG genotype was conducted. The statistical significance of the differences in expression level was examined using an independent samples t test (SPSS version 13.0): AA:GG (P = 0.02). Each RT-PCR reaction was run twice, and the error bars indicate the 95.0% confidence interval of the mean.
FIG. 4 shows the results of a western blot analysis of HtrA1 expression in human RPE. HtrA1 protein expression level in AA genotype is 1.7 fold compared to that of GG genotype normalized to β-actin. Significance was examined using an independent samples t-test (SPSS version 13.0): AA:GG (*p=0.049). The error bars indicate the standard deviation of the mean. Mean values are relative to GG expression. GG, n=6; AA, n=4.
FIG. 5(A) shows the results of a western blot analysis of HtrA1 polyclonal antibody. HtrA1 polyclonal antibody as produced herein recognizes recombinant HtrA1 (a) and HtrA1 in human RPE (c and d). Specificity was confirmed by lack of signal following preabsorption with recombinant HtrA1 protein (e and f), (b) was a negative control consisting of a recombinant protein preparation from bacteria transfected with vector only.
FIG. 5(B) provides an image showing HtrA1 polyclonal antibody stained on mammalian cells expressing recombinant human HtrA1.
FIG. 5(C) provides an image showing isolectin staining of retinal blood vessels.
FIG. 5(D) provides and image showing HtrA1 polyclonal antibody staining of retinal blood vessels.
FIG. 5(E) is a combination of the images of FIG. 5(C) and FIG. 5(D) with TO-PRO3 staining to show nuclei (blue), which provides an image demonstrating that HtrA1 polyclonal antibody stains pathologic endothelial cells located beyond internal limiting membrane (yellow/bottom arrows) but not intraretinal endothelial cells (white/top and middle arrows). As used in FIG. 5 (C-E), "ONL" refers to the outer nuclear layer, "INL" refers to the inner nuclear layer, "GCL" refers to the ganglion cell layer, and "ILM" refers to the internal limiting membrane.
FIG. 6(A) - FIG. 6(C) illustrate the results of immunohistochemistry studies of HtrA1 in AMD human donor eyes.

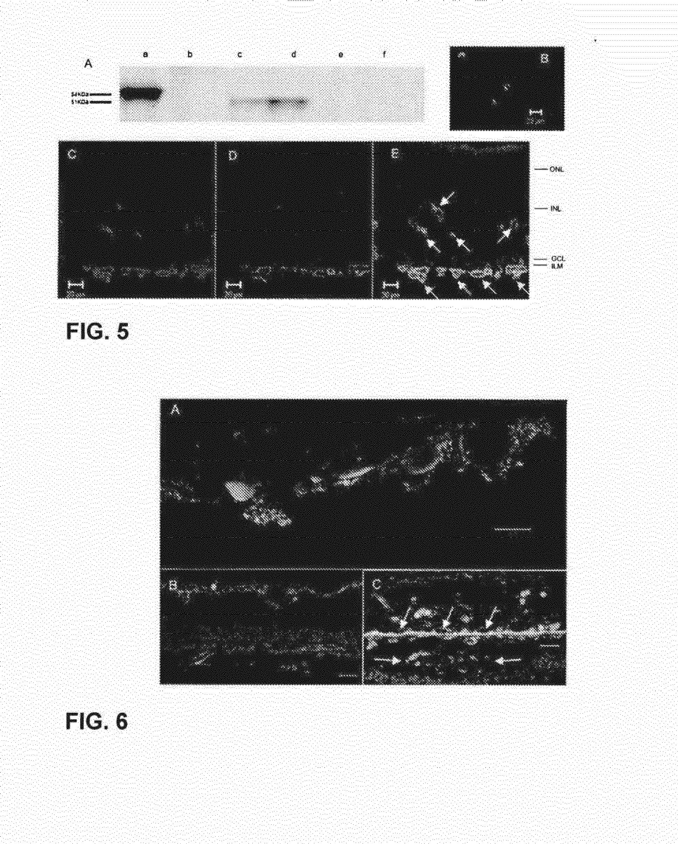

FIG. 6(A) provides an image showing HtrA1 nonuniformly expressed on drusen, the hallmark of age related macular degeneration, and Bruch's membrane.
FIG. 6(B) provides an images showing that HtrA1 is not only expressed on drusen, but also on retinal blood vessels, the retinal pigment epithelium (RPE) and internal limiting membrane.
FIG. 6(C) shows that, in a wet age related macular degeneration donor eye, HtrA1 is expressed on choroidal vessels (white/top arrows) and in choroidal neovascularization (yellow/bottom arrows). Green channel is the staining of HtrA1 polyclonal antibody. Nuclei were counter-stained with propidium iodine (red). The scale bar provided in the figure represents a 20 µm length.

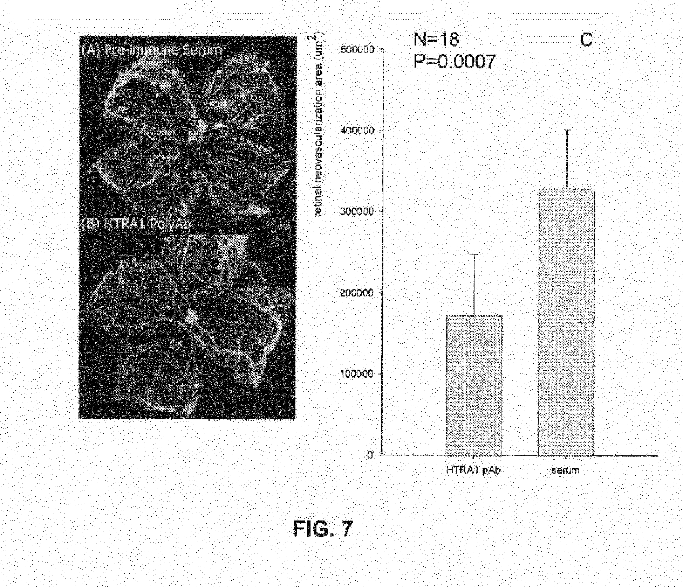

FIG. 7 illustrates the results of testing conducted in an OIR mouse model, wherein a polyclonal HtrA1 antibody and a negative control pre-immune serum were administered to the mice.

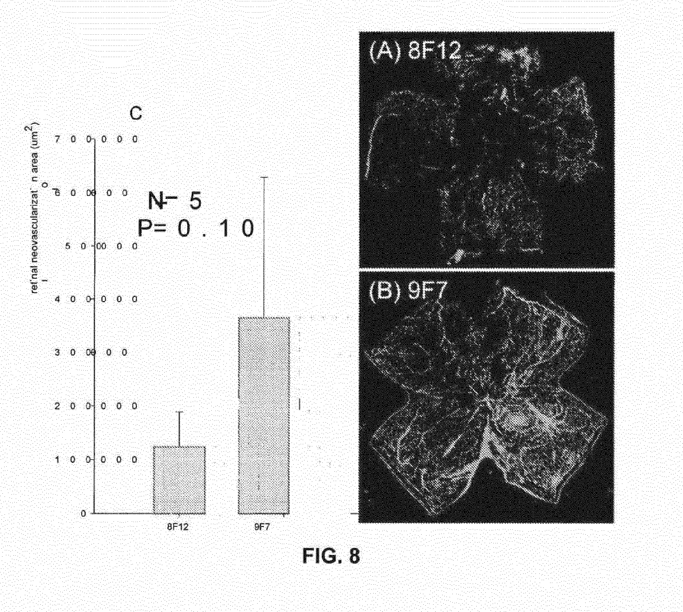

FIG. 8 illustrates the results of testing conducted in an OIR mouse model, wherein 8F12 HtrA1 monoclonal antibody and 9F7 HtrA1 monoclonal antibody were administered to the mice.

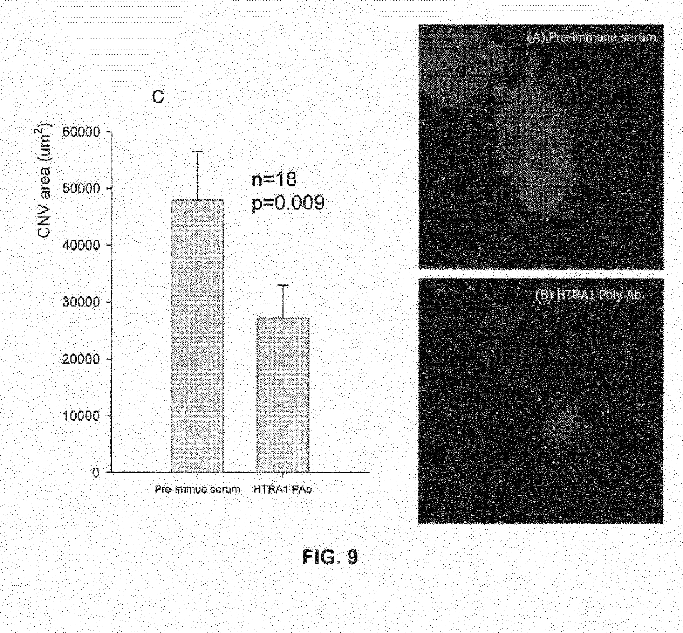

FIG. 9 illustrates the results of testing conducted in a CNV mouse model, wherein a polyclonal HtrA1 antibody and a negative control a pre-immune serum were administered to the mice.

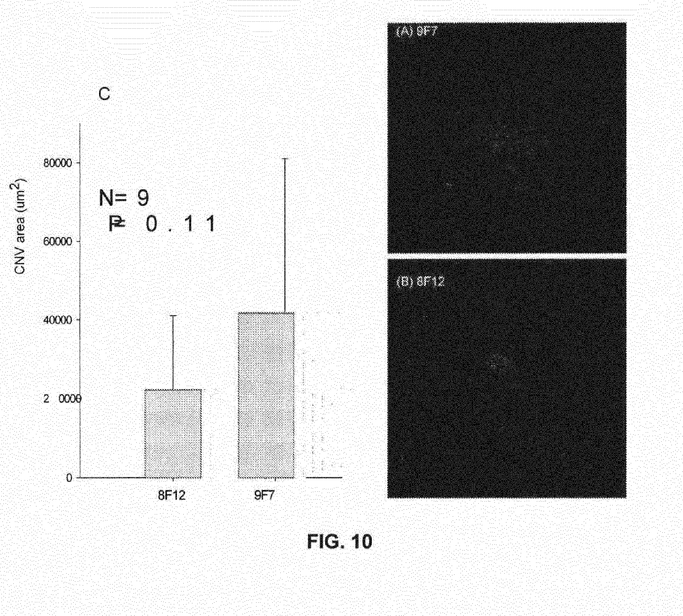

FIG. 10 illustrates the results of testing conducted in CNV mouse model, wherein 8F12 HtrA1 monoclonal antibody and 9F7 HtrA1 monoclonal antibody were administered to the mice.

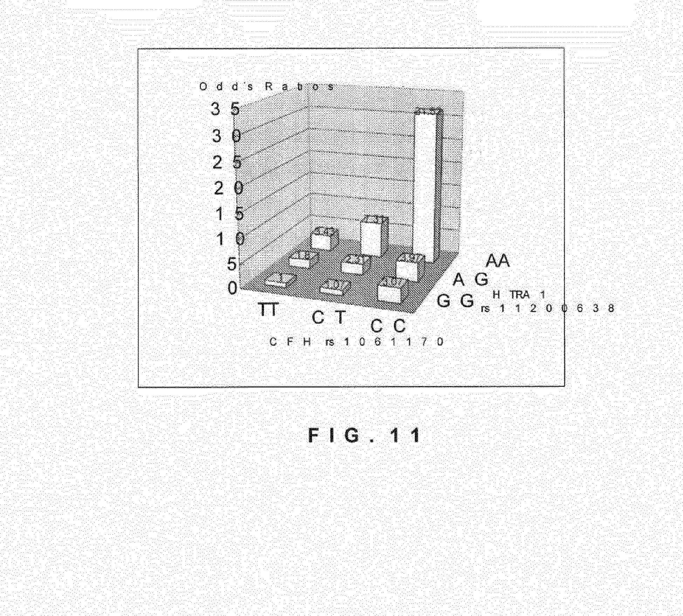

FIG. 11 illustrates the association or odds ratios with respect to AMD for HtrA1 rs11200638 and CFH rs1061170. Odds ratios in parentheses were calculated to compare each genotypic combination to the baseline of homozygosity for the common allele at both loci (TT/GG).

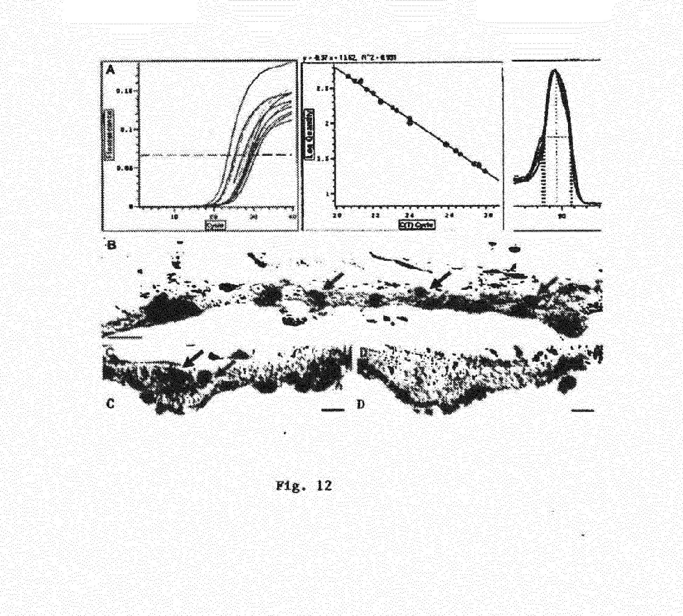

FIG. 12(A) illustrates the results of Real Time RT-PCR semiquantitative analysis of HtrA1 RNA from AMD subject blood samples. AMD subjects include GG and AA genotypes. Example of amplification reactions (left), standard curve measurements (standards-black circles; samples-gray circles) (center), and Htra1 melting curve (right).
FIG. 12(B) illustrates that HtrA1 immunolabeling shows focal staining in a majority of drusen. However, not all drusen were labeled by anti-HtrA1 antibody.
FIG. 12(C) illustrates (under higher magnification) drusen and thickened regions of Bruch's membrane showing diffuse staining as well as more intensely labeled focal areas (arrows).
FIG. 12(D) shows an adjacent section (to C) that served as a negative control omitting the primary antibody.

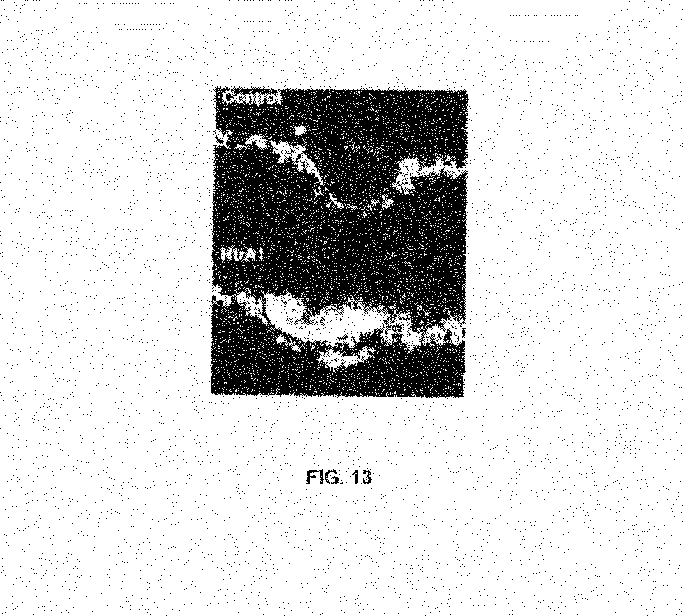

FIG. 13 illustrates immunohistochemical detection of HtrA1 in a dry Age-Related Macular Degeneration donor eye using confocal microscopy according to an embodiment of the present invention. The upper panel illustrates in the adjacent section (to low panel) a negative control omitting the primary antibody. The purple signal represents lipofuscin autofluorescence in RPE. The lower panel: drusen is immunolabeled with HTRA1 antibody (green) in an eye from a patient with dry Age-Related Macular Degeneration.

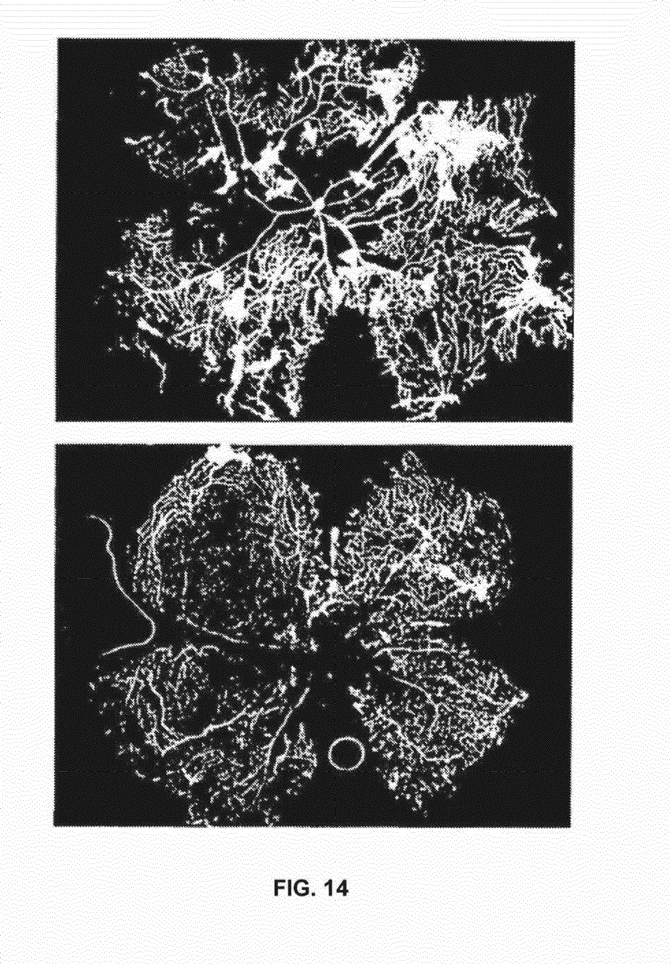

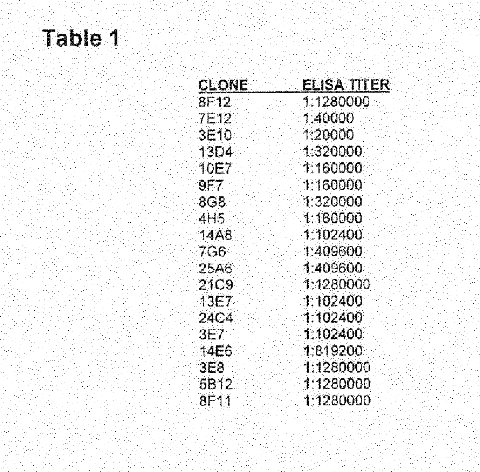

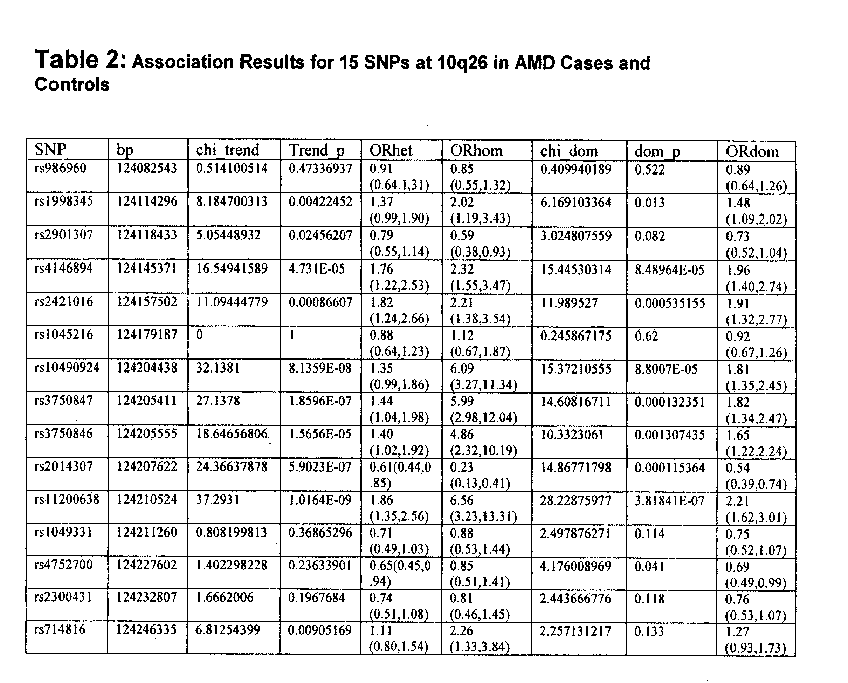

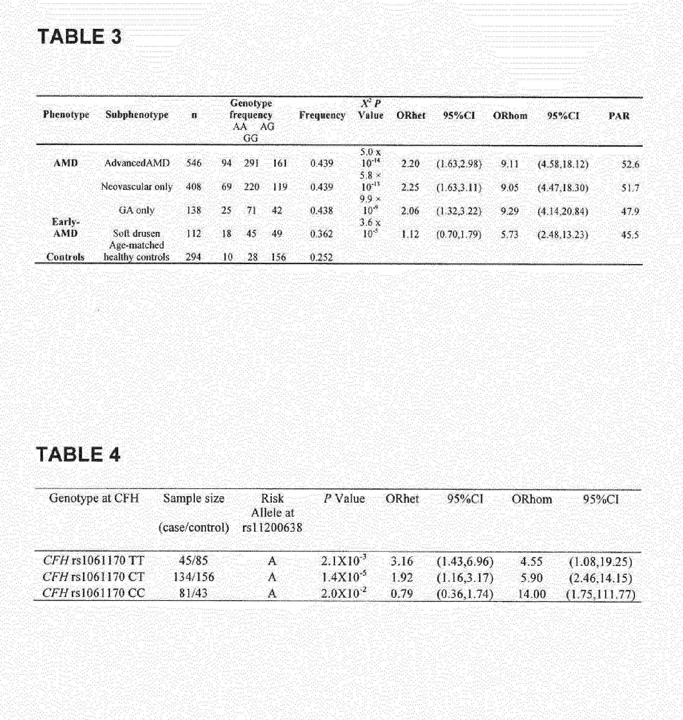

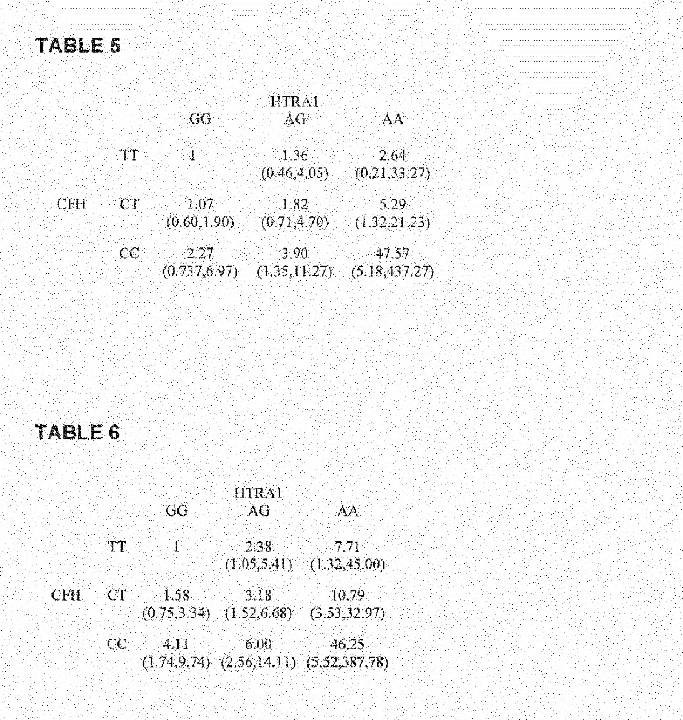

FIG. 14 illustrates rescue of retinal vascular defects and suppression of retinal neovascularization in a mouse model of retinopathy of prematurity and diabetic retinopathy induced by hypoxia according to an embodiment of the present invention. The upper panel illustrates a retina flat mount of a mouse model of retinal neovascularzation with large areas of capillary vasculature non-perfusion/drop-out (arrows, black areas) and retinal neovascularization (arrow heads, bright areas). This particular eye was injected with PBS solution as a control. The lower panel illustrates a retina flat mount of a mouse model of retinal neovascularization illustrating restoration of normal capillary vasculature and elimination of retinal neovascularization after treatment with 1ul of polyclonal antibody directed against HTRA1.

TABLE 1: provides the measured titers for HtrA1 monoclonal antibodies produced by the hybridoma cell lines therein and described in the present specification.

TABLE 2: Genotypes of samples from subjects using a panel of 15 SNPs.

TABLE 3: Association between subphenotypes of AMD and HTRA1 variant (rs11200638) in the Utah cohort. Illustrated are calculations for the A allele of the HTRA1 variant (rs11200638) and the corresponding number of unrelated affected individuals (N), the allelic frequency in affected individuals (Frq), p-value, odds ratio (OR) assuming a multiplicative model, and population attributable risk (PAR). The control group was age-matched to the patient group and the individuals had normal eye examinations.

TABLE 4: Conditional association for *HTRA1* rs11200638 on genotype at CFH rs1061170.

TABLE 5: Two-locus Odds Ratios for HtrA1 rs11200638 and CFH rs1061170 in AMD cases with GA phenotype. Odds ratios with 95% confidence intervals in parentheses were calculated to compare each genotypic combination to the baseline of homozygosity for the common allele at both loci (TT/GG).

TABLE 6: Two-locus Odds Ratios for *HTRA1* rs11200638 and *CFH* rs1061170 in wet AMD cases. Odds ratios with 95% confidence intervals in parentheses were calculated to compare each genotypic combination to the baseline of homozygosity for the common allele at both loci (TT/GG).

### DETAILED DESCRIPTION

### Definitions

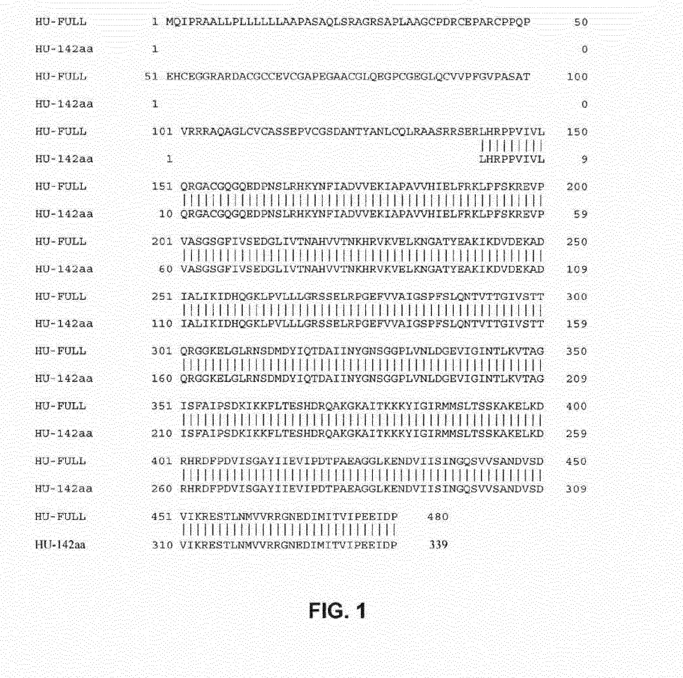

As used herein, the terms "HtrA1," "HtrA1 protein" and "HtrA1 polypeptide" refer to a protein or peptide, or a fragment thereof, encoded by the HtrA1 gene, such as, for example, the human HtrA1 fragment (SEQ. ID. NO.: 1) or the full-length human HtrA1 (SEQ. ID. NO.: 2) having the deduced amino acid sequences shown in FIG. 1.

As used herein, the terms "CFH," "CFH protein" and "CFH polypeptide" refer to a protein or peptide, or a fragment thereof, encoded by a CFH gene.

The term "polypeptide" refers to a polymer of amino acids and its equivalent and does not refer to a specific length of the product. Thus, peptides, oligopeptides and proteins are included within the definition of a polypeptide. This term also does not exclude modifications of the polypeptide, for example, glycosylations, acetylations, phosphorylations, and the like. Included within the definition are, for example, polypeptides containing one or more analogs of an amino acid (including, for example, unnatural amino acids, etc.), polypeptides with substituted linkages as well as other modifications known in the art, both naturally and non-naturally occurring.

"HtrA1 variant" refers to an HtrA1 variant having at least about 80%, 81%, 82%, 83%, 84%, 85%, 86%, 87%, 88%, 89%, 90%, 91%, 92%, 93%, 94%, 95%, 96%, 97%, 98% or 99% amino acid sequence identity with the HtrA1 fragment (SEQ. ID. NO.: 1) or the full-length HtrA1 (SEQ. ID. NO.: 2) having the deduced amino acid sequences shown in FIG. 1. Such HtrA1 variants include, for instance, HtrA1 polypeptides wherein one or more amino acid residues are substituted and HtrA1 polypeptides wherein one or more amino acid residues are added, or deleted (*i*.*e*., fragments), at the N- or C-terminus of the HtrA molecule. Also included are proteins encoded by DNA which hybridize under high or low stringency conditions, to HtrA1-encoding nucleic acids and closely related polypeptides or proteins retrieved by antisera to the HtrA1 protein(s). The length of polypeptide sequences compared for homology may be at least about 16 amino acids, at least about 20 residues, at least about 24 residues, at least about 28 residues, or more than about 35 residues.

"CFH variant" refers to a CFH variant having at least about 80%, 81%, 82%, 83%, 84%, 85%, 86%, 87%, 88%, 89%, 90%, 91%, 92%, 93%, 94%, 95%, 96%, 97%, 98% or 99% amino acid sequence identity with a wild-type CFH protein. Such CFH variants include, for instance, CFH polypeptides wherein one or more amino acid residues are substituted and CFH polypeptides wherein one or more amino acid residues are added, or deleted (*i*.*e*., fragments), at the N- or C-terminus of the CFH molecule. Also included are proteins encoded by DNA which hybridize under high or low stringency conditions to CFH-encoding nucleic acids and closely related polypeptides or proteins retrieved by antisera to the CFH protein(s). The length of polypeptide sequences compared for homology may be at least about 16 amino acids, at least about 20 residues, at least about 24 residues, at least about 28 residues, or more than about 35 residues

"Fragments" of the polypeptides described herein may retain biological activities include ligand-binding, immunological activity and other biological activities characteristic of the full-length polypeptides from which they are derived. Immunological activities include both immunogenic function in a target immune system, as well as sharing of immunological epitopes for binding, serving as either a competitor or substitute antigen for an epitope of the wild-type protein. The terms a polypeptide "fragment", "portion" or "segment" refer to a stretch of amino acid residues of at least about five to seven contiguous amino acids, at least about seven to nine contiguous amino acids, at least about nine to 13 contiguous amino acids, or at least about 20 to 30 or more contiguous amino acids.

"Percent (%) amino acid sequence identity" with respect to a polypeptide identified herein is defined as the percentage of amino acid residues in a candidate sequence that are identical with the amino acid residues in a second polypeptide sequence to which the candidate sequence is compared, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as, for example, ALIGN™ or Megalign (DNASTAR). Protein analysis software typically matches similar sequences using measure of homology assigned to various substitutions, deletions and other modifications. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine. Those of ordinary skill in the art can determine appropriate parameters for measuring alignment, including any algorithms needed to achieve maximal alignment over the full length of the sequences being compared.

The term "HtrA1 alleles" refers to normal alleles (also referred to as wild-type alleles) of the HtrA1 locus as well as alleles carrying variations that predispose subjects to develop ocular disease of pathologies as described herein. Such predisposing alleles are also called "HtrA1 risk alleles," "HtrA1 susceptibility alleles" or "HtrA1 mutant alleles".

The term "CFH alleles" refers to normal alleles (also referred to as wild-type alleles) of the CFH locus as well as alleles carrying variations that predispose subjects to develop ocular disease of pathologies as described herein. Such predisposing alleles are also called "CFH risk alleles," "CFH susceptibility alleles," or "CFH mutant alleles".

The terms "HtrA1 locus", "HtrA1 gene", "HtrA1 nucleic acids" or "HtrA1 polynucleotide" each refer to polynucleotides, which are in the HtrA1 region. The locus is indicated in part by mutations that predispose subjects to develop disease or pathologic conditions of the eye, and such mutations fall within the HtrA1 region described.

The HtrA1 locus is intended to include HtrA1 coding sequences, intervening sequences and regulatory elements controlling transcription and/or translation. The HtrA1 locus is intended to include all allelic variations of the DNA sequence.

The term "HtrA1 nucleic acids" or "HtrA1 polynucleotides" is also extended to refer to nucleic acids that encode a HtrA1 polypeptide, polypeptide variants of HtrA1, protein fusions and deletions of any of the above. These nucleic acids comprise a sequence which is either derived from, or substantially similar to a natural HtrA1-encoding gene or one having substantial homology with a natural HtrA1-encoding gene or a portion thereof.

The terms "CFH locus", "CFH gene", "CFH nucleic acids" or "CFH polynucleotide" each refer to polynucleotides, which are in the CFH region. The locus is indicated in part by mutations that predispose subjects to develop disease or pathologic conditions of the eye, and such mutations fall within the CFH region described.

The CFH locus is intended to include CFH coding sequences, intervening sequences and regulatory elements controlling transcription and/or translation. The CFH locus is intended to include all allelic variations of the DNA sequence.

The term "CFH nucleic acids" or "CFH polynucleotides" is also extended to refer to nucleic acids that encode a CFH polypeptide, polypeptide variants of CFH, protein fusions and deletions of any of the above. These nucleic acids comprise a sequence which is either derived from, or substantially similar to a natural CFH-encoding gene or one having substantial homology with a natural CFH-encoding gene or a portion thereof.

The polynucleotide compositions of this invention include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (*e.g.,* methyl phosphonates, phosphotriesters, phosphoramidates, carbamates, etc.), charged linkages (*e.g.,* phosphorothioates, phosphorodithioates, etc.), pendent moieties (e.g., polypeptides), intercalators (*e.g.,* acridine, psoralen, etc.), chelators, alkylators, and modified linkages (*e.g.,* alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term "HtrA1 region" refers to a portion of human chromosome 10 that contains the HtrA1 locus, including the HtrA1 gene.

The terms "HtrA1 locus", "HtrA1 allele" and "HtrA1 region" all refer to the double-stranded DNA comprising the locus, allele, or region, as well as either of the single-stranded DNAs comprising the locus, allele or region.

The term a "portion" of the HtrRA1 locus or region or allele is defined as having a minimal size of at least about eight nucleotides, at least about 15 nucleotides, or at least about 25 nucleotides, and may have a minimal size of at least about 40 nucleotides. This definition includes all sizes in the range of 8-40 nucleotides as well as greater than 40 nucleotides.

The term "HtrA1 region" refers to a portion of human chromosome 10 that contains the HtrA1 locus, including the HtrA1 gene.

The terms "CFH locus", "CFH allele" and "CFH region" all refer to the double-stranded DNA comprising the locus, allele, or region, as well as either of the single-stranded DNAs comprising the locus, allele or region.

The term a "portion" of the CFH locus or region or allele is defined as having a minimal size of at least about eight nucleotides, at least about 15 nucleotides, or at least about 25 nucleotides, and may have a minimal size of at least about 40 nucleotides. This definition includes all sizes in the range of 8-40 nucleotides as well as greater than 40 nucleotides.

The term "CFH region" refers to a portion of human chromosome 1 that contains the CFH locus, including the CFH gene.

The term "regulatory sequences" refers to those sequences normally within 100-1000 kilobases (kb) of the coding region of a locus, but they may also be more distant from the coding region, which affect the expression of the gene. Such regulation of expression comprises transcription of the gene, and translation, splicing, and stability of the messenger RNA.

The term "operably linked" refers to a juxtaposition wherein the components so described are in a relationship permitting them to function in their intended manner. For instance, a promoter is operably linked to a coding sequence if the promoter affects its transcription or expression. The term "operably linked" may refer to functional linkage between a nucleic acid expression control sequence (*e.g.,* a promoter, enhancer, or array of transcription factor binding sites) and a second nucleic acid sequence, wherein the expression control sequence directs transcription of the nucleic acid corresponding to the second sequence.

The term "vector" or "recombinant DNA cloning vehicle" refers to a specifically designed nucleic acid, polynucleotide or DNA molecule capable of autonomous existence and replication in an appropriate host cell. The vector comprises a member selected from the group comprising plasmid, bacteriophage, and artificial chromosome construct. This vehicle or vector may "carry" inserted DNA, said inserted DNA may comprise HtrA1 polynucleotide or nucleic acid or CFH polynucleotide or nucleic acid. The vector may allow expression of one or more genes carried on the inserted DNA in an appropriate host cell. The expressed gene product may be a polypeptide or RNA. The vector may allow expression of the antisense RNA of a gene. The vector may allow for production of antisense RNA or DNA of the inserted gene or genes in a cell-free system by methods well known in the art (Maniatis et al., 1982; Sambrook et al., 1989; Ausubel et al., 1992). The vector may exist in a host cell or in substantially pure form. The vector may exist in a host cell as an autonomous replicating unit or an autonomous unit, or alternatively it may integrate into the genome of the host cell.

The "vector" may be a recombinant DNA or polynucleotide molecule comprising all or part of the HtrA1 region or the CFH region or all or part of both the HtrA1 region and the CFH region. The recombinant construct may be capable of replicating autonomously in a host cell. Alternatively, the recombinant construct may become integrated into the chromosomal DNA of the host cell. Such a recombinant polynucleotide comprises a polynucleotide of genomic DNA, cDNA, semi-synthetic, or synthetic origin which, by virtue of its origin or manipulation, 1) is not associated with all or a portion of a polynucleotide with which it is associated in nature; 2) is linked to a polynucleotide other than that to which it is linked in nature; or 3) does not occur in nature.

Therefore, recombinant polynucleotides comprising sequences otherwise not naturally occurring are provided by this invention. Although the wild-type sequence may be employed, it will often be altered, e.g., by deletion, substitution or insertion.

Genomic DNA or cDNA libraries of various types may be screened as natural sources of the nucleic acids of the present invention, or such nucleic acids may be provided by amplification of sequences resident in genomic DNA or other natural sources, e.g., by PCR. The choice of cDNA libraries normally corresponds to a tissue source which is abundant in mRNA for the desired proteins. Phage libraries are normally preferred, but other types of libraries may be used. Clones of a library are spread onto plates, transferred to a substrate for screening, denatured and probed for the presence of desired sequences.

The DNA sequences used in this invention will usually comprise at least about five codons (15 nucleotides), more usually at least about 7-15 codons, and most preferably, at least about 35 codons. One or more introns may also be present. This number of nucleotides is usually about the minimal length required for a successful probe that would hybridize specifically with one of a desired sequence from a target region (e.g., a desired sequence from the HtrA1 region or a desired sequence from the CFH region).

Techniques for nucleic acid manipulation are described generally, for example, in Sambrook et al., 1989 or Ausubel et al., 1992. Reagents useful in applying such techniques, such as restriction enzymes and the like, are widely known in the art and commercially available from such vendors as New England BioLabs, Boehringer Mannheim, Amersham, Promega Biotech, U.S. Biochemicals, New England Nuclear, and a number of other sources. The recombinant nucleic acid sequences used to produce fusion proteins of the present invention may be derived from natural or synthetic sequences. Many natural gene sequences are obtainable from various cDNA or from genomic DNA libraries using appropriate probes. See, GenBank, National Institutes of Health.

The term "recombinant nucleic acid, polynucleotide or DNA" refers to a nucleic acid molecule which is not naturally occurring, or which is made by the artificial combination of two otherwise separated segments of sequence. This artificial combination is often accomplished by either chemical synthesis means, or by the artificial manipulation of isolated segments of nucleic acids, *e.g.,* by genetic engineering techniques. Such is usually done to replace a codon with a redundant codon encoding the same or a conservative amino acid, while typically introducing or removing a sequence recognition site, for example, for a restriction endonuclease. Alternatively, it is performed to join together nucleic acid segments of desired functions to generate a desired combination of functions.

The terms "probe" or "probes" refer to polynucleotide probes for the purpose of detecting, by hybridization, polynucleotide polymorphisms associated with a targeted allele (*e.g.,* a targeted HtrA1 allele or a targeted CFH allele) which predisposes a subject to disease or pathologic condtions of the eye. Each probe is designed to form a stable hybrid with that of the target sequence, under highly stringent to moderately stringent hybridization and wash conditions. If it is expected that a probe will be perfectly complementary to the target sequence, high stringency conditions will be used. Hybridization stringency may be lessened if some mismatching is expected, for example, if variants are expected with the result that the probe will not be completely complementary. Conditions are chosen which rule out nonspecific/adventitious bindings, that is, which minimize noise. (It should be noted that throughout this disclosure, if it is simply stated that "stringent" conditions are used that is meant to be read as "high stringency" conditions are used.) Since such indications identify neutral DNA polymorphisms as well as mutations.

Probes for targeted alleles may be derived from the sequences of the relevant genetic region or its cDNAs. The probes may be of any suitable length, which span all or a portion of the relevant genetic region, and which allow specific hybridization to the relevant genetic region. If the target sequence contains a sequence identical to that of the probe, the probes may be short, *e.g.,* in the range of about 8-30 base pairs, since the hybrid will be relatively stable under even highly stringent conditions. If some degree of mismatch is expected with the probe, *i*.*e*., if it is suspected that the probe will hybridize to a variant region, a longer probe may be employed which hybridizes to the target sequence with the requisite specificity.

The probes will include an isolated polynucleotide attached to a label or reporter molecule and may be used to isolate other polynucleotide sequences, having sequence similarity by standard methods. For techniques for preparing and labeling probes see, *e.g.,* Sambrook et al., 1989 or Ausubel et al., 1992. Other similar polynucleotides may be selected by using homologous polynucleotides. Alternatively, polynucleotides encoding these or similar polypeptides may be synthesized or selected by use of the redundancy in the genetic code. Various codon substitutions may be introduced, *e.g.,* by silent changes (thereby producing various restriction enzyme recognition sites) or to optimize expression for a particular system. Mutations may be introduced to modify the properties of the polypeptide, perhaps to change ligand-binding affinities, interchain or intermolecular affinities, or the polypeptide degradation or turnover rate.

Probes comprising synthetic oligonucleotides or other polynucleotides of the present invention may be derived from naturally occurring or recombinant single-or double-stranded polynucleotides, or be chemically synthesized. Probes may also be labeled by nick translation, Klenow fill-in reaction, or other methods known in the art.

Portions of the polynucleotide sequence having at least about eight nucleotides, usually at least about 15 nucleotides, and fewer than about 6 kilobases (kb), usually fewer than about 1.0 kb, from a polynucleotide sequence encoding HtrA1 may be used as probes. This definition therefore includes, for example, probes of sizes 8 nucleotides through 6000 nucleotides. The probes may also be used to determine whether mRNA encoding are targeted polypeptide (*e.g.* an HtrA1 or CFH polypeptide) is present in a cell or tissue.

The term "substantial homology or similarity" when referring to a nucleic acid or fragment thereof indicates that when optimally aligned (with appropriate nucleotide insertions or deletions) with the other nucleic acid (or its complementary strand), there is nucleotide sequence identity in at least about 60% of the nucleotide bases, at least about 70% of the nucleotide bases, at least about 80% of the nucleotide bases, at least about 90% of the nucleotide bases, and at least about 95-98% of the nucleotide bases.

Alternatively, substantial homology or similarity exists when a nucleic acid or fragment thereof hybridizes to another nucleic acid (or a complementary strand thereof) under selective hybridization conditions, to a strand, or to its complement. Selectivity of hybridization exists when hybridization which is substantially more selective than total lack of specificity occurs. Typically, selective hybridization will occur when there is at least about 55%, at least about 65%, at least about 75%, or at least about 90% homology over a stretch of at least about 14 nucleotides (See, Kanehisa, 1984). The length of homology comparison, as described, may be over longer stretches, and in certain embodiments will often be over a stretch of at least about nine nucleotides, at least about 20 nucleotides, at least about 24 nucleotides, at least about 28 nucleotides, at least about 32 nucleotides, and at least about 36 or more nucleotides.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C., typically in excess of 37°C., and preferably in excess of 45°C. Stringent salt conditions will ordinarily be less than 1000 mM, typically less than 500 mM, and preferably less than 200 mM. However, the combination of parameters is much more important than the measure of any single parameter (See, *e.g.,* Wetmur and Davidson, 1968). Probe sequences may also hybridize specifically to duplex DNA under certain conditions to form triplex or other higher order DNA complexes. The preparation of such probes and suitable hybridization conditions are well known in the art.

The term "target region" refers to a region of the nucleic acid which is amplified and/or detected. The term "target sequence" refers to a sequence with which a probe or primer will form a stable hybrid under desired conditions.

The terms "analyte polynucleotide", "polynucleotide in analyte", and "analyte strand" refer to a single- or double-stranded polynucleotide which is suspected of containing a target sequence, and which may be present in a variety of types of samples, including biological samples.

The term "protein modifications or fragments" refers to HtrA1 polypeptides or fragments thereof and CFH polypeptides and fragments thereof that are substantially homologous to primary structural sequence but which include, *e.g.,* in vivo or in vitro chemical and biochemical modifications or which incorporate unusual amino acids. Such modifications include, for example, acetylation, carboxylation, phosphorylation, glycosylation, ubiquitination, labeling, e.g., with radionuclides, and various enzymatic modifications, as will be readily appreciated by those well skilled in the art. A variety of methods for labeling polypeptides and of substituents or labels useful for such purposes are well known in the art, and include radioactive isotopes such as ³²P, ligands which bind to labeled antiligands (*e.g.,* antibodies), fluorophores, chemiluminescent agents, enzymes, and antiligands which can serve as specific binding pair members for a labeled ligand. The choice of label depends on the sensitivity required, ease of conjugation with the primer, stability requirements, and available instrumentation. Methods of labeling polypeptides are well known in the art (See, Sambrook et al., 1989 or Ausubel et al., 1992).

The term "epitope" refers to a region of a polypeptide that provokes a response by an antibody. This region needs not comprise consecutive amino acids. The term epitope is also known in the art as "antigenic determinant". An epitope may comprise three amino acids in a spatial conformation which is unique to the epitope. Generally, an epitope consists of at least five such amino acids, and more usually consists of at least 8-10 such amino acids. Methods of determining the spatial conformation of such amino acids are known in the art.

For immunological purposes, tandem-repeat polypeptide segments may be used as immunogens, thereby producing highly antigenic proteins. Alternatively, such polypeptides will serve as highly efficient competitors for specific binding. Production of antibodies specific for HtrA1 polypeptides, CFH polypeptides or fragments thereof is described below.

The term "fusion protein" refers to fusion polypeptides comprising HtrA1 polypeptides, fragments of HtrA1, CFH polypeptides, or fragments of CFH polypeptides. Homologous polypeptide fusions may be between two or more related polypeptide sequences (*e.g.* polypeptide sequences derived from the same protein or related proteins). Heterologous fusions may be constructed which would exhibit a combination of properties or activities of the derivative polypeptides. A fusion protein may have the DNA binding domain of first derivative polypeptide and the transcription activation domain of another derivative polypeptide (for example, the transcriptional activation domain of the yeast GAL4 protein (Ma and Ptashne, 1987)). Such homologous or heterologous fusion polypeptides may display altered strength or specificity of binding. A heterologous polypeptide or polypeptide fragment may confer a new activity not possessed by one of the derivative polypeptides. Fusion partners include, *inter alia,* GST, immunoglobulins, bacterial beta-galactosidase, trpE, protein A, ß-lactamase, α amylase, maltose binding protein, alcohol dehydrogenase, polyhistidine (for example, six histidine at the amino and/or carboxyl terminus of the polypeptide), green fluorescent protein, yeast α mating factor, GAL4 transcription activation or DNA binding domain, and luciferase (See, Godowski et al., 1988). Fusion proteins will typically be made by either recombinant nucleic acid methods, as described above, or may be chemically synthesized. Techniques for the synthesis of polypeptides are described, for example, in Merrifield, 1963.

The terms "substantial homology" or "substantial identity", when referring to polypeptides, indicate that the polypeptide or protein in question exhibits at least about 30% amino acid sequence identity with an entire naturally-occurring protein or a portion thereof, at least about 70% amino acid sequence identity with an entire naturally-occurring protein or a portion thereof, or at least about 95% amino acid sequence identity with an entire naturally-occurring protein or a portion thereof.

The term "substantially similar function" refers to the function of a modified nucleic acid or a modified polypeptide (or protein) with reference to the wild-type nucleic acid or wild-type polypeptide (*e.g.,* the wild-type HtrA1 nucleic acid, wild-type HtrA1 polypeptide, wild-type CFH nucleic acid or the wild-type CFH polypeptide). The modified polypeptide will be substantially homologous to the wild-type polypeptide and will have substantially the same biological acitivity. The modified polypeptide may have an altered amino acid sequence and/or may contain modified amino acids. In addition to the similarity of function, the modified polypeptide may have other useful properties, such as a longer half-life. The similarity of function (biological activity) of the modified polypeptide may be substantially the same as the biological activity of the wild-type polypeptide. Alternatively, the similarity of function of the modified polypeptide may be higher than the activity of the wild-type polypeptide. The modified polypeptide is synthesized using conventional techniques, or is encoded by a modified nucleic acid and produced using conventional techniques. The modified nucleic acid is prepared by conventional techniques. A nucleic acid with a function substantially similar to the wild-type gene function produces a modified protein as described herein.

"Isolated," when used to describe biomolecules disclosed herein, means a polypeptide, nucleic acid, antibody that has been identified and separated and/or recovered from a component of its natural environment. Contaminant components of its natural environment are materials that would typically interfere with diagnostic or therapeutic uses for the polypeptide or antibody, and may include enzymes, hormones, and other proteinaceous or non-proteinaceous materials. Methods for isolation and purification of biomolecules described herein are known and available in the art, and one of ordinary skill in the art can determine suitable isolation and purification methods in light of the material to be isolated or purified. Though isolated biomolecules will typically be prepared using at least one purification step, as it is used herein, "isolated" additionally refers to, for example, polypeptide, antibody, or nucleic acid materials *in*-*situ* within recombinant cells, even if expressed in a homologous cell type. In particular, where an HtrA1 polypeptide, CFH polypeptide, HtrA1 antibody, or CFH antibody as described herein is recombinantly expressed, at least one component of the polypeptide or antibody natural environment will not be present.

Further, where the terms "isolated", "substantially pure", and "substantially homogeneous" are used to describe a monomeric protein they are used interchangeably herein. A monomeric protein is substantially pure when at least about 60 to 75% of a sample exhibits a single polypeptide sequence. A substantially pure protein can typically comprise about 60 to 90% W/W of a protein sample, and where desired, a substantially pure protein can be greater than about 90%, about 95%, or about 99% pure. Protein purity or homogeneity can be indicated by a number of means well known in the art, such as polyacrylamide gel electrophoresis of a protein sample, followed by visualizing a single polypeptide band upon staining the gel. For certain purposes, higher resolution can be provided by using HPLC or other means well known in the art which are utilized for purification.

The terms "amino acid" and "amino acids" refer to all naturally occurring L-alpha-amino acids. This definition includes norleucine, ornithine, and homocysteine. Amino acids are identified by either the standard single-letter or standard three-letter designations.

The terms "antagonist" and "antagonistic" when used herein refer to or describe a molecule which is capable of, directly or indirectly, counteracting, reducing or inhibiting HtrA1 biological activity or CFH biological activity.

The term "antibody" is used in the broadest sense and specifically covers single anti-HtrA1 monoclonal antibodies (including agonist, antagonist, and neutralizing or blocking antibodies) and anti-HtrA1 antibody compositions with polyepitopic specificity (*e*.*g*., compositions including two or more different antibodies exhibiting specificity to different epitopic regions). "Antibody" as used herein encompases intact or full-length immunoglobulin or antibody molecules, polyclonal antibodies, multispecific antibodies (i.e., bispecific antibodies formed from at least two intact antibodies) as well as immunoglobulin fragments (such as Fab, F(ab')₂, or Fv), provided that such fragments exhibit one or more desired biologic actibities or properties, such as, for example, the binding affinity, titer, anti-neovascularization, agonistic or antagonistic properties described herein.

Antibodies are typically proteins or polypeptides which exhibit binding specificity to a specific antigen. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light chain variable domain is aligned with the variable domain of the heavy chain. Particular amino acid residues are believed to form an interface between the light and heavy chain variable domains (See, Chothia et al., J. Mol. Biol., 186:651-663 (1985); Novotny and Haber, Proc. Natl. Acad. Sci. USA, 82:4592-4596 (1985)). The light chains of antibodies from any vertebrate species can be assigned to one of two clearly distinct types, called kappa and lambda, based on the amino acid sequences of their constant domains. Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. The heavy chain constant domains that correspond to the different classes of immunoglobulins are called alpha, delta, epsilon, gamma, and mu, respectively.

"Antibody fragments" comprise a portion of an intact antibody, generally the antigen binding or variable region of the intact antibody. Examples of antibody fragments include Fab, Fab', F(ab')₂, and Fv fragments, diabodies, single chain antibody molecules, and multispecific antibodies formed from antibody fragments.

The term "variable" is used herein to describe certain portions of the variable domains which differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a ß-sheet configuration, connected by three CDRs, which form loops connecting, and in some cases forming part of, the ß-sheet structure. The CDRs in each chain are held together in close proximity by the FR regions and, with the CDRs from the other chain, contribute to the formation of the antigen binding site of antibodies (See, Kabat, E. A. et al., Sequences of Proteins of Immunological Interest, National Institutes of Health, Bethesda, Md. (1987)).

The term "monoclonal antibody" as used herein refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen.

The monoclonal antibodies herein include chimeric, hybrid and recombinant antibodies produced by splicing a variable (including hypervariable) domain of an anti-HtrA1 antibody or an anti-CFH antibody with a constant domain (e.g., "humanized" antibodies), or a light chain with a heavy chain, or a chain from one species with a chain from another species, or fusions with heterologous proteins, regardless of species of origin or immunoglobulin class or subclass designation, as well as antibody fragments (*e*.*g*., Fab, F(ab')₂, and Fv), provided, again, that such fragments exhibit one or more desired biological activity or property as set out herein (See, *e.g.* U.S. Patent No. 4,816,567 and Mage et al., in Monoclonal Antibody Production Techniques and Applications, pp. 79-97 (Marcel Dekker, Inc.: New York, 1987)).

The modifier "monoclonal," therefore, indicates the character of the antibody as being obtained from a substantially homogeneous population of antibodies, and is not to be construed as requiring production of the antibody by any particular method. For example, monoclonal antibodies according to the present description can be produced by the hybridoma method described by Kohler and Milstein, Nature, 256:495 (1975), by the methods set out in Harlow, E and Lane, D, Antibodies: A laboratory Manual. 1988 Cold Spring Harbor, NY: Cold Spring Harbor Laboratory Press, or by recombinant DNA methods, such as the methods described in U.S. Patent No. 4,816,567. The "monoclonal antibodies" may also be isolated from phage libraries generated using the techniques described in McCafferty et al., Nature, 348:552-554 (1990), for example.

"Humanized" forms of non-human (*e.g.,* murine) antibodies are specific chimeric immunoglobulins, immunoglobulin chains, or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin.

A "human antibody" is one which possesses an amino acid sequence which corresponds to that of an antibody produced by a human and/or has been made using any of the techniques for making human antibodies known in the art or as disclosed herein. This definition of a human antibody includes antibodies comprising at least one human heavy chain polypeptide or at least one human light chain polypeptide, for example an antibody comprising murine light chain and human heavy chain polypeptides. Human antibodies can be produced using various techniques known in the art. In one embodiment, the human antibody is selected from a phage library, where that phage library expresses human antibodies (Vaughan et al. Nature Biotechnology, 14:309-314 (1996): Sheets et al. PNAS, (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368:812-13 (1994); Fishwild et al., Nature Biotechnology, 14: 845-51 (1996); Neuberger, Nature Biotechnology, 14: 826 (1996); Lonberg and Huszar, Intern. Rev. Immunol., 13:65-93 (1995). Alternatively, the human antibody may be prepared via immortalization of human B lymphocytes producing an antibody directed against a target antigen (such B lymphocytes may be recovered from an subject or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and U.S. Patent No. 5,750,373.

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology, 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene, 169:147-155 (1995); Yelton et al. J. Immunol., 155:1994-2004 (1995); Jackson et al., J. Immunol., 154(7):3310-9 (1995); and Hawkins et al, J. Mol. Biol., 226:889-896 (1992).

The term "immunospecific" as used in "immunospecific binding of antibodies" for example, refers to the antigen specific binding interaction that occurs between the antigen-combining site of an antibody and the specific antigen recognized by that antibody.

When referring to an antibody or other therapeutic entity "biologically active" and "desired biological activity" refer to an ability to modulate HtrA1 activity or HtrA1 activation or CFH activity or activation. HtrA1 activity or activation refers to, by way of example, the function or activation of one or more of the HtrA1 trypsin domain, PDZ domain, a serine protease (SP) domain, insulin-like growth factor biding domain (IGFBP), Kazal-type trypsin (KI) inhibitory domain, Mac25 domain, or the follistatin (FS) domain. In particular, embodiments, when used in conjunction with an the biologically active agents described herein, "biologically active" and "desired biological activity" refer to an ability to directly or indirectly inhibit or block HtrA1 activity or activation, such as by, for example, inhibiting or blocking the activity or activation of one or more of the HtrA1 trypsin domain, PDZ domain, a serine protease (SP) domain, insulin-like growth factor biding domain (IGFBP), Kazal-type trypsin (KI) inhibitory domain, Mac25 domain, or the follistatin (FS) domain. CFH activation or activity refers, for example, to binding of complement 3b, inactivation of complement 3b (resulting in ic3B), inhibition complement 3b to properdin.

As used herein, the terms "treat," "treating," and "treatment" refer to a therapeutic benefit, whereby the detrimental effect(s) or progress of a particular disease, condition, event or injury is prevented, reduced, halted or slowed.

"Therapeutically-effective" refers to an amount of a pharmaceutically active substance, including an antibody, capable of treating a particular disease, condition, event or injury.

The term "subject" refers to an animal or human, preferably a mammal, subject in need of treatment for a given disease, condition, event or injury.

The terms "pathologic" or "pathologic conditions" refer to any deviation from a healthy, normal, or efficient condition which may be the result of a disease, condition, event or injury.

As used herein, the terms "ocular tissue(s)" and "tissue(s) of the eye" include, by way of example and not limitation, the retina, including the macula, the retinal pigment epithelium (RPE), the Bruch's membrane, the internal limiting membrane, and the choriod.

The terms "sample" or "test sample" as used herein refer to biological samples of tissue or fluid taken from a subject and suspected of containing an analyte polynucleotide or polypeptide from an individual including. Such a sample may comprise, by way of example and not limitation, plasma, serum, spinal fluid, lymph fluid, the external sections of the skin, respiratory, intestinal, and genitourinary tracts, tears, saliva, blood cells, tumors, organs, tissue, samples of *in-vitro* cell culture constituents, or any other tissue or bodily fluid from which sufficient DNA or RNA can be obtained. Samples may be collected by a variety of means for collecting cells, such as for example, a buccal swab.

### Ocular Disease

The pathologic growth of new blood vessels (neovascularization) in and around the tissues of the eye is associated with a variety of ocular diseases. In particular, hypoxia is known to be the primary stimulus for pathologic neovascularization of the choroid and Bruch's membrane that results in the often catastrophic vision loss associated with diabetic retinopathy, retinopathy of prematurity, and the wet form of AMD. Such ischemia-induced neovascularization is known to be induced or mediated by angiogenic factors such as, for example, vascular endothelial growth factor (VEGF), fibroblast growth factor (FGF-2), and transforming growth factor-ß (TGF-ß). Ischemia-induced neovascularization need not necessarily be caused by disease. For example, injury or insult to the ocular tissue can lead to hypoxia of ocular tissues and thereby cause ischemia-induced neovascularization that results in loss of vision.

Where pathologic neovascularization of, for example, the choroid and Bruch's membrane occurs, vision loss and tissue damage is not only caused by the physical intrusion of the new blood vessels, but such vessels tend to be fragile, often leak blood into the surrounding tissue, and can result in macular edema. Macular edema occurs when fluid (e.g., blood), lipids and protein deposits collect on or under the macula, causing the macula to thicken, swell, and/or raise away from its normal place at the back of the eye, further distorting or destroying vision.

As contemplated herein, however, the terms "ocular disease," "disease of the eye," and "pathologic conditions" of the eye are not limited to conditions associated with pathologic neovascularization and encompass, for example, the diseases and disorders described herein under the headings "Ocular Disease," "Diabetic Retinopathy," Retinopathy of Prematurity," and "Age Related Macular Degeneration." The terms "ocular disease," "disease of the eye," and "pathologic conditions" also encompass, by way of example, and not limitation, retinal vein occlusion, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cottonwool spots, hollenhorst plaque, central retinal vein occlusion, superior hemicentral retinal occlusion, branch retinal vein occlusion, chronic BRVO, retinal artery macroaneurism, and juxtafoveal telangiectas.

### Diabetic Retinopathy

Diabetic retinopathy (DR) is result of vascular damage that can result from poor blood sugar control. Diabetes mellitus is often characterized by reduced blood circulation, and the small blood vessels present in tissues of the eye are especially vulnerable to damage that may result from poor circulation and the resulting over accumulation of sugars present in the blood. For example, diabetes can result in hyperglycemia-induced pericyte death and thickening of the basement membrane and lead to incompetence of the vascular walls of the vessels present in the tissues of the eye. Such damage can change the detrimentally alter the of the blood retinal barrier, which consists of cells that are joined tightly together and work prevent certain substances from entering the tissue of the retina. The vascular damage caused resulting from diabetes mellitus can also lead to localized ischemia of ocular tissues and cause blood vessels present in the tissues of the eye become more permeable. Such localized ischemia and increased vascular permeability both lead to pathologic neovascularization, which ultimately results in the severe vision loss associated with DR.

### Retinopathy of Prematurity

Retinopathy of prematurity (ROP), also known as retrolental fibroplasia (RLF), is an eye disease that affects prematurely born babies. ROP can be mild and may resolve spontaneously. However, the condition may also progress and lead to severe visual impairment, even blindness. Maturation of ocular tissues normally proceeds in-utero, and at term, the tissues of the eye are fully vascularized. However, where an infant is born prematurely the tissues of eye are often not fully vascularized. ROP is thought to occur when the development of the vasculature of the eye is inhibited or arrested and then proceeds abnormally and in a disorganized fashion, leading to fibrovascular proliferation. Fibrovascular proliferation is the abnormal growth new vessels (neovascularization) that leads to formation of fibrous tissue (i.e., scar tissue) that may contract and cause retinal detachment. Multiple factors can determine whether ROP progresses. For example, the overall health, birth weight, and the stage of ROP at initial diagnosis can all effect either the extent to which the disease will progress or the treatability of the disease once detected.

### Age Related Macular Degeneration

Age-related macular degeneration (AMD) is the leading cause of irreversible blindness in developed countries. AMD is defined as an abnormality of the retinal pigment epithelium (RPE) that leads to overlying photoreceptor degeneration of the macula and consequent loss of central vision. Early AMD is characterized by drusen (>63µm) and hyper- or hypo-pigmentation of the RPE. Intermediate AMD is characterized by the accumulation of focal or diffuse drusen (>120 um) and hyper- or hypo-pigmentation of the RPE. Advanced AMD is associated with vision loss due to either geographic atrophy of the RPE and photoreceptors (dry AMD) or neovascular choriocapillary invasion across Bruch's membrane into the RPE and photoreceptor layers (wet AMD). AMD leads to a loss of central visual acuity, and can progress in a manner that results in severe visual impairment and blindness. Visual loss in wet AMD is more sudden and may be more severe than in dry AMD.

It is estimated that 1.75 million people in the United States alone suffer from advanced AMD (dry and wet AMD). Also in the United States alone, it is estimated that an additional 7.3 million people suffer from intermediate AMD, which puts them at increased risk for developing the advanced forms of the disease. It is projected that such numbers will increase significantly over the next 10 to 15 years. At this point, subjects with wet AMD are treated with laser photocoagulation, photodynamic therapy, or anti-angiogenic therapies to stabilize their disease and prevent further loss of vision. However, there is no treatment or cure for the more prevalent "dry" form of the disease.

The etiology and pathophysiology of AMD are poorly understood. The pathology is characterized by accumulation of membranous debris underneath the RPE basement membrane, which results in the formation of drusen. Drusen are small yellowish, extracellular deposits of lipid, protein, and cellular debris, accumulated between the RPE and Bruch's membrane or within Bruch's membrane. Drusen are made of protein and lipid material including complement components and modulators and HtrA1. It is though that the formation of drusen may be caused by RPE dysfunction or a change in Bruch's membrane composition (e.g., increased lipid deposition and protein cross linking) or the permeability of the Bruch's membrane to nutrients (e.g., impaired diffusion of water soluble plasma constituents across Bruch's membrane).

### HtrA1

HtrA1 is secretory protein that belongs to the mammalian family of HtrA serine proteases. Several members of the HtrA family have been characterized, with each member having a highly conserved trypsin and PDZ domains. Human HtrA1 (hHtrA1) includes a serine protease (SP) domain and its PDZ domain in the C-terminal region of the molecule. hHtrA1 also a signal sequence for secretion, an insulin-like growth factor biding domain (IGFBP), and a Kazal-type trypsin inhibitory domain (KI) in the N-terminal region of the molecule. Together, the IGFBP and KI domains are also known as the Mac25 domain or the follistatin (FS) domain. In addition to these domains, hHtrA1 also contains a linker region between the KI and trypsin domain. In has been reported that hHtrA1 is overexpressed in articular chondrocytes of osteoarthritis subjects, suggesting a potential role for HtrA1 in matrix remodeling and inflammatory processes. The PDZ domain of hHtrA1 has also been shown to interact with various extracellular matrix proteins, whereas the signal sequence and linker region have been shown to interact with the transforming growth factor (TGF) family of proteins. A catalytic triad (serine-328, histidine-220, and aspartic acid-250) in the trypsin domain of hHtrA1 is essential for protease activity, and mutation of serine-328 to alanine (SA mutation) has been shown to abrogate the protease activity of hHtrA1.

### Genetic Variation Associated with AMD

A genetic variation associated with an increased risk of developing AMD is described herein. In one embodiment, the genetic variation is a variation in the HtrA1 gene that results in increased expression of HtrA1 protein relative to a wild-type gene. In another embodiment, the genetic variation is a variation in the promoter region of the HtrA1 gene, wherein such genetic variation causes increased expression of HtrA1. In yet another embodiment, the genetic variation is identified as SNP rs11200638.

SNP rs11200638 is found at the 10q locus. To identify the critical gene at this locus, samples from 442 subjects suffering from AMD and samples from 309 control subjects that did not exhibit AMD were genotyped using a panel of 15 SNPs known to be located near SNP rs10490924, identified as a high risk SNP. Referring to Table 2, in agreement with previous reports, rs10490924 was found to have a highly significant association signal (p=8.1×10⁻⁸ for an additive allele-dosage model, ORₕₑₜ=1.35 (0.99, 1.86), ORₕₒₘ=6.09 (3.27, 11.34); T allele: 39.7% in subjects with AMD versus 24.7% in control subjects). However, of the 15 SNPs analyzed, rs11200638 was determined to be the variant most significantly associated with AMD (p = 1×10⁻⁹, ORₕₑₜ=1.86 (1.35, 2.56), ORₕₒₘ=6.56 (3.23, 13.31), A allele: 40.3% in subjects with AMD versus 25.2% in control subjects). The results of this analysis are provided in FIG. 11. In terms of the significance of the association, the TA haplotype across rs10490924 and rs11200638 was superior to rs10490924 (p=2.2×10⁻⁹), but inferior to rs11200638. In light of this information, an additional 139 samples were taken from subjects suffering from AMD were genotyped and analyzed for these two variants. The results for both SNPs increased in significance (rs10490924: p=1.2×10⁻⁸, rs11200638: p=1.6×10⁻¹¹), with variant rs11200638 remaining the best single variant explaining the association (ORₕₑₜ=1.90(1.40, 2.58), ORₕₒₘ=7.51(3.75, 15.04)).

Though initial efforts focused on determining the association of SNP rs11200638 with the wet form of AMD, it was also established that the presence of the same SNP rs11200638 is also correlated to an increased risk of the dry form of AMD. An expanded Utah Age-Related Macular Degeneration (AMD) and control cohort was genotyped using rs11200638. SNP rs11200638 exhibited a highly significant association with wet AMD (p=5,8 × 10⁻¹³ for an additive allele-dosage model, ORₕₑₜ=2.25 (1.63, 3.11), ORₕₒₘ=9.05 (4.47, 18.30); A allele: 43.9% in cases versus 25.2% in controls, Table 3) and conferred an estimated population attributable risk of 51.7%. Additionally, the HTRA1 rs11200638 exhibited a highly significant association with geographic atrophy (GA) (p=9.9 × 10⁻⁹ for an additive allele-dosage model, ORₕₑₜ=2.06 (1.32, 3.22), ORₕₒₘ=9.29 (4.14, 20.84); A allele: 43.8% in cases versus 25.2% in controls, (Table 3) and conferred an estimated population attributable risk of 47.9%. Soft confluence drusen was also associated with HTRA1 rs11200638 risk allele (p=3.6 × 10⁻⁵ for an additive allele-dosage model, ORₕₑₜ=1.12 (0..70, 1.79), ORₕₒₘ=5.73 (2.48, 13.23); A allele: 36.2% in cases versus 25.2% in controls, (Table 3). Thus, SNP rs11200638 contributes equally to both forms of advanced AMD (GA and wet AMD). The findings for rs11200638 appeared consistent over AMD subtypes (wet AMD: p**=**1.4×10⁻¹², dry AMD: p=8.6×10⁻⁹, soft confluent drusen p=1.1×10⁻³).

An association analyses based on genotypes at both rs11200638 and the CFH rs1061170 (Y402H) variant at chromosome 1q31 was undertaken and stratified according to different AMD phenotypes (soft confluent drusen, GA, and wet AMD). In a global two-locus analysis enumerating all nine two-locus genotype combinations, the association with AMD was significant (χ²=56.56, 8 df; p=2.2×10⁻⁹). FIG. 11 shows the risk estimates for each two-locus genotype combination compared to the baseline of no risk genotypes (TT at CFHY402H and GG at rs11200638). The association of rs11200638 to AMD was significant when analyzed conditional on the presence of the CFH C risk allele (p=5.9×10⁻⁸), and the association for rs11200638 in different AMD subtypes appeared to remain significant conditional on the genotype at CFH Y402H for the individuals that also carried the CFH C risk allele (See, Table 4, Table 5 and Table 6). In particular, this conditional analysis indicates an allele-dosage effect such that homozygotes for the A risk allele of rs11200638 are at an increased risk (ORₕₒₘ=7.29(3.18, 16.74)) over that of heterozygotes (ORₕₑₜ = 1.83(1.25,2.68)) in all AMD subjects, even when compared to a baseline that includes subjects who carry the risk genotypes at CFH. With an allele-dosage model, the estimated population attributable risk (PAR) for rs11200638 is 49.3%. Consistent with an additive effect, the estimated PAR from a joint model with CFH Y402H (that is, for a risk allele at either locus) is 71.4%.

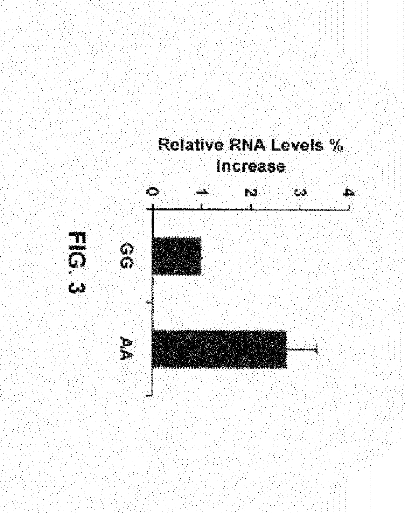

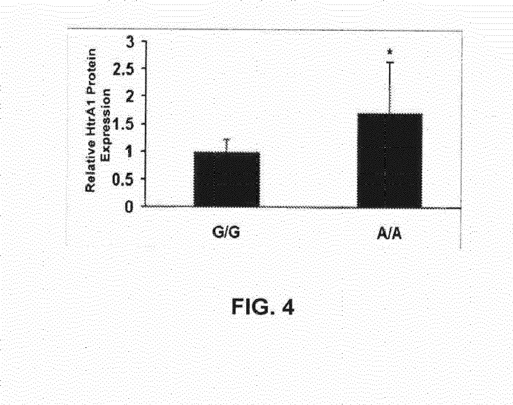

The SNP rs11200638 is located 512 bp upstream of the transcription start site of the HtrA1 gene (also known as *PRSS11*, *NM_002775*). Using MatInspector to scan putative transcription factor binding sites within this region, a conserved AP2/SRF binding cis element that is altered by the A risk allele was identified. To investigate the functional significance of the associated A risk allele, expression studies using real-time RT-PCR were used to study the expression levels of HtrA1 mRNA in lymphocytes of four AMD subjects carrying the risk allele AA and three normal controls carrying the normal allele GG. The HtrA1 mRNA levels in lymphocytes of AMD subjects with the AA genotype were approximately 2.7 fold higher than those in normal controls with the GG genotype (See, FIG. 3). The mean HtrA1 protein level in RPE of four AMD donor eyes with a homozygous AA risk allele were also 1.7 fold higher compared to that of six normal controls with a homozygous GG allele (See, FIG. 4). The data to date, therefore, indicate a trend toward higher expression of HtrA1 when the AA genotype is present.

Through immunohistochemistry experiments, it has been determined that that HtrA1 immunolabeling is present in the drusen of subjects suffering from AMD and that HtrA1 is non-uniformly expressed on drusen (See, FIG. 6, FIG. 12). Further, in subjects with AMD, the HtrA1 gene is expressed in the retina, internal limiting membrane, and RPE. These findings indicate, therefore, that HtrA1 expression promotes, facilitates or is otherwise associated with both the wet and dry forms of AMD.

It is thought that HtrA1 is an enzyme that plays a role in the regulation of the degradation of extracellular matrix proteoglycans, and such activity is thought to facilitate access of other degradative matrix enzymes such as collagenases and matrix metalloproteinases to their substrates. It is also believed that HtrA1 binds to and inhibits TGF-ß signaling, which is an important regulator of extracellular matrix deposition and angiogenesis.

Without being bound by the particular theories set forth herein, it is presently thought that increased expression of HtrA1 associated with the SNP rs11200638 may, for example, contribute to the formation of drusen through increased production of degradation products that result from degradation of extracellular matrix proteoglycans and by increased activity of other degradative matrix enzymes. In addition, such an increase in the presence of degradation products may contribute to pathologic neovascularization, vascular leak, and edema that leads to the wet form of AMD. Alternatively or in addition, HtrA1 may contribute to an inflammatory response or physiologic conditions, such as, for example, inhibition of TGF-ß signaling, that enable or promote angiogenesis and pathologic neovascularization, even where drusen are not present. Even further, it is possible that overexpression of HtrA1 alters the integrity of Bruch's membrane, favoring the invasion of choroid capillaries across the extracellular matrix, as occurs in wet AMD.

### Diagnostic & Prognostic Methods

The discovery that genetic mutations segregate with ocular disease allows for testing for susceptibility to ocular disease and pathologic conditions of the eye based on detecting mutations at the HtrA1 locus along, the CFH locus alone, or at both the HtrA1 locus and the CFH locus. Accordingly diagnostic and prognostic methods are described herein, wherein alteration of the wild-type HtrA1 locus is detected, alteration of the wild-type CFH locus is detected, or alteration of both the wild-type HtrA1 locus and the wild-type CFH locus is detected. "Alteration of a wild-type" gene or locus encompasses all forms of genetic variations and mutations including deletions, insertions and point mutations in the coding and non-coding regions. Deletions may be of the entire gene or of only a portion of the gene. Point mutations may result in stop codons, frameshift mutations or amino acid substitutions. Point mutations or deletions in the promoter can change transcription and thereby alter the gene function. Somatic mutations are those which occur only in certain tissues and are not inherited in the germline. Germline mutations can be found in any of a body's tissues and are inherited. The finding of HtrA1 and CFH germ line mutations thus provides diagnostic information.

The association of HtrA1 mutations to AMD in both its wet and dry forms and the correlation of SNP rs11200638 with increased expression of HtrA1 can be employed in methods of identifying subjects at risk for developing one or more ocular diseases or pathologic conditions of the eye, including, for example, AMD in both its wet and dry forms, ROP, DR, and macular edema. In one embodiment, such a method includes taking a sample from a subject and analyzing the sample for the presence or absence of an alteration from the wild-type HtrA1 gene, wherein the presence of such an alteration indicates an increased risk for developing an ocular disease or pathologic condition as described herein. In a particular embodiment, such a method includes taking a sample from a subject and analyzing the sample for the presence or absence of an alteration from the wild-type HtrA1 gene, wherein the presence of such an alteration indicates an increased risk for the formation of drusen, pathologic neovascularization, vascular leak, and edema in the tissues of the eye. In another embodiment, such a method includes taking a sample from the subject and analyzing the sample for the presence or absence of an alteration from the wild-type HtrA1 gene, wherein the presence of such a alteration indicates an increased risk for developing an ocular disease or condition selected from AMD in both its wet and dry forms, DR, ROP, ischemia-induced neovascularization, and macular edema. In particular embodiments of the methods for identifying subjects at risk for developing one or more diseases or pathologic conditions of the eye, as described herein, analyzing the sample for the presence or absence of an alteration from the wild-type HtrA1 gene may include analyzing the sample for the presence or absence of at least one SNP selected from, for example, rs11200638, rs1061170, rs10490924, rs3750848, rs3793917, rs932275, and combinations thereof, wherein the presence of the at least one SNP indicates an increased risk for developing an ocular disease selected from AMD in its dry and wet forms, ROP, and DR or a "pathologic condition selected from the formation of drusen, pathologic neovascularization, vascular leak, and edema in the tissues of the eye, such as, for example macular edema. In additional particular embodiments of the methods for identifying subjects at risk for developing one or more diseases or pathologic conditions of the eye as described herein, analyzing the sample for the presence or absence of a risk allele may include providing a polynucleotide probe or primer specific to a portion of the HtrA1 gene associated with targeted risk allele, exposing the sample taken from the subject to the polynucleotide probe or primer, and determining whether the polynucleotide probe or primer hybridizes to nucleic acids contained within the sample, wherein such hybridization indicates the presence of the targeted risk allele.

The association of CFH mutations to AMD in both its wet and dry forms can be employed in methods of identifying subjects at risk for developing one or more ocular diseases or pathologic conditions of the eye, including, for example, AMD in both its wet and dry forms, ROP, DR, and macular edema. In one embodiment, such a method includes taking a sample from a subject and analyzing the sample for the presence or absence of an alteration from the wild-type CFH gene, wherein the presence of such an alteration indicates an increased risk for developing an ocular disease or pathologic condition as described herein. In a particular embodiment, such a method includes taking a sample from a subject and analyzing the sample for the presence or absence of an alteration from the wild-type CFH gene, wherein the presence of such an alteration indicates an increased risk for the formation of drusen, pathologic neovascularization, vascular leak, and edema in the tissues of the eye. In another embodiment, such a method includes taking a sample from the subject and analyzing the sample for the presence or absence of an alteration from the wild-type CFH gene, wherein the presence of such a alteration indicates an increased risk for developing an eye disease or pathologic condition selected from AMD in both its wet and dry forms, DR, ROP, ischemia-induced neovascularization, and macular edema. In particular embodiments of the methods for identifying subjects at risk for developing one or more diseases or pathologic conditions of the eye, as described herein, analyzing the sample for the presence or absence of an alteration from the wild-type CFH gene may include analyzing the sample for the presence or absence of at least one SNP selected from, for example, rs1061170 (Y402H variant at 1q31 chromosome locus), rs529825, rs800292, rs1061147, rs203674, rs2274700, and combinations thereof, wherein the presence of the at least one SNP indicates an increased risk for developing an eye disease selected from AMD in its dry and wet forms, ROP, and DR or a pathologic condition selected from the formation of drusen, pathologic neovascularization, vascular leak, and edema in the tissues of the eye, such as, for example macular edema. In additional particular embodiments of the methods for identifying subjects at risk for developing one or more diseases of the eye, as described herein, analyzing the sample for the presence or absence of a risk allele may include providing a polynucleotide probe or primer specific to a portion of the CFH gene associated with targeted risk allele, exposing the sample taken from the subject to the polynucleotide probe or primer, and determining whether the polynucleotide probe or primer hybridizes to nucleic acids contained within the sample, wherein such hybridization indicates the presence of the targeted risk allele.

The methods of identifying subjects at risk for developing one or more ocular diseases or pathologic conditions of the eye as contemplated herein include methods wherein a sample is taken from a subject and such sample is analyzed for genetic variation at more than one locus. In one embodiment, such a method includes taking a sample from a subject and analyzing the sample for the presence or absence of an alteration from the wild-type HtrA1 and the presence or absence of an alteration from the wild-type CFH gene, wherein the presence of an alteration in one or both of the HtrA1 gene and the CFH gene indicates an increased risk for developing an eye disease or pathologic condition as described herein. In a particular embodiment, such a method includes taking a sample from a subject and analyzing the sample for the presence or absence of an alteration from the wild-type CFH gene and an alteration from the wild-type HtrA1 gene, wherein the presence of an alteration in one or both of the HtrA1 gene and the CFH gene indicates an increased risk for the formation of drusen, pathologic neovascularization, vascular leak, and edema in the tissues of the eye. In another embodiment, such a method includes taking a sample from the subject and analyzing the sample for the presence or absence of an alteration from the wild-type HtrA1 gene and an alteration from the wild-type CFH gene, wherein the presence of an alteration in one or both of the HtrA1 gene and the CFH gene indicates an increased risk for developing an eye disease or condition selected from AMD in both its wet and dry forms, DR, ROP, ischemia-induced neovascularization, and macular edema. In particular embodiments of the methods for identifying subjects at risk for developing one or more diseases or pathologic conditions of the eye, as described herein, analyzing the sample for the presence or absence of an alteration from the wild-type HtrA1 gene and an alteration from the wild-type CFH gene may include analyzing the sample for the presence or absence of at least one SNP selected from, for example, rs11200638, rs1061170, rs10490924, rs3750848, rs3793917, rs932275, rs1061170 (Y402H variant at 1q31 chromosome locus), rs529825, rs800292, rs1061147, rs203674, rs2274700, and combinations thereof, wherein the presence of the at least one SNP indicates an increased risk for developing an eye disease selected from AMD in its dry and wet forms, ROP, and DR or a pathologic ocular condition selected from the formation of drusen, pathologic neovascularization, vascular leak, and edema in the tissues of the eye, such as, for example macular edema. In additional particular embodiments of the methods for identifying subjects at risk for developing one or more diseases of the eye, as described herein, analyzing the sample for the presence or absence of a risk allele may include providing a polynucleotide probe or primer specific to a portion of the HtrA1 gene associated with targeted risk allele, providing a polynucleotide probe or primer specific to a portion of the CFH gene associated with targeted risk allele, exposing the sample taken from the subject to the polynucleotide probe(s) or primer(s), and determining whether the polynucleotide probe(s) or primer(s) hybridizes to nucleic acids contained within the sample, wherein such hybridization indicates the presence of at least one of the targeted risk alleles.

The association of HtrA1 mutations to AMD in both its wet and dry forms and the correlation of SNP rs11200638 with increased expression of HtrA1 can be employed in methods of diagnosing subjects suffering from one or more diseases or pathologic conditions of the eye, including, for example, AMD in both its wet and dry forms, ROP, DR, and macular edema. In one embodiment, such a method includes taking a sample from a subject and analyzing the sample for the presence or absence of an alteration from the wild-type HtrA1 gene, wherein the presence of such an alteration indicates the presence of an ocular disease or pathologic condition of the eye as described herein. In a particular embodiment, such a method includes taking a sample from a subject and analyzing the sample for the presence or absence of an alteration from the wild-type HtrA1 gene, wherein the presence of such an alteration indicates the presence of a condition selected from the formation of drusen, pathologic neovascularization, vascular leak, and edema in the tissues of the eye. In another embodiment, such a method includes taking a sample from the subject and analyzing the sample for the presence or absence of an alteration from the wild-type HtrA1 gene, wherein the presence of such a alteration indicates the presence of an ocular disease of pathologic condition of the eye selected from, for example, AMD in both its wet and dry forms, DR, ROP, ischemia-induced neovascularization, and macular edema. In particular embodiments of the methods for identifying subjects at risk for developing one or more ocular diseases or pathologic conditions of the eye, as described herein, analyzing the sample for the presence or absence of an alteration from the wild-type HtrA1 gene may include analyzing the sample for the presence or absence of at least one SNP selected from, for example, rs11200638, rs1061170, rs10490924, rs3750848. rs3793917, rs932275, and combinations thereof, wherein the presence of the at least one SNP indicates the presence of an ocular disease selected from, for example, AMD in its dry and wet forms, ROP, and DR or a condition selected from the formation of drusen, pathologic neovascularization, vascular leak, and edema in the tissues of the eye, such as, for example macular edema. In additional particular embodiments of the methods for identifying subjects at risk for developing one or more diseases or pathologic conditions of the eye, as described herein, analyzing the sample for the presence or absence of a risk allele may include providing a polynucleotide probe or primer specific to a portion of the HtrA1 gene associated with targeted risk allele, exposing the sample taken from the subject to the polynucleotide probe or primer, and determining whether the polynucleotide probe or primer hybridizes to nucleic acids contained within the sample, wherein such hybridization indicates the presence of the targeted risk allele.

The association of CFH mutations to AMD in both its wet and dry forms can be employed in methods of diagnosing subjects suffering from one or more diseases or "pathologic conditions of the eye, including, for example, AMD in both its wet and dry forms, ROP, DR, and macular edema. In one embodiment, such a method includes taking a sample from a subject and analyzing the sample for the presence or absence of an alteration from the wild-type CFH gene, wherein the presence of such an alteration indicates the presence of an eye disease or condition as described herein. In a particular embodiment, such a method includes taking a sample from a subject and analyzing the sample for the presence or absence of an alteration from the wild-type CFH gene, wherein the presence of such an alteration indicates the presence of a condition selected from the formation of drusen, pathologic neovascularization, vascular leak, and edema in the tissues of the eye. In another embodiment, such a method includes taking a sample from the subject and analyzing the sample for the presence or absence of an alteration from the wild-type CFH gene, wherein the presence of such a alteration indicates the presence of an ocular disease of pathologic condition of the eye selected from, for example, AMD in both its wet and dry forms, DR, ROP, ischemia-induced neovascularization, and macular edema. In particular embodiments of the methods for identifying subjects at risk for developing one or more ocular diseases or pathologic conditions of the eye, as described herein, analyzing the sample for the presence or absence of an alteration from the wild-type CFH gene may include analyzing the sample for the presence or absence of at least one SNP selected from, for example, for example, rs1061170 (Y402H variant at 1q31 chromosome locus), rs529825, rs800292, rs1061147, rs203674, rs2274700, and combinations thereof, wherein the presence of the at least one SNP indicates an increased risk for developing an eye disease selected from AMD in its dry and wet forms, ROP, and DR or a pathologic ocular condition selected from the formation of drusen, pathologic neovascularization, vascular leak, and edema in the tissues of the eye, such as, for example macular edema. In additional particular embodiments of the methods for identifying subjects at risk for developing one or more diseases of the eye, as described herein, analyzing the sample for the presence or absence of a risk allele may include providing a polynucleotide probe or primer specific to a portion of the CFH gene associated with targeted risk allele, exposing the sample taken from the subject to the polynucleotide probe or primer, and determining whether the polynucleotide probe or primer hybridizes to nucleic acids contained within the sample, wherein such hybridization indicates the presence of the targeted risk allele.

The methods of diagnosing one or more ocular diseases or pathologic conditions of the eye as contemplated herein include methods wherein a sample is taken from a subject and such sample is analyzed for genetic variation at more than one locus. In one embodiment, such a method includes taking a sample from a subject and analyzing the sample for the presence or absence of an alteration from the wild-type HtrA1 and the presence or absence of an alteration from the wild-type CFH gene, wherein the presence of an alteration in one or both of the HtrA1 gene and the CFH gene indicates the presence an eye disease or pathologic condition as described herein. In a particular embodiment, such a method includes taking a sample from a subject and analyzing the sample for the presence or absence of an alteration from the wild-type CFH gene and an alteration from the wild-type HtrA1 gene, wherein the presence of an alteration in one or both of the HtrA1 gene and the CFH gene indicates the presence of a condition selected from, for example, the formation of drusen, pathologic neovascularization, vascular leak, and edema in the tissues of the eye. In another embodiment, such a method includes taking a sample from the subject and analyzing the sample for the presence or absence of an alteration from the wild-type HtrA1 gene and an alteration from the wild-type CFH gene, wherein the presence of an alteration in one or both of the HtrA1 gene and the CFH gene indicates the presence of an eye disease or pathologic condition selected from, for example, AMD in both its wet and dry forms, DR, ROP, ischemia-induced neovascularization, and macular edema. In particular embodiments of the methods for diagnosing a subject suffering from one or more diseases or pathologic conditions of the eye, as described herein, analyzing the sample for the presence or absence of an alteration from the wild-type HtrA1 gene and an alteration from the wild-type CFH gene may include analyzing the sample for the presence or absence of at least one SNP selected from, for example, rs11200638, rs1061170, rs10490924, rs3750848, rs3793917, rs932275, rs1061170 (Y402H variant at 1q31 chromosome locus), rs529825, rs800292, rs1061147, rs203674, rs2274700, and combinations thereof, wherein the presence of the at least one SNP indicates, for example, the presence of an eye disease selected from AMD in its dry and wet forms, ROP, and DR or a pathologic ocular condition selected from the formation of drusen, pathologic neovascularization, vascular leak, and edema in the tissues of the eye, such as, for example macular edema. In additional particular embodiments of the methods for diagnosing subjects suffering from one or more diseases of the eye, as described herein, analyzing the sample for the presence or absence of a risk allele may include providing a polynucleotide probe or primer specific to a portion of the HtrA1 gene associated with targeted risk allele, providing a polynucleotide probe or primer specific to a portion of the CFH gene associated with targeted risk allele, exposing the sample taken from the subject to the polynucleotide probe(s) or primer(s), and determining whether the polynucleotide probe(s) or primer(s) hybridizes to nucleic acids contained within the sample, wherein such hybridization indicates the presence of at least one of the targeted risk alleles.

The present invention also relates to a method of detecting, in a sample obtained from a subject, an alteration of the HtrA1 gene that is correlated with the occurrence of AMD in humans. In one embodiment, such a method may comprise:
(a) combining the sample with a polynucleotide probe that hybridizes, under stringent conditions, to an alteration of the HtrA1 gene that is correlated with AMD in humans, but not to a wild type HtrA1 gene; and (b) determining whether hybridization occurs. The occurrence of hybridization indicates that an alteration of the HtrA1 gene that is correlated with AMD is present in the sample.

The present invention also relates to a method of detecting, in a sample obtained from a subject, an alteration of the CFH gene that is correlated with the occurrence of AMD in humans. In one embodiment, such a method may comprise:
(a) combining the sample with a polynucleotide probe that hybridizes, under stringent conditions, to an alteration of the CFH gene that is correlated with AMD in humans, but not to a wild type CFH gene; and (b) determining whether hybridization occurs. The occurrence of hybridization indicates that an alteration of the CFH gene that is correlated with AMD is present in the sample.

The present invention also relates to a method of detecting, in a sample obtained from a subject, an alteration of the HtrA1 gene that is correlated with the occurrence of AMD in humans and an alteration of the CFH gene that is correlated with the occurrence of AMD in humans and. In one embodiment, such a method may comprise: (a) combining the sample with (1) a polynucleotide probe that hybridizes, under stringent conditions, to an alteration of the HtrA1 gene that is correlated with AMD in humans, but not to a wild type HtrA1 gene, and (2) a polynucleotide probe that hybridizes, under stringent conditions, to an alteration of the CFH gene that is correlated with AMD in humans, but not to a wild type CFH gene; and (b) determining whether hybridization occurs with either or both of the polynucleotide probes. The occurrence of hybridization indicates that an alteration of the HtrA1 gene, an alteration of the CFH gene, or alterations of both genes correlated with AMD is or are present in the sample.

In particular embodiments of methods for diagnosing or determining a predisposition to ocular disease according to the present description, the methods include providing an isolated polynucleotide probe for the detection of an alteration in the HtrA1 gene, wherein the isolated polynucleotide probe comprises a nucleic acid molecule that specifically detects an alteration in the HtrA1 gene, such as, for example, a SNP that is selected from rs11200638, rs1061170. rs10490924, rs3750848, rs3793917, and rs932275 and is correlated with the occurrence of ocular disease or condition as described herein. In such an embodiment, the polynucleotide probe is placed in contact with a sample taken from the subject and the extent to which the probe hybridizes to nucleic acids present in the sample is determined.

In additional embodiments of methods for diagnosing or determining a predisposition to ocular disease according to the present description, the methods include providing an isolated polynucleotide probe for the detection of an alteration in the CFH gene, wherein the isolated polynucleotide probe comprises a nucleic acid molecule that specifically detects an alteration in the HtrA1 gene, such as, for example a SNP that is selected from rs1061170 (Y402H variant at 1q31 chromosome locus), rs529825, rs800292, rs1061147, rs203674, and rs2274700 and is correlated with the occurrence of ocular disease or condition as described herein. In such an embodiment, the polynucleotide probe is placed in contact with a sample taken from the subject and the extent to which the probe hybridizes to nucleic acids present in the sample is determined.

In another embodiment, a method of identifying or aiding in identifying an subject at risk for developing AMD, includes: (a) obtaining DNA from an subject; (b) analyzing the sample for one or more alterations in the HtrA1 gene and CFH gene sequences associated with AMD; and (c) determining whether or not a variation exists. The presence of the variation indicates that the subject is at risk for developing an ocular disease, as described herein, such as AMD in both its dry and wet forms.

Of course, methods for detecting an alteration to the HtrA1 gene or the CFH gene may be just one component or step in processes for evaluating a subject's risk for or predisposition to one or more ocular diseases, diagnosing the presence of one or more or "pathologic conditions. That is, as will be appreciated by those of ordinary skill in the art, other factors such as, for example, environmental conditions, the presence or absence of one or more other pathologic conditions, including disease (e.g., diabetes mellitus, cardiovascular disease, including high blood pressure) or injury may also contribute to a subject's diagnosis or assessment of risk for a particular disease of the eye or pathologic condition.

Probes/primers useful in diagnostic and prognostic testing are described herein. In one embodiment, such a probe is a substantially purified polynucleotide probe, which polynucleotide probe comprises a region of nucleotide sequence that hybridizes under stringent conditions to at least approximately 12, preferably 25, more preferably 40, 50 or 75 consecutive nucleotides of sense or antisense sequence of the HtrA1 locus or the CFH locus, including 5' and/or 3' untranslated regions. In specific embodiments, the polynucleotide probe further comprises a detectable label group attached thereto, wherein, for example, the label group is selected from amongst radioisotopes, fluorescent compounds, enzymes, and enzyme co-factors. The selection of probes and primers for diagnosis of a susceptibility of ocular disease (*i.e*., detection of HtrA1 mutations or CFH mutations) is within the capability of the skilled artisan apprised of the invention.

Useful diagnostic techniques include, but are not limited to fluorescent in situ hybridization (FISH), direct DNA sequencing, PFGE (pulsed-field gel electrophoresis) analysis, Southern blot analysis, single stranded conformation analysis (SSCA), RNase protection assay, allele-specific oligonucleotide (ASO), dot blot analysis and PCR-SSCP, as discussed in detail further below. Also useful is the technique of DNA microchip technology. In addition to the techniques described herein, similar and other useful techniques are also described in U.S. Patent Nos. 5,837,492 and 5,800,998.

Predisposition to ocular disease or pathology can be ascertained by testing any tissue of a subject for mutations of the HtrA1 gene, the CFH gene, or both the HtrA1 gene and the CFH gene. For example, a subject who has inherited a germline as described herein may be prone to developing ocular disease. This can be determined by testing DNA from any tissue of the subject's body. Most simply, blood can be drawn and DNA extracted from the cells of the blood. In addition, prenatal diagnosis can be accomplished by testing fetal cells, placental cells or amniotic cells for mutations of the HtrA1 gene, the CFH gene, or both the HtrA1 and the CFH gene. Alteration of a wild-type alleles, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. Another approach is the single-stranded conformation polymorphism assay (SSCA) (Orita, et al. (1989) Proc. Natl. Acad. Sci. USA 86:2776 2770). This method does not detect all sequence changes, especially if the DNA fragment size is greater than 200 bp, but can be optimized to detect most DNA sequence variation. The reduced detection sensitivity is a disadvantage, but the increased throughput possible with SSCA makes it an attractive, viable alternative to direct sequencing for mutation detection on a research basis. The fragments which have shifted mobility on SSCA gels are then sequenced to determine the exact nature of the DNA sequence variation. Other approaches based on the detection of mismatches between the two complementary DNA strands include clamped denaturing gel electrophoresis (CDGE) (Sheffield, V. C., et al. (1991) Am. J. Hum. Genet. 49:699 706), heteroduplex analysis (HA) (White, M. B., et al., (1992) Genomics 12:301 306) and chemical mismatch cleavage (CMC) (Grompe, M., et al., (1989) Proc. Natl. Acad. Sci. USA 86:5855 5892). None of the methods described above will detect large deletions, duplications or insertions, nor will they detect a regulatory mutation which affects transcription or translation of the protein. Other methods which can detect these classes of mutations such as a protein truncation assay or the asymmetric assay, detect only specific types of mutations and would not detect missense mutations. A review of currently available methods of detecting DNA sequence variation can be found in a recent review by Grompe, M., (1993) Nature Genetics 5:111 117. Once a mutation is known, an allele specific detection approach such as allele specific oligonucleotide (ASO) hybridization can be utilized to rapidly screen large numbers of other samples for that same mutation.

Detection of point mutations may be accomplished by molecular cloning of the targeted alleles and sequencing the allele(s) using techniques well known in the art. Alternatively, the gene sequences can be amplified directly from a genomic DNA preparation from the tissue or cells, using known techniques. The DNA sequence of the amplified sequences can then be determined.

There are well known methods for a more complete, yet still indirect, test for confirming the presence of a susceptibility allele. For example, the following methods may be used to confirm the presence of a susceptibility allele: 1) single-stranded conformation analysis (SSCA) (Orita, et al. (1989) Proc. Natl. Acad. Sci. USA 86:2776 2770); 2) denaturing gradient gel electrophoresis (DGGE) (Wartell, R. M., et al. (1990) Nucl. Acids Res. 18:2699 2705; Sheffield, V. C., et al. (1989) Proc. Natl. Acad. Sci. USA 86:232 236); 3) RNase protection assays (Finkelstein, J., et al. (1990) Genomics 7:167 172; Kinszler, K. W., et al. (1991) Science 251:1366 1370); 4) allele-specific oligonucleotides (ASOs) (Conner, B. J., et al. (1983) Proc. Natl. Acad. Sci. USA 80:278 282); 5) the use of proteins which recognize nucleotide mismatches, such as the E. coli mutS protein (Modrich, P. (1991) Ann. Rev. Genet. 25:229 253); and 6) allele-specific PCR (Rano & Kidd (1989) Nucl. Acids Res. 17:8392). For allele-specific PCR, primers are used which hybridize at their 3' ends to a particular HtrA1 of CFH mutation. If the particular mutation is not present, an amplification product is not observed. Amplification Refractory Mutation System (ARMS) can also be used, as disclosed in European Patent Application Publication No. 0332435 and in Newton, C. R., et al. (1989) Nucl. Acids Res. 17:2503 2516. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected subject for the presence of one or more mutations found in that subject. Other techniques for detecting insertions and deletions as known in the art can be used.

In the first three methods (SSCA, DGGE and RNase protection assay), a new electrophoretic band appears. SSCA detects a band which migrates differentially because the sequence change causes a difference in single-strand, intramolecular base pairing. RNase protection involves cleavage of the mutant polynucleotide into two or more smaller fragments. DGGE detects differences in migration rates of mutant sequences compared to wild-type sequences, using a denaturing gradient gel. In an allele-specific oligonucleotide assay, an oligonucleotide is designed which detects a specific sequence, and the assay is performed by detecting the presence or absence of a hybridization signal. In the mutS assay, the protein binds only to sequences that contain a nucleotide mismatch in a heteroduplex between mutant and wild-type sequences.

Mismatches, according to the present invention, are hybridized nucleic acid duplexes in which the two strands are not 100% complementary. Lack of total homology may be due to deletions, insertions, inversions or substitutions. Mismatch detection can be used to detect point mutations in the gene or in its mRNA product. While these techniques are less sensitive than sequencing, they are simpler to perform on a large number of tumor samples. An example of a mismatch cleavage technique is the RNase protection method. In the practice of the methods described herein, an RNase protection method involves the use of a labeled riboprobe which is complementary to the human wild-type gene coding sequence. The riboprobe and either mRNA or DNA isolated from the tumor tissue are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full length duplex RNA for the riboprobe and the mRNA or DNA. The riboprobe need not be the full length of the targeted mRNA or gene but can be a segment of either. If the riboprobe comprises only a segment of the targeted mRNA or gene, it may be desirable to use a number of such probes to screen the whole mRNA sequence for mismatches.

In similar fashion, DNA probes can be used to detect mismatches, through enzymatic or chemical cleavage (See, e.g., Cotton, et al. (1988) Proc. Natl. Acad. Sci. USA 85:4397 4401; Shenk, et al. (1975) Proc. Natl. Acad. Sci. USA 72:989; and Novack, et al. (1986) Proc. Natl. Acad. Sci. USA 83:586). Alternatively, mismatches can be detected by shifts in the electrophoretic mobility of mismatched duplexes relative to matched duplexes. See, e.g., Cariello (1988) Human Genetics 42:726. With either riboprobes or DNA probes, the cellular mRNA or DNA which might contain a mutation can be amplified using PCR before hybridization. Changes in DNA of, for example, the HtrA1 gene of the CFH gene can also be detected using Southern hybridization, especially if the changes are gross rearrangements, such as deletions and insertions.

DNA sequences of the genes referenced herein, which have been amplified by use of PCR, may also be screened using allele-specific probes. These probes are nucleic acid oligomers, each of which contains a region of a target sequence harboring a known mutation. For example, one oligomer may be about 30 nucleotides in length (although shorter and longer oligomers are also usable as well recognized by those of skill in the art), corresponding to a portion of the HtrA1 gene sequence or the CFH gene sequence. By use of a battery of such allele-specific probes, PCR amplification products can be screened to identify the presence of a previously identified mutation. Hybridization of allele-specific probes with amplified target sequences can be performed, for example, on a nylon filter. Hybridization to a particular probe under high stringency hybridization conditions indicates the presence of the same mutation in the tissue as in the allele-specific probe.

The technique of nucleic acid analysis via microchip technology is also applicable in the present context. In this technique, literally thousands of distinct oligonucleotide probes are built up in an array on a silicon chip. Nucleic acid to be analyzed is fluorescently labeled and hybridized to the probes on the chip. It is also possible to study nucleic acid-protein interactions using these nucleic acid microchips. Using this technique one can determine the presence of mutations, sequence the nucleic acid being analyzed, and/or one can measure expression levels of a gene of interest. The method is one of parallel processing of many, even thousands, of probes at once and can tremendously increase the rate of analysis. Several papers have been published which use this technique (See, *e.g.,* Hacia J G, et al. (1996) Nature Genetics 14:441 447; Shoemaker D D, et al. (1996) Nature Genetics 14:450 456; Chee, M., et al. (1996) Science 274:610 614; Lockhart D J, et al. (1996) Nature Biotechnology 14:1675 1680; DeRisi, J., et al. (1996) Nat. Genet. 14:457 460; Lipshutz R J, et al. (1995) BioTechniques 19:442 447). This method has already been used to screen subject for mutations in the breast cancer gene BRCA1 (Hacia, J G, et al. (1996) Nature Genetics 14:441 447). DNA microchip screening technology has also been reviewed in a news article in Chemical and Engineering News (Borman, S. (1996) Chemical & Engineering News, December 9 issue, pp. 42 43) and been the subject of an editorial (Nature Genetics, 1996; see also, Fodor, S. P. A. (1997) Science 277:393 395).

The most definitive test for mutations in a candidate locus is to directly compare genomic sequences from disease subjects with those from a control population. Alternatively, one could sequence messenger RNA after amplification, *e.g.,* by PCR, thereby eliminating the necessity of determining the exon structure of the candidate gene.

Mutations from disease subjects falling outside the coding region of a targeted gene can be detected by examining the non-coding regions, such as introns and regulatory sequences near or within the targeted gene. An early indication that mutations in non-coding regions are important can come from Northern blot experiments that reveal messenger RNA molecules of abnormal size or abundance in disease subjects as compared to control subjects.

Alteration of CFH or HtrA1 mRNA expression can be detected by any techniques known in the art. These include Northern blot analysis, PCR amplification and RNase protection. Diminished or increased mRNA expression indicates an alteration of the wild-type gene. Alteration of wild-type HtrA1 and CFH genes can also be detected by screening for alteration of wild-type proteins. For example, monoclonal antibodies immunoreactive with a targeted wild-type protein can be used to screen a sample. Lack of cognate antigen would indicate a mutation. Antibodies specific for products of mutant alleles could also be used to detect mutant HtrA1 gene and CFH gene products. Such immunological assays can be done in any convenient formats known in the art. These include Western blots, immunohistochemical assays and ELISA assays. Any means for detecting an altered protein can be used to detect alteration of wild-type HtrA1 and CFH genes. Functional assays, such as protein binding determinations, can be used. In addition, assays can be used which detect HtrA1 or CFH biochemical function. Finding a mutant gene product(s) indicates alteration of a wild-type gene.

The primer pairs of the present invention are useful for determination of the nucleotide sequence of a target allele using PCR. The pairs of single-stranded DNA primers can be annealed to sequences within or surrounding the target gene (e.g., the HtrA1 gene on chromosome 10 or the CFH gene on chromosome 1) in order to prime amplifying DNA synthesis of the target gene itself. A complete set of these primers allows synthesis of all of the nucleotides of the target gene coding sequences, i.e., the exons. The set of primers preferably allows synthesis of both intron and exon sequences. Allele-specific primers can also be used. Such primers anneal only to particular mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from target sequences or sequences adjacent to such sequences, except for the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the information available regarding the sequences of, for example, the HtrA1 gene and the CFH gene, the design of particular primers is well within the skill of the art.

The polynucleotide probes described herein are useful for a number of purposes. They can be used in Southern hybridization to genomic DNA and in the RNase protection method for detecting point mutations already discussed above. The probes can be used to detect PCR amplification products. They can also be used to detect mismatches with the target gene or mRNA using other techniques.

As described herein, the presence of an altered (or a mutant) HtrA1 gene, an altered CFH gene, or both an altered HtrA1 gene and an altered CFH gene can be correlated to an increased risk of ocular disease. In order to detect an specific gene mutation, a biological sample is prepared and analyzed for a difference between the sequence of the target allele being analyzed and the sequence of the wild-type allele. Mutant alleles can be initially identified by any of the techniques described above. The mutant alleles are then sequenced to identify the specific mutation of the particular mutant allele. Alternatively, mutant alleles can be initially identified by identifying mutant (altered) proteins, using conventional techniques. The mutant alleles are then sequenced to identify the specific mutation for each allele. The mutations, especially those which lead to an increased expression of the protein, may then be used for the diagnostic methods described herein.

With respect to the HtrA1 gene or locus and the CFH gene or locus, the present description employs definitions specifically set out herein and those commonly used in the art. Such definitions can be found in U.S. Patent Nos. 5,837,492; 5,800,998; 6,261,801; 6,274,720 and 6,274,376. Such definitions are employed herein unless indicated otherwise.

Large amounts of the polynucleotides described herein may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration into the genome) cultured mammalian or plant or other eukaryotic cell lines. The purification of nucleic acids produced by the methods of the present invention is described, e.g., in Sambrook et al., 1989 or Ausubel et al., 1992.

The polynucleotides of the present invention may also be produced by chemical synthesis, e.g., by the phosphoramidite method described by Beaucage and Caruthers, 1981 or the triester method according to Matteucci and Caruthers, 1981, and may be performed on commercial, automated oligonucleotide synthesizers. A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

Polynucleotide constructs prepared for introduction into a prokaryotic or eukaryotic host may comprise a replication system recognized by the host, and comprises the intended polynucleotide fragment encoding the desired polypeptide, preferably with transcription and translational initiation regulatory sequences operably linked to the polypeptide-encoding polynucleotide segment. Expression vectors may include, for example, an origin of replication or autonomously replicating sequence (ARS) and expression control sequences, a promoter, an enhancer and necessary processing information sites, such as ribosome-binding sites, RNA. splice sites, polyadenylation sites, transcriptional termination sequences, and mRNA stabilizing sequences. Secretion signals may also be included where appropriate, whether from a native protein or from other receptors or from secreted polypeptides of the same or related species. These secretion signals thereby allow the protein to cross and/or lodge in cell membranes, and thus attain its functional topology, or be secreted from the cell. Such vectors may be prepared by means of standard recombinant techniques well known in the art and discussed, for example, in Sambrook et al., 1989 or Ausubel et al. 1992.

An appropriate promoter and other necessary vector sequences will be selected so as to be functional in the host, and may include, when appropriate, those naturally associated with the gene(s) to be expressed. Examples of workable combinations of cell lines and expression vectors are described in Sambrook et al., 1989, or Ausubel et al., 1992; see also, e.g., Metzger et al., 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzeman et al., EP 73,675A. Appropriate non-native mammalian promoters might include the early and late promoters from SV40 (Fiers et al., 1978) or promoters derived from murine Moloney leukemia virus, mouse tumor virus, cytomegalovirus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences (See also, Enhancers and Eukaryotic Gene Expression, Cold Spring Harbor Press, Cold Spring Harbor, N.Y. (1983)).

While such expression vectors may replicate autonomously, they may also replicate by being inserted into the genome of the host cell, by methods well known in the art.

Expression and cloning vectors may contain a selectable marker, a gene encoding a protein necessary for survival or growth of a host cell transformed with the vector. The presence of this gene ensures growth of only those host cells bearing the cloning vehicle. Typical selection genes encode proteins that a) confer resistance to antibiotics or other toxic substances, *e.g*., ampicillin, neomycin, methotrexate, etc.; b) complement auxotrophic deficiencies; or c) supply critical nutrients not available from complex media, *e.g.,* the gene encoding D-alanine racemase for Bacilli. The choice of the proper selectable marker will depend on the host cell, and appropriate markers for different hosts are well known in the art.

The vectors containing the nucleic acids of interest can be transcribed in vitro, and the resulting RNA introduced into the host cell by well-known methods, *e.g*., by injection (See, Kubo et al., 1988), or the vectors can be introduced directly into host cells by methods well known in the art, which vary depending on the type of cellular host, including electroporation, transfection employing calcium chloride, rubidium chloride, calcium phosphate, DEAE-dextran, or other substances; microprojectile bombardment; lipofection; infection (where the vector is an infectious agent, such as a retroviral genome); and other methods. See generally, Sambrook et al., 1989 and Ausubel et al., 1992. The introduction of the polynucleotides into the host cell by any method known in the art, including, inter alia, those described above, will be referred to herein as "transformation." The cells into which nucleic acids described above have been introduced are meant to also include the progeny of such cells.

Large quantities of the nucleic acids and polypeptides of the present invention may be prepared by expressing the HtrA1 nucleic acids, CFH nucleic acids, combinations thereof or portions thereof in vectors or other expression vehicles in compatible prokaryotic or eukaryotic host cells. The most commonly used prokaryotic hosts are strains of Escherichia coli, although other prokaryotes, such as Bacillus subtilis or Pseudomonas may also be used.

Mammalian or other eukaryotic host cells, such as those of yeast, filamentous fungi, plant, insect, amphibian or avian species, may also be useful for production of the proteins of the present invention. Propagation of mammalian cells in culture is per se well known (See, Jakoby and Pastan, 1979). Examples of commonly used mammalian host cell lines are VERO and HeLa cells, Chinese hamster ovary (CHO) cells, and WI38, BHK, and COS cell lines. An example of a commonly used insect cell line is SF9. However, it will be appreciated by one of skill in the art that other cell lines may be appropriate, e.g., to provide higher expression, desirable glycosylation patterns, or other features.

Clones are selected by using markers, the choice of which depends on the mode of the vector construction. The marker may be on the same or a different DNA molecule, preferably the same DNA molecule. In prokaryotic hosts, the transformant may be selected, *e.g.*, by resistance to ampicillin, tetracycline or other antibiotics. Production of a particular product based on temperature sensitivity may also serve as an appropriate marker.

Prokaryotic or eukaryotic cells transformed with the polynucleotides of the present invention will be useful not only for the production of the nucleic acids and polypeptides of the present invention, but also, for example, in studying the characteristics of HtrA1 polypeptides.

Antisense polynucleotide sequences may be useful in preventing or diminishing the expression of the HtrA1 locus, the CFH locus, or both the HtrA1 locus and the CFH locus, as will be appreciated by those skilled in the art. For example, polynucleotide vectors containing all or a portion of the target locus or other sequences from the target region (particularly those flanking the target locus) may be placed under the control of a promoter in an antisense orientation and introduced into a cell. Expression of such an antisense construct within a cell will interfere with transcription and/or translation and/or replication of the target locus.

As used herein, a nucleic acid or fragment thereof has substantial identity with another if, when optimally aligned (with appropriate nucleotide insertions or deletions) with the other nucleic acid (or its complementary strand), there is nucleotide sequence identity selected from at least about 60% of the nucleotide bases, at least about 70% of the nucleotide bases, at least about 80% of the nucleotide bases, at least about 90% of the nucleotide bases, and at least about 95-98% of the nucleotide bases. A protein or fragment thereof has substantial identity with another if, optimally aligned, there is an amino acid sequence identity selected from at least about 30% identity with an entire naturally-occurring protein or a portion thereof, at least about 70% identity with an entire naturally-occurring protein or a portion thereof, at least about 80% identity with an entire naturally-occurring protein or a portion thereof, at least about 90% identity with an entire naturally-occurring protein or a portion thereof, and at least about 95% identity with an entire naturally-occurring protein or a portion thereof.

Identity, as used in this context, means the degree of sequence relatedness between two polypeptide or two polynucleotide sequences as determined by the identity of the match between two strings of such sequences, such as the full and complete sequence. Identity can be readily calculated. While there exist a number of methods to measure identity between two polynucleotide or polypeptide sequences, the term "identity" is well known to skilled artisans (See, e.g., Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991). Methods commonly employed to determine identity between two sequences include, but are not limited to, those disclosed in Guide to Huge Computers, Martin J. Bishop, ed., Academic Press, San Diego, 1994, and Carillo, H., and Lipman, D., SIAM J Applied Math. 48:1073 (1988). Preferred methods to determine identity are designed to give the largest match between the two sequences tested. Such methods are codified in computer programs. Computer program methods to determine identity between two sequences include, but are not limited to, GCG (Genetics Computer Group, Madison Wis.) program package (Devereux, J., et al., Nucleic Acids Research 12:387 (1984)), BLASTP, BLASTN, FASTA (Altschul, S. F. et al. (1990) J. Mol. Biol. 215:403 410; and Altschul, S. F. et al. (1997) Nucl. Acids Res. 25:3389 3402). The well-known Smith Waterman algorithm may also be used to determine identity.

As an illustration, by a polynucleotide having a nucleotide sequence having at least, for example, 95% "identity" to a reference nucleotide sequence, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence can occur at the 5' or 3' terminal positions of the reference nucleotide sequence or anywhere between those terminal positions, interspersed either individually among nucleotides in the reference sequence or in one or more contiguous groups within the reference sequence.

Alternatively, substantial homology or (similarity) exists when a nucleic acid or fragment thereof will hybridize to another nucleic acid (or a complementary strand thereof) under selective hybridization conditions, to a strand, or to its complement. Selectivity of hybridization exists when hybridization which is substantially more selective than total lack of specificity occurs. Typically, selective hybridization occurs when there is at least about 55% homology over a stretch of at least about 14 nucleotides, preferably at least about 65%, more preferably at least about 75%, and most preferably at least about 90%. The length of homology comparison, as described, can be over longer stretches, and in certain embodiments can be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Nucleic acid hybridization can be affected by such conditions as salt concentration, temperature, or organic solvents, in addition to the base composition, length of the complementary strands, and the number of nucleotide base mismatches between the hybridizing nucleic acids, as is readily appreciated by those skilled in the art. Stringent temperature conditions will generally include temperatures in excess of 30°C., typically in excess of 37°C.. and preferably in excess of 45°C. Stringent salt conditions will ordinarily be less than 1000 mM, typically less than 500 mM, and preferably less than 200 mM. However, the combination of parameters is much more important than the measure of any single parameter. The stringency conditions are dependent on the length of the nucleic acid and the base composition of the nucleic acid, and can be determined by techniques well known in the art (See, *e.g.,* Ausubel, F. M., et al. (1992) Current Protocols in Molecular Biology, (J. Wiley and Sons, NY); Wetmur, J. G. and Davidson, N. (1968) J. Mol. Biol. 31:349 370).

Thus, as herein used, the term "stringent conditions" means hybridization will occur only if there is at least 95% and preferably at least 97% identity between the sequences. Such hybridization techniques are well known to those of skill in the art. Stringent hybridization conditions are as defined above or, alternatively, conditions under overnight incubation at 42°C. in a solution comprising: 50% formamide, 5XSSC (150 mM NaCl, 15 mM trisodium citrate), 50 mM sodium phosphate (pH7.6), 5X Denhardt's solution, 10% dextran sulfate, and 20 µg/ml denatured, sheared salmon sperm DNA, followed by washing the filters in 0.1XSSC at about 65° C.

Large amounts of the nucleic acids as described herein can be produced by (a) replication in a suitable host or transgenic animals or (b) chemical synthesis using techniques well known in the art. Constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals can also be included where appropriate which allow the protein to cross and/or lodge in cell membranes, and thus attain its functional topology, or be secreted from the cell. Such vectors may be prepared by means of standard recombinant techniques well known in the art.

In order to detect the presence of an allele predisposing an subject to a disease or pathologic condition of the eye, a sample such as blood is prepared and analyzed for the presence or absence of predisposing alleles. Results of these tests and interpretive information are returned to the health care provider for communication to the tested subject. Such diagnoses can be performed by diagnostic laboratories, or, alternatively, diagnostic kits are manufactured and sold to health care providers or to private subjects for self-diagnosis. Diagnostic or prognostic tests can be performed as described herein or using well known techniques, such as described in U.S. Patent No. 5,800,998.

Initially, the screening method can involve amplification of the relevant HtrA1 sequences. In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

Additionally, target amplification is useful in this context. Here, the target nucleic acid sequence is amplified with a polymerase. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

When the probes are used to detect the presence of the target sequences (for example, in screening for ocular disease susceptibility), the biological sample to be analyzed, such as blood or serum, may be treated, if desired, to extract the nucleic acids. The sample nucleic acid can be prepared in various ways to facilitate detection of the target sequence, with such methods including, for example, denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence may need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 10. Therefore, high stringency conditions are desirable in order to prevent false positives. However, conditions of high stringency are used only if the probes are complementary to regions of the chromosome which are unique in the genome. The stringency of hybridization is determined by a number of factors during hybridization and during the washing procedure, including temperature, ionic strength, base composition, probe length, and concentration of formamide. These factors are outlined in, for example, Maniatis T., et al. (1982) Molecular Cloning: A Laboratory Manual (Cold Spring Harbor Laboratory, Cold Spring Harbor, N.Y.) and Sambrook, J., et al. (1989) Molecular Cloning: A Laboratory Manual, 2nd Ed. (Cold Spring Harbor Laboratory, Cold Spring Harbor, N.Y.). Under certain circumstances, the formation of higher order hybrids, such as triplexes, quadraplexes, etc., may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labeled moiety. A number of these variations are reviewed in, e.g., Matthews and Kricka (1988) Anal. Biochem. 169:1; Landegren, et al. (1988) Science 242:229; Mittlin (1989) Clinical Chem. 35:1819; U.S. Patent No. 4,868,105, and in EPO Publication No. 225,807.

As noted above, non-PCR based screening assays are also contemplated in this invention. In one embodiment, a nucleic acid probe (or an analog such as a methyl phosphonate backbone replacing the normal phosphodiester) is hybridized to the low level DNA target. The nucleic acid probe can have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in, for example, approximately a 10³ to 10⁶ increase in sensitivity. For an example relating to the preparation of oligodeoxynucleotide-alkaline phosphatase conjugates and their use as hybridization probes, see Jablonski, E., et al. (1986) Nuc. Acids Res. 14:6115 6128.

Two-step label amplification methodologies are also known in the art. These assays work on the principle that a small ligand (such as digoxigenin, biotin, or the like) is attached to a nucleic acid probe capable of specifically binding HtrA1. Allele specific probes are also contemplated within the scope of this example and exemplary allele specific probes include probes encompassing the predisposing or potentially predisposing mutations summarized in herein.

In one example, the small ligand attached to the nucleic acid probe is specifically recognized by an antibody-enzyme conjugate. In one embodiment of this example, digoxigenin is attached to the nucleic acid probe. Hybridization is detected by an antibody-alkaline phosphatase conjugate which turns over a chemiluminescent substrate. For methods for labeling nucleic acid probes according to this embodiment, see, e.g., Martin et al., 1990. In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays, see, e.g., Rigby, P. W. J., et al. (1977) J. Mol, Biol. 113:237 251 and Nguyen, Q., et al. (1992) BioTechniques 13:116 123.

It is also contemplated within the scope of this invention that the nucleic acid probe assays described herein may employ a cocktail of nucleic acid probes capable of detecting an alteration of the HtrA1 gene, the CFH gene, or both the HtrA1 gene and the CFH gene. Thus, in one example, to detect the presence of a gene alteration as described herein in a cell sample, more than one probe complementary to gene in which the alteration occurs is employed, and in particular, the number of different probes is alternatively 2, 3, or 5 different nucleic acid probe sequences. In an example, to detect the presence of one or more specific genetic alterations in a subject, more than one probe complementary to gene in which the alteration occurs is employed, wherein the cocktail includes probes capable of binding to allele-specific mutations identified in populations of subjects with alterations in gene in which the genetic alteration occurs. In such an embodiment, any number of probes can be used, and can preferably include probes corresponding to gene mutations identified as predisposing an subject to ocular disease or a pathologic condition of the eye. In specific embodiments, candidate probes contemplated within this disclosure include probes that include or are specific to a SNP, such as a SNP selected from, for example, rs11200638, rs1061170, rs10490924, rs3750848, rs3793917, rs932275, rs1061170 (Y402H variant at 1q31 chromosome locus), rs529825, rs800292, rs1061147, rs203674, and rs2274700.

Methods for assaying HtrA1 and CFH expression are also provided herein. Such methods may be used, for example for prognostic and diagnostic assays. In particular, methods for assaying HtrA1 and CFH expression by detection and quanitifcation of mRNA transcribed from, for example, the HtrA1 locus and the CFH locus, are known in the art. Moreover, anti-HtrA1 antibodies or anti-CFH antibodies may be used to detect and quantify the level of HtrA1 of CFH in a sample. Antibodies suitable for such a method include those described herein.

In one such embodiment HtrA1 antibodies are used to immunoprecipitate HtrA1 proteins or fragments of the HtrA1 protein from solution as well as react with HtrA1 peptides on Western or immunoblots of polyacrylamide gels. Diagnostic assays that utilize HtrA1 antibodies according to the present description and may be used to detect expression or over expression of HtrA1 include, for example, ELISA or western blot assays.

Various diagnostic assay techniques that are suitable for use with the HtrA1 antibodies and CFH antibodies described herein are known in the art and include, for example, *in-vivo* imaging assays, *in-vitro* competitive binding assays, direct or indirect sandwich assays, and immunoprecipitation assays conducted in either heterogeneous or homogeneous phases (See, *e.g.,* Zola, Monoclonal Antibodies: A Manual of Techniques, CRC Press, Inc. (1987) pp. 147-158). Where desired, the antibodies used in the diagnostic assays can be labeled with a detectable moiety, with the detectable moiety being capable of directly or indirectly producing a detectable signal. Examples of detectable moieties include radioisotopes, such as ³H, ¹⁴C, ³²P, ³⁵S, or ¹²5I, fluorescent or chemiluminescent compounds, such as fluorescein isothiocyanate, rhodamine, or luciferin, and enzymes, such as alkaline phosphatase, beta-galactosidase or horseradish peroxidase. Any method known in the art for conjugating the antibody to the detectable moiety may be employed, including, for example, those methods described by Hunter et al., Nature, 144:945 (1962); David et al., Biochemistry, 13:1014-1021 (1974); Pain et al., J. Immunol. Meth., 40:219-230 (1981); and Nygren, J. Histochem. and Cytochem., 30:407-412 (1982). Sandwich assays may also be used, and exemplary sandwich assays are described by David et al. in U.S. Patent Nos. 4,376,110 and 4,486,530. In another embodiment, HtrA1 antibodies are used to detect HtrA1 proteins and protein fragments in prepared tissue samples (e.g., paraffin or frozen tissue sections) using known immunological techniques. Once the level, extent and/or location of HtrA1 expression is determined, the results of such analysis can be used to determine a subject's predisposition to develop an ocular disease or pathological condition as described herein or to assist in diagnosing such disease or condition. In yet another embodiment, CFH antibodies are used to detect CFH proteins and protein fragments in prepared tissue samples (e.g., paraffin or frozen tissue sections) using known immunological techniques. Once the level, extent and/or location of CFH expression is determined, the results of such analysis can be used to determine a subject's predisposition to develop an ocular disease or pathological condition as described herein or to assist in diagnosing such disease or condition.

### Methods of Drug Screening

Polypeptides as described herein may also may be used to assess the binding of small molecule substrates and ligands in, for example, cells, cell-free preparations, chemical libraries, and natural product mixtures. These substrates and ligands may be natural substrates and ligands or may be structural or functional mimetics (See, e.g., Coligan et al., Current Protocols in Immunology 1(2):Chapter 5 (1991). Thus, the invention also provides a method of screening compounds to identify those which enhance (agonist) or block (antagonist) the action of HtrA1 or CFH polypeptides or polynucleotides, particularly those compounds for treating or preventing disease or pathologic conditions of the eye.

This invention is particularly useful for screening compounds by using a wild-type or mutant HtrA1 polypeptide or a binding fragment thereof in any of a variety of drug screening techniques. The individual components of the assays described herein are available commercially and/or can be produced by an ordinary skilled artisan. The screens of the invention are intended to encompass the use of HtrA1 homologs and HtrA1 interacting proteins from humans or a desired model organism. Drug screening can be performed as described herein or using well known techniques, such as described in U.S. Patent Nos. 5,800,998 and 5,891,628, each incorporated herein by reference. Preferably test compounds that disrupt HtrA1 bioactivity are tested in a secondary assay such as an animal model of an ocular disease or pathologic condition. Furthermore, as the skilled artisan readily understands, the screening assays described herein can be performed in a variety of configurations based on known HtrA1 biochemistry, including, but not limited to testing to disrupt of inhibit the function or activity or one or more of the IGFBP domain, trypsin domain, PDZ domain, SP domain, KI domain, Mac25 domain, or FS domain of an HtrA1 molecule.

The HtrA1 polypeptide or fragment employed in such a test can either be free in solution, affixed to a solid support, or borne on a cell surface. One method of drug screening utilizes eukaryotic or prokaryotic host cells which are stably transformed with recombinant polynucleotides expressing the polypeptide or fragment, preferably in competitive binding assays. Such cells, either in viable or fixed form, can be used for standard binding assays. One may measure, for example, for the formation of complexes between an HtrA1 polypeptide or fragment and the agent being tested, or examine the degree to which the interaction between an HtrA1 polypeptide or fragment and a known ligand or substrate is interfered with by the agent being tested.

Thus, the present invention provides methods of screening for drugs comprising contacting such an agent with an HtrA1 polypeptide or fragment thereof and assaying (i) for the presence of a complex between the agent and the HtrA1 polypeptide or fragment, or (ii) for the presence of a complex between the HtrA1 polypeptide or fragment and a ligand, or (iii) for interaction between HtrA1 and one or more substrate of HtrA1, by methods well known in the art. In competitive binding assays the HtrA1 polypeptide or fragment is typically labeled. Free HtrA1 polypeptide or fragment is separated from that present in a protein:protein complex, and the amount of free (i.e., uncomplexed) label is a measure of the binding of the agent being tested to HtrA1 or its interference with HtrA1:ligand binding, respectively.

Another technique for drug screening provides high throughput screening for compounds having suitable binding affinity to the HtrA1 polypeptides and is described in detail in Geysen, PCT published application WO 84/03564, published on Sep. 13, 1984. Briefly stated, large numbers of different small peptide test compounds are synthesized on a solid substrate, such as plastic pins or some other surface. The peptide test compounds are reacted with HtrA1 polypeptides and washed. Bound HtrA1 polypeptides are then detected by methods well known in the art.

Purified HtrA1 can be coated directly onto plates for use in the aforementioned drug screening techniques. However, non-neutralizing antibodies to the polypeptide can be used to capture antibodies to immobilize the HtrA1 polypeptide on the solid phase.

This invention also contemplates the use of competitive drug screening assays in which neutralizing antibodies capable of specifically binding the HtrA1 polypeptide compete with a test compound for binding to the HtrA1 polypeptide or fragments thereof. In this manner, the antibodies can be used to detect the presence of any peptide which shares one or more antigenic determinants of the HtrA1 polypeptide.

A further technique for drug screening involves the use of host eukaryotic cell lines or cells which express a wild-type or mutant HtrA1 gene and as a consequence of expression of wild type or mutant HtrA1 gene demonstrate a specific phenotype. The phenotype of the cells is examined to determine if the compound is capable of modulating the phenotype and thereby the pathologic conditions associated with HtrA1 expression or function.

Briefly, a method of screening for a substance which modulates activity of a polypeptide may include contacting one or more test substances with the polypeptide in a suitable reaction medium, testing the activity of the treated polypeptide and comparing that activity with the activity of the polypeptide in comparable reaction medium untreated with the test substance or substances. A difference in activity between the treated and untreated polypeptides is indicative of a modulating effect of the relevant test substance or substances.

Prior to or as well as being screened for modulation of activity, test substances may be screened for ability to interact with the polypeptide, e.g., in a yeast two-hybrid system (See, e.g., Bartel, P. L., et al. (1993) In: Cellular Interactions in Development: A Practical Approach, Oxford University Press, pp. 153 179; Fields, S. and Song, O-K. (1989) Nature 340:245 246; Chevray, P. M. and Nathans, D. N. (1992) Proc. Natl. Acad. Sci. USA 89:5789 5793; Lee, J. E., et al. (1995) Science 268:836 844). Such a system can be used as a coarse screen prior to testing a substance for actual ability to modulate activity of the polypeptide. Alternatively, the screen could be used to screen test substances for binding to an HtrA1 specific binding partner, or to find mimetics of an HtrA1 polypeptide.

In another embodiment, the invention provides methods of screening inhibitors of HtrA1 activity or an altered HtrA1 activity. In one aspect of this embodiment, the assays are configured to identify compounds that inhibit the activity or function of one or more of the IGFBP domain, trypsin domain, PDZ domain, SP domain, KI domain, Mac25 domain, or FS domain of an HtrA1 polypeptide.

Thus, test compounds can be screened in an *in-vitro* binding assay to identify compounds capable of binding or affecting a protein--protein interaction between HtrA1 (including homologues, derivatives, altered (mutant) forms or fragments thereof) and proteins with which it interacts. In addition, *in-vitro* dissociation assays may also be employed to select compounds capable of dissociating or destabilizing the protein complexes comprising HtrA1. An *in-vitro* screening assay can also be used to identify compounds that trigger or initiate the formation of, or stabilize, a protein complex of the present invention. In one embodiment, in vivo assays such as yeast two-hybrid assays and various derivatives thereof, preferably reverse two-hybrid assays, are utilized in identifying compounds that interfere with or disrupt protein--protein interactions between HtrA1 or a homologue, derivative, altered form, mutant, or fragment thereof and an interacting partner which it interacts. In addition, systems such as yeast two-hybrid assays are also useful in selecting compounds capable of triggering or initiating, enhancing or stabilizing protein--protein interactions between HtrA1 or a homologue, derivative, altered form, mutant, or fragment thereof and a protein with which it interacts. For example, the assays can entail (1) contacting the interacting members of the protein complex with each other in the presence of a test compound; and (2) detecting the interaction between the interacting members.

The test compounds may be screened in an *in-vitro* assay to identify compounds capable of binding the protein complexes or interacting protein members thereof in accordance with the present invention. For this purpose, a test compound is contacted with a protein complex or an interacting protein member thereof under conditions and for a time sufficient to allow specific interaction between the test compound and the target components to occur, thereby resulting in the binding of the compound to the target, and the formation of a complex. Subsequently, the binding event is detected. Various screening techniques known in the art may be used in the present invention. The protein complexes and the interacting protein members thereof may be prepared by any suitable methods, e.g., by recombinant expression and purification. The protein complexes and/or interacting protein members thereof (both are referred to as "target" hereinafter in this section) may be free in solution. A test compound may be mixed with a target forming a liquid mixture. The compound may be labeled with a detectable marker. Upon mixing under suitable conditions, the binding complex having the compound and the target can be co-immunoprecipitated and washed. The compound in the precipitated complex can be detected based on the marker on the compound.

In a preferred embodiment, the target is immobilized on a solid support or on a cell surface. Preferably, the target can be arrayed into a protein microchip according to methods well-known in the art. For example, a target may be immobilized directly onto a microchip substrate such as glass slides or onto multi-well plates using non-neutralizing antibodies, i.e., antibodies capable of binding to the target but do not substantially affect its biological activities. To affect the screening, test compounds can be contacted with the immobilized target to allow binding to occur to form complexes under standard binding assay conditions. Either the targets or test compounds are labeled with a detectable marker using well-known labeling techniques. For example, U.S. Patent No. 5,741,713 discloses combinatorial libraries of biochemical compounds labeled with NMR active isotopes. To identify binding compounds, one may measure the formation of the target-test compound complexes or kinetics for the formation thereof. When combinatorial libraries of organic non-peptide non-nucleic acid compounds are screened, it is preferred that labeled or encoded (or "tagged") combinatorial libraries are used to allow rapid decoding of lead structures. This is especially important because, unlike biological libraries, individual compounds found in chemical libraries cannot be amplified by self-amplification. Tagged combinatorial libraries are provided in, e.g., Borchardt and Still, J. Am. Chem. Soc., 116:373 374 (1994) and Moran et al., J. Am. Chem. Soc., 117:10787 10788 (1995), both of which are incorporated herein by reference.

Alternatively, the test compounds can be immobilized on a solid support, e.g., forming a microarray of test compounds. The target protein or protein complex is then contacted with the test compounds. The target can be labeled with any suitable detection marker. For example, the target can be labeled with radioactive isotopes or fluorescence marker before binding reaction occurs. Alternatively, after the binding reactions, antibodies that are immunoreactive with the target and are labeled with radioactive materials, fluorescence markers, enzymes, or labeled secondary anti-Ig antibodies can be used to detect any bound target thus identifying the binding compound. One example of this embodiment is the protein probing method. That is, the target provided in accordance with the present invention is used as a probe to screen expression libraries of proteins or random peptides. The expression libraries can be phage display libraries, in vitro translation-based libraries, or ordinary expression cDNA libraries. The libraries may be immobilized on a solid support such as nitrocellulose filters (See, e.g., Sikela and Hahn, Proc. Natl. Acad. Sci. USA, 84:3038 3042 (1987)). The probe can be labeled with a radioactive isotope or a fluorescence marker. Alternatively, the probe can be biotinylated and detected with a streptavidin-alkaline phosphatase conjugate. More conveniently, the bound probe can be detected with an antibody.

In yet another embodiment, a known ligand capable of binding to the target can be used in competitive binding assays. Complexes between the known ligand and the target can be formed and then contacted with test compounds. The ability of a test compound to interfere with the interaction between the target and the known ligand is measured. One exemplary ligand is an antibody capable of specifically binding the target. Particularly, such an antibody is especially useful for identifying peptides that share one or more antigenic determinants of the target protein complex or interacting protein members thereof.

In a specific embodiment, a protein complex used in the screening assay includes a hybrid protein which is formed by fusion of two interacting protein members or fragments or interaction domains thereof. The hybrid protein can also be designed such that it contains a detectable epitope tag fused thereto. Suitable examples of such epitope tags include sequences derived from, e.g., influenza virus hemagglutinin (HA), Simian Virus 5 (V5), polyhistidine (6.times.His), c-myc, lacZ, GST, and the like.

Test compounds may also be screened in *in-vitro* assays to identify compounds capable of dissociating the protein complexes identified in accordance with the present description. Thus, for example, dissociation of a protein complex comprising HtrA1 and at least one interacting partner following treatment with a test compound can be detected. Conversely, test compounds may also be screened to identify compounds capable of enhancing the interaction between HtrA1 and at least one interacting partner or stabilizing the protein complex formed by the proteins.

The assay can be conducted in similar manners as the binding assays described above. For example, the presence or absence of a particular protein complex can be detected by an antibody selectively immunoreactive with the protein complex. Thus, after incubation of the protein complex with a test compound, an immunoprecipitation assay can be conducted with the antibody. If the test compound disrupts the protein complex, then the amount of immunoprecipitated protein complex in this assay will be significantly less than that in a control assay in which the same protein complex is not contacted with the test compound. Similarly, two proteins the interaction between which is to be enhanced can be incubated together with a test compound. Thereafter, a protein complex can be detected by the selectively immunoreactive antibody. The amount of protein complex can be compared to that formed in the absence of the test compound. Various other detection methods can be suitable in the dissociation assay, as will be apparent to a skilled artisan apprised of the present disclosure.

Protein complexes comprising HtrA1 and an interacting partner can be used in screening assays to identify modulators of protein complexes comprising HtrA1. In addition, mutants, homologues, derivatives or fragments of HtrA1 and protein complexes containing such homologues, derivatives, mutants, or fragments may also be used in such screening assays. As used herein, the term "modulator" encompasses any compounds that can cause any form of alteration of the biological activities or functions of the proteins or protein complexes, including, e.g., enhancing or reducing their biological activities, increasing or decreasing their stability, altering their affinity or specificity to certain other biological molecules, etc. In addition, the term "modulator" as used herein also includes any compounds that simply bind HtrA1, mutant HtrA1, and/or the proteins complexes of the present invention. For example, a modulator can be an antagonist capable of interfering with or disrupting or dissociating protein--protein interaction between HtrA1 or a homologue, fragment or derivative thereof. A modulator can also be an agonist that initiates or strengthens the interaction between the protein members of the protein complex of the present invention, or homologues, fragments, mutants, or derivatives thereof.

The modulators selected in accordance with the screening methods of the present invention can be effective in modulating the functions or activities of HtrA1 alone, or the protein complexes comprising HtrA1 or mutant HtrA1. For example, compounds capable of binding the protein complexes can be capable of modulating the functions of the protein complexes. Additionally, compounds that interfere with, weaken, dissociate or disrupt, or alternatively, initiate, facilitate or stabilize the protein--protein interaction between the interacting protein members of the protein complexes can also be effective in modulating the functions or activities of the protein complexes. Thus, the compounds identified in the screening methods of the present invention can be made into therapeutically or prophylactically effective drugs for treating diseases or pathologic conditions caused by or associated with protein complexes comprising HtrA1. Alternatively, they can be used as leads to aid the design and identification of therapeutically or prophylactically effective compounds for diseases, disorders or symptoms caused by or associated with protein complexes comprising HtrA1. The protein complexes and/or interacting protein members thereof in accordance with the present invention can be used in any of a variety of drug screening techniques. Drug screening can be performed as described herein or using well-known techniques, such as those described in U.S. Patent Nos. 5,800,998 and 5,891,628.

Similarly, cells and animals which carry a mutant HtrA1 allele can be used as model systems to study and test for substances which have potential as therapeutic agents. These can be isolated from subjects with HtrA1 locus mutations, either somatic or germline. Alternatively, the cell line can be engineered to carry the mutation in the HtrA1 allele, as described above. After a test substance is applied to the cells, the phenotype of the cell is determined. Any trait of the transformed cells can be assessed using techniques well known in the art. Transformed hosts, transgenic/knockout animals and models are prepared and used as described herein or using well known techniques, such as those described in U.S. Patent Nos. 5,800,998 and 5,891,628.

Animals for testing therapeutic agents can be selected after mutagenesis of whole animals or after treatment of germline cells or zygotes. Such treatments include insertion of mutant HtrA1 alleles, usually from a second animal species, as well as insertion of disrupted homologous genes. Alternatively, the endogenous HtrA1 gene(s) of the animals can be disrupted by insertion or deletion mutation or other genetic alterations using conventional techniques (See, Capecchi, M. R. (1989) Science 244:1288; Valancius and Smithies, 1991; Hasty, P., K., et al. (1991) Nature 350:243; Shinkai, Y., et al. (1992) Cell 68:855; Mombaerts, P., et al. (1992) Cell 68:869; Philpott, K. L, et al. (1992) Science 256:1448; Snouwaert, J. N., et al. (1992) Science 257:1083; Donehower, L. A., et al. (1992) Nature 356:215) to produce knockout or transplacement animals. A transplacement is similar to a knockout because the endogenous gene is replaced, but in the case of a transplacement the replacement is by another version of the same gene. Methods for producing knockout animals are described generally by Shastry (Shastry et al. (1995) Experientia 51:1028 1039; Shastry et al. (1998) Mol. Cell. Biochem. 181:163 179) and Osterrieder and Wolf (1998) Rev. Sci. Tech. 17:351 364. The production of conditional knockout animals, in which the gene is active until knocked out at the desired time is generally described by Feil et al., (1996) Proc. Natl. Acad. Sci. USA 93:10887 10890; Gagneten et al. (1997) Nucl. Acids Res. 25:3326 3331; and Lobe & Nagy (1998) Bioessays 20:200 208. Transgenic animals and cell lines derived from such animals can find use in certain testing experiments. In this regard, transgenic animals and cell lines capable of expressing wild-type or a mutant HtrA1 locus can be exposed to test substances. These test substances can be screened for the ability to alter the expression or function of HtrA1 as expressed by a mutant HtrA1 locus.

### Agents For Modulating HtrA1 activity and CFH Activity

Biologically active molecules capable of modulating HtrA1 activity are provided herein. A substance identified as a modulator of polypeptide function can be peptide or non-peptide in nature. Non-peptide "small molecules" are often preferred for many *in-vivo* pharmaceutical uses, and a small molecule mimetic or mimic of a biologically active substance, particularly if such substance is relatively large peptide or nucleic acid, may be designed for pharmaceutical use. In a particular embodiment, a small molecule for modulating HtrA1 activity provides, for example, one or more biological activity exhibited by the antibodies described herein In one embodiment, the biologically active molecule capable of modulating HtrA1 activity as described herein, directly or indirectly inhibits or blocks the activity or function of one or more of the IGFBP domain, trypsin domain, PDZ domain, SP domain, KI domain, Mac25 domain, or FS domain of an HtrA1 polypeptide. In one such embodiment, the biologically active molecule may be selected from antibodies, nucleic acids, such as RNAi compounds, a molecule that inhibits or blocks HtrA1 protein binding to TGF-ß, a molecule that stimulates TGF-ß, biologically active small molecules, and any combination thereof.

Biologically active molecules capable of modulating CFH activity are also described. A substance identified as a modulator of polypeptide function can be peptide or non-peptide in nature. Again, small molecules are often preferred for in-*vivo* pharmaceutical uses, and a small molecule mimetic or mimic of a biologically active substance, particularly if such substance is relatively large peptide or nucleic acid, may be designed for pharmaceutical use. In one embodiment, the biologically active molecule capable of modulating CFH activity as described herein, directly or indirectly inhibits, for example, CFH binding to complement 3b.

### Nucleic Acid, Ribozyme & Antisense Agents

Compounds which may exhibit anti-HtrA1 activity or anti-CFH activity include antisense, siRNA, ribozyme, and triple helix molecules. Such molecules may be designed to reduce or inhibit HtrA1 or CFH activity or expression. Techniques for the production and use of such molecules are known to those of skill in the art, such as described herein or in U.S. Patent No. 5,800,998, incorporated herein by reference.

Antisense RNA and DNA molecules act to directly block the translation of mRNA by binding to targeted mRNA and preventing protein translation. With respect to antisense DNA, oligodeoxyribonucleotides derived from the translation initiation site, e.g., between the -10 and +10 regions of the target sequence are preferred.

In one embodiment, the inhibitors of cellular levels of HtrA1 are double-stranded small interfering RNA (siRNA) compounds or a modified equivalent thereof. In another embodiment, the inhibitors of cellular levels of CFH are double-stranded small interfering RNA (siRNA) compounds or a modified equivalent thereof. The skilled artisan, apprised of this disclosure, is capable of providing siRNA useful for reducing the levels of a target protein or mutant thereof (e.g., an HtrA1 protein, a CFH protein, or mutants thereof).

As is generally known in the art, siRNA compounds are RNA duplexes comprising two complementary single-stranded RNAs of 21 nucleotides that form 19 base pairs and possess 3' overhangs of two nucleotides (See, Elbashir et al., Nature 411:494 498 (2001); and PCT Publication Nos. WO 00/44895; WO 01/36646; WO 99/32619; WO 00/01846; WO 01/29058; WO 99/07409; and WO 00/44914). When appropriately targeted via its nucleotide sequence to a specific mRNA in cells, a siRNA can specifically suppress gene expression through a process known as RNA interference (RNAi) (See, e.g., Zamore & Aronin, Nature Medicine, 9:266 267 (2003)). siRNAs can reduce the cellular level of specific mRNAs, and decrease the level of proteins coded by such mRNAs. siRNAs utilize sequence complementarity to target an mRNA for destruction, and are sequence-specific. Thus, they can be highly target-specific, and in mammals have been shown to target mRNAs encoded by different alleles of the same gene. Because of this precision, side effects typically associated with traditional drugs may be reduced or eliminated. In addition, they are relatively stable, and like antisense and ribozyme molecules, they can also be modified to achieve improved pharmaceutical characteristics, such as increased stability, deliverability, and ease of manufacture. Moreover, because siRNA molecules take advantage of a natural cellular pathway, *i.e*., RNA interference, they are highly efficient in destroying targeted mRNA molecules. As a result, it is relatively easy to achieve a therapeutically effective concentration of an siRNA compound in patients. Thus, siRNAs are a new class of drugs being actively developed by pharmaceutical companies.

*In-vivo* inhibition of specific gene expression by RNAi has been achieved in various organisms including mammals. For example, Song et al., Nature Medicine, 9:347 351 (2003) demonstrate that intravenous injection of Fas siRNA compounds into laboratory mice with autoimmune hepatitis specifically reduced Fas mRNA levels and expression of Fas protein in mouse liver cells. The gene silencing effect persisted without diminution for 10 days after the intravenous injection. The injected siRNA was effective in protecting the mice from liver failure and fibrosis. Song et al., Nature Medicine, 9:347 351 (2003). Several other approaches for delivery of siRNA into animals have also proved to be successful (See, e.g., McCaffery et al., Nature, 418:38 39 (2002); Lewis et al., Nature Genetics, 32:107 108 (2002); and Xia et al., Nature Biotech., 20:1006 1010 (2002)).

The siRNA compounds provided according to the present description can be synthesized using conventional RNA synthesis methods. For example, they can be chemically synthesized using appropriately protected ribonucleoside phosphoramidites and a conventional DNA/RNA synthesizer. Various applicable methods for RNA synthesis are disclosed in, e.g., Usman et al., J. Am. Chem. Soc., 109:7845 7854 (1987) and Scaringe et al., Nucleic Acids Res., 18:5433 5441 (1990). Custom siRNA synthesis services are available from commercial vendors such as Ambion (Austin, Tex., USA), Dharmacon Research (Lafayette, Colo., USA), Pierce Chemical (Rockford, III., USA), ChemGenes (Ashland, Mass., USA), Proligo (Hamburg, Germany), and Cruachem (Glasgow, UK).

The siRNA compounds may also be various modified equivalents of the structures in of any HtrA1 siRNA or any CFH siRNA. As used herein, "modified equivalent" means a modified form of a particular siRNA compound having the same target-specificity (i.e., recognizing the same mRNA molecules that complement the unmodified particular siRNA compound). Thus, a modified equivalent of an unmodified siRNA compound can have modified ribonucleotides, that is, ribonucleotides that contain a modification in the chemical structure of an unmodified nucleotide base, sugar and/or phosphate (or phospodiester linkage). As is known in the art, an "unmodified ribonucleotide" has one of the bases adenine, cytosine, guanine, and uracil joined to the 1' carbon of beta-D-ribo-furanose.

Modified siRNA compounds may contain modified backbones or non-natural internucleoside linkages, *e.g*., modified phosphorous-containing backbones and non-phosphorous backbones such as morpholino backbones; siloxane, sulfide, sulfoxide, sulfone, sulfonate, sulfonamide, and sulfamate backbones; formacetyl and thioformacetyl backbones; alkene-containing backbones; methyleneimino and methylenehydrazino backbones; amide backbones, and the like.

Examples of modified phosphorous-containing backbones include, but are not limited to phosphorothioates, phosphorodithioates, chiral phosphorothioates, phosphotriesters, aminoalkylphosphotriesters, alkyl phosphonates, thionoalkylphosphonates, phosphinates, phosphoramidates, thionophosphoramidates, thionoalkylphosphotriesters, and boranophosphates and various salt forms thereof (See, *e.g.*, U.S. Patent Nos. 3,687,808; 4,469,863; 4,476,301; 5,023,243; 5,177,196; 5,188,897; 5.264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050).

Examples of the non-phosphorous containing backbones described above are disclosed in, e.g., U.S. Patent Nos. 5,034,506; 5,185,444; 5,214,134; 5,216,141; 5,235,033; 5,264,562; 5,264,564; 5,405,938; 5,434,257; 5,470,967; 5,489,677; 5,541,307; 5,561,225; 5,596,086; 5,610,289; 5,602,240; 5,608,046; 5,610,289; 5,618,704; 5,623,070; 5,663,312; 5,677,437; and 5,677,439.

Modified forms of siRNA compounds can also contain modified nucleosides (nucleoside analogs), i.e., modified purine or pyrimidine bases, e.g., 5-substituted pyrimidines, 6-azapyrimidines, pyridin-4-one, pyridin-2-one, phenyl, pseudouracil, 2,4,6-trimethoxy benzene, 3-methyl uracil, dihydrouridine, naphthyl, aminophenyl, 5-alkylcytidines (e.g., 5-methylcytidine), 5-alkyluridines (e.g., ribothymidine), 5-halouridine (e.g., 5-bromouridine) or 6-azapyrimidines or 6-alkylpyrimidines (e.g. 6-methyluridine), 2-thiouridine, 4-thiouridine, 5(carboxyhydroxymethyl)uridine, 5'-carboxymethylaminomethyl-2-thiouridine, 5-carboxymethylaminomethyluridine, 5-methoxyaminomethyl-2-thiouridine, 5-methylaminomethyluridine, 5-methylcarbonylmethyluridine, 5-methyloxyuridine, 5-methyl-2-thiouridine, 4-acetylcytidine, 3-methylcytidine, propyne, quesosine, wybutosine, wybutoxosine, beta-D-galactosylqueosine, N-2, N-6 and O-substituted purines, inosine, 1-methyladenosine, 1-methylinosine, 2,2-dimethylguanosine, 2-methyladenosine, 2-methylguanosine, N6-methyladenosine, 7-methylguanosine, 2-methylthio-N-6-isopentenyladenosine, beta-D-mannosylqueosine, uridine-5-oxyacetic acid, 2-thiocytidine, threonine derivatives, and the like (See, *e.g*., U.S. Patent Nos. 3,687,808; 4,845,205; 5,130,302; 5,175,273; 5,367,066; 5,432,272; 5,459,255; 5,484,908; 5,502,177; 5,525,711; 5,587,469; 5,594,121; 5,596,091; 5,681,941; and 5,750,692, PCT Publication No. WO 92/07065; PCT Publication No. WO 93/15187; and Limbach et al., Nucleic Acids Res., 22:2183 (1994)).

In addition, modified siRNA compounds can also have substituted or modified sugar moieties, *e*.*g*., 2'-O-methoxyethyl sugar moieties. See e.g., U.S. Patent Nos. 4,981,957; 5,118,800; 5,319,080; 5,393,878; 5,446,137; 5,466,786; 5,514,785; 5,567,811; 5,576,427; 5,591,722; 5,610,300; 5,627,0531 5,639,873; 5,646,265; 5,658,873; 5,670,633; and 5,700,920.

Modified siRNA compounds may be synthesized by the methods disclosed in, *e.g*., U.S. Patent No. 5,652,094; International Publication Nos. WO 91/03162; WO 92/07065 and WO 93/15187; European Patent Application No. 92110298.4; Perrault et al., Nature, 344:565 (1990); Pieken et al., Science, 253:314 (1991); and Usman and Cedergren, Trends in Biochem. Sci., 17:334 (1992).

The 3' overhangs of the siRNAs as described herein may be modified to provide resistance to cellular nucleases. In one embodiment, the 3' overhangs comprise 2'-deoxyribonucleotides. In another embodiment these 3' overhangs comprise a dinucleotide made of two 2`-deoxythymine residues (*i.e*., dTdT) linked by a 5'-3' phosphodiester linkage.

siRNA compounds may be administered to a subject by various methods through different routes. For example, they can be administered by intravenous injection (See, Song et al., Nature Medicine, 9:347 351 (2003)). They can also be delivered directly to a particular organ or tissue by any suitable localized administration methods. Several other approaches for delivery of siRNA into animals have also proved to be successful (See, *e.g.*, McCaffery et al., Nature, 418:38 39 (2002); Lewis et al., Nature Genetics, 32:107 108 (2002); and Xia et al., Nature Biotech., 20:1006 1010 (2002)). Alternatively, siRNA compounds may be delivered encapsulated in liposome's, by iontophoresis, or by incorporation into other vehicles such as hydrogels, cyclodextrins, biodegradable nanocapsules, and bioadhesive microspheres.

In addition, siRNA compounds can also be delivered via a gene therapy approach, using a DNA vector from which siRNA compounds in, *e.g.*, small hairpin form (shRNA), can be transcribed directly. Recent studies have demonstrated that while double-stranded siRNAs are very effective at mediating RNAi, short, single-stranded, hairpin-shaped RNAs can also mediate RNAi, presumably because they fold into intramolecular duplexes that are processed into double-stranded siRNAs by cellular enzymes (See, *e.g.,* Sui et al., Proc. Natl. Acad. Sci. U.S.A., 99:5515 5520 (2002); Yu et al., Proc. Natl. Acad. Sci. U.S.A., 99:6047 6052 (2002); and Paul et al., Nature Biotech., 20:505 508 (2002)). This discovery has significant and far-reaching implications, since the production of such shRNAs can be readily achieved *in-vivo* by transfecting cells or tissues with DNA vectors bearing short inverted repeats separated by a small number of (e.g., 3 to 9) nucleotides that direct the transcription of such small hairpin RNAs. Additionally, if mechanisms are included to direct the integration of the transcription cassette into the host cell genome, or to ensure the stability of the transcription vector, the RNAi caused by the encoded shRNAs, can be made stable and heritable. Not only have such techniques been used to "knock down" the expression of specific genes in mammalian cells, but they have been successfully employed to knock down the expression of exogenously expressed transgenes, as well as endogenous genes in the brain and liver of living mice (See generally, Hannon, Nature. 418:244 251 (2002) and Shi, Trends Genet., 19:9 12 (2003); see also Xia et al., Nature Biotech., 20:1006 1010 (2002)).

Additional siRNA compounds targeted at different sites of the mRNA corresponding to HtrA1 of CFH can also be designed and synthesized according to general guidelines provided herein and generally known to skilled artisans (See, *e.g*., Elbashir, et al. (Nature 411: 494 498 (2001)). For example, guidelines have been compiled into "The siRNA User Guide," which is available at the following web address: www.mpibpc.gwdg.de/abteilungen/100/105/sima.html.

Additionally, to assist in the design of siRNAs for the efficient RNAi-mediated silencing of any target gene, several siRNA supply companies maintain web-based design tools that utilize these general guidelines for "picking" siRNAs when presented with the mRNA or coding DNA sequence of the target gene. Examples of such tools can be found at the web sites of Dharmacon, Inc. (Lafayette, Colo.), Ambion, Inc. (Austin, Tex.). A criterion to be considered is whether or not the target sequence includes a known polymorphic site. If so, siRNAs designed to target one particular allele may not effectively target another allele, since single base mismatches between the target sequence and its complementary strand in a given siRNA can greatly reduce the effectiveness of RNAi induced by that siRNA. Given the target sequence and such design tools and design criteria, an ordinarily skilled artisan apprised of the present disclosure should be able to design and synthesize additional siRNA compounds useful in reducing the level of HtrA1 or CFH mRNA and, thereby, reduce HtrA1 or CFH protein levels.

In another embodiment, inhibitors of cellular levels of HtrA1 or CFH may be antisense compounds, or a modified equivalent thereof. Such antisense compounds and methods may be employed to treat and/or prevent ocular disease and pathologic conditions according to the therapeutic methods described herein. The antisense compounds according to this embodiment specifically inhibit the expression of the target protein (e.g., HtrA1 or CFH). As is known in the art, antisense drugs generally act by hybridizing to a particular target nucleic acid thus blocking gene expression (particularly protein translation from mRNA). Methods for designing antisense compounds and using such compounds in treating diseases are well known and well developed in the art. For example, the antisense drug Vitravene™ (fomivirsen), a 21-base long oligonucleotide, has been successfully developed and marketed by Isis Pharmaceuticals, Inc. for treating cytomegalovirus (CMV)-induced retinitis.

Antisense compounds useful in inhibiting protein translation from the HtrA1 mRNA and from the CFH mRNA can be designed and prepared. Any methods for designing and making antisense compounds may be used for purpose of the present invention (See, generally, Sanghvi et al., eds., Antisense Research and Applications, CRC Press, Boca Raton, 1993). Typically, antisense compounds are oligonucleotides designed based on the nucleotide sequence of the host cell's protein(s) involved in viral budding (or egress) mRNA or gene. In particular, antisense compounds can be designed to specifically hybridize to a particular region of the host cell's protein(s) involved in viral budding (or egress) genomic sequence or mRNA to interfere with replication, transcription, or translation. As used herein, the term "specifically hybridize" or variations thereof means a sufficient degree of complementarity or pairing between an antisense oligo and a target DNA or mRNA such that stable and specific binding occurs therebetween. In particular, 100% complementarity or pairing is desirable but not required. Specific hybridization occurs when sufficient hybridization occurs between the antisense compound and its intended target nucleic acids in the substantial absence of non-specific binding of the antisense compound to non-target sequences under predetermined conditions, *e.g.,* for purposes of in vivo treatment, preferably under physiological conditions. Preferably, specific hybridization results in the interference with normal expression of the gene product encoded by the target DNA or mRNA.

For example, an antisense compound can be designed to specifically hybridize to the replication or transcription regulatory regions of a target gene, or the translation regulatory regions such as translation initiation region and exon/intron junctions, or the coding regions of a target mRNA.

As is generally known in the art, commonly used oligonucleotides are oligomers or polymers of ribonucleic acid or deoxyribonucleic acid having a combination of naturally-occurring purine and pyrimidine bases, sugars and covalent linkages between nucleosides including a phosphate group in a phosphodiester linkage. However, it is noted that the term "oligonucleotides" also encompasses various non-naturally occurring mimetics and derivatives, *i.e*., modified forms, of naturally-occurring oligonucleotides as described below. Typically an antisense compound of the present invention is an oligonucleotide having from about 6 to about 200, or from about 8 to about 30 nucleoside bases.

The antisense compounds preferably contain modified backbones or non-natural internucleoside linkages, including, but not limited to, modified phosphorous-containing backbones and non-phosphorous backbones such as morpholino backbones; siloxane, sulfide, sulfoxide, sulfone, sulfonate, sulfonamide, and sulfamate backbones; formacetyl and thioformacetyl backbones; alkene-containing backbones; methyleneimino and methylenehydrazino backbones; amide backbones, and the like.

Examples of modified phosphorous-containing backbones include, but are not limited to phosphorothioates, phosphorodithioates, chiral phosphorothioates, phosphotriesters, aminoalkylphosphotriesters, alkyl phosphonates, thionoalkylphosphonates, phosphinates, phosphoramidates, thionophosphoramidates, thionoalkylphosphotriesters, and boranophosphates and various salt forms thereof (See, e.g., U.S. Patent Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050).

Examples of the non-phosphorous containing backbones described above are disclosed in, e.g., U.S. Patent Nos. 5,034,506; 5,185,444; 5,214,134; 5,216,141; 5,235,033; 5,264,562; 5,264,564; 5,405,938; 5,434,257; 5,470,967; 5,489,677; 5,541,307; 5,561,225; 5,596,086; 5,610,289; 5,602,240; 5,608,046; 5,610,289; 5,618,704; 5,623,070; 5,663,312; 5,677,437; and 5,677,439).

Another useful modified oligonucleotide is peptide nucleic acid (PNA), in which the sugar-phosphate backbone of an oligonucleotide is replaced with an amide containing backbone, e.g., an aminoethylglycine backbone (See, U.S. Patent Nos. 5,539,082 and 5,714,331; and Nielsen et al., Science, 254, 1497 1500 (1991)). PNA antisense compounds are resistant to RNAse H digestion and thus exhibit longer half-lives within cells. In addition, various modifications may be made in PNA backbones to impart desirable drug profiles such as better stability, increased drug uptake, higher affinity to target nucleic acid, etc.

Alternatively, the antisense compounds are oligonucleotides containing modified nucleosides, i.e., modified purine or pyrimidine bases, e.g., 5-substituted pyrimidines, 6-azapyrimidines, and N-2, N-6 and O-substituted purines, and the like (See, e.g., U.S. Patent Nos. 3,687,808; 4,845,205; 5,130,302; 5,175,273; 5,367,066; 5,432,272; 5,459,255; 5,484,908; 5,502,177; 5,525,711; 5,587,469; 5,594,121; 5,596,091; 5,681,941; and 5,750,692)

In addition, oligonucleotides with substituted or modified sugar moieties may also be used. For example, an antisense compound may have one or more 2'-O-methoxyethyl sugar moieties (See, e.g., U.S. Patent Nos. 4,981,957; 5,118,800; 5,319,080; 5,393,878; 5,446,137; 5,466,786; 5,514,785; 5,567,811; 5,576,427; 5,591,722; 5,610,300; 5,627,0531 5,639,873; 5,646,265; 5,658,873; 5,670,633; and 5,700,920).

Other types of oligonucleotide modifications are also useful, including linking an oligonucleotide to a lipid, phospholipid or cholesterol moiety, cholic acid, thioether, aliphatic chain, polyamine, polyethylene glycol (PEG), or a protein or peptide. The modified oligonucleotides may exhibit increased uptake into cells, and/or improved stability, *i.e.,* resistance to nuclease digestion and other biodegradation (See, *e.g*., U.S. Patent No. 4,522,811; Burnham, Am. J. Hosp. Pharm., 15:210 218 (1994)).

Antisense compounds can be synthesized using any suitable methods known in the art. In fact, antisense compounds may be custom made by commercial suppliers. Alternatively, antisense compounds may be prepared using DNA/RNA synthesizers commercially available from various vendors, *e.g.,* Applied Biosystems Group of Norwalk, Conn.

The antisense compounds can be formulated into a pharmaceutical composition with suitable carriers and administered into a patient using any suitable route of administration. Alternatively, the antisense compounds may also be used in a "gene-therapy" approach. That is, the oligonucleotide is subcloned into a suitable vector and transformed into human cells. The antisense oligonucleotide is then produced *in-vivo* through transcription.

Ribozymes are enzymatic RNA molecules capable of catalyzing the specific cleavage of RNA. The mechanism of ribozyme action involves sequence specific hybridization of the ribozyme molecule to complementary target RNA, followed by an endonucleolytic cleavage. The composition of ribozyme molecules must include one or more sequences complementary to the target mRNA (e.g., an HtrA1 mRNA or a CFH mRNA), and must include the well known catalytic sequence responsible for mRNA cleavage. For such a sequence, see U.S. Patent No. 5,093,246. As such, within the scope of the invention are engineered hammerhead motif ribozyme molecules that specifically and efficiently catalyze endonucleolytic cleavage of RNA sequences encoding HtrA1 and sequences encoding CFH.

Specific ribozyme cleavage sites within any potential RNA target are initially identified by scanning the target molecule for ribozyme cleavage sites which include the following sequence: GUA, GUU and GUC. Once identified, short RNA sequences of between 15 and ribonucleotides corresponding to the region of the target gene containing the cleavage site may be evaluated for predicted structural features, such as secondary structure, that may render the oligonucleotide sequence unsuitable. The suitability of candidate targets may also be evaluated by testing their accessibility to hybridization with complementary oligonucleotides, using ribonuclease protection assays.

Nucleic acid molecules to be used in triplex helix formation should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC⁺ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of guanidine residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3',3'-5' manner, such that they base pair with one strand of a duplex first and then the other, eliminating the necessity for a sizeable stretch of either purines or pyrimidines to be present on one strand of a duplex.

It is possible that the siRNA, antisense, ribozyme, and/or triple helix molecules described herein may reduce or inhibit the translation of mRNA produced by both normal and mutant alleles of HtrA1 and of CFH. In order to ensure that substantial normal levels of HtrA1 or CFH activity are maintained in the cell, nucleic acid molecules that encode and express HtrA1 polypeptides exhibiting normal HtrA1 or CFH expression and activity can be introduced into cells which do not contain sequences susceptible to the siRNA, antisense, ribozyme, or triple helix treatments. Such sequences can be introduced via gene therapy methods. Alternatively, it may be preferable to co-administer normal HtrA1 protein, normal CFH protein, or both, depending on which protein translation is inhibited, into the cell or tissue in order to maintain the requisite level of cellular or tissue activity.

Antisense RNA and DNA molecules, siRNA molecules, ribozyme molecules, and triple helix molecules as described herein can be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules can be generated by *in-vitro* and *in*-*vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Various well-known modifications to the DNA molecules can be introduced as a means of increasing intracellular stability and half-life. Possible modifications include, but are not limited to, the addition of flanking sequences of ribo- or deoxynucleotides to the 5' and/or 3' ends of the molecule or the use of phosphorothioate or 2' O-methyl rather than phosphodiesterase linkages within the oligodeoxyribonucleotide backbone.

Gene therapy would be carried out according to generally accepted methods, for example, as described in further detail in U.S. Patent Nos. 5,837,492 and 5,800,998 and references cited therein, all incorporated by reference herein. Expression vectors in the context of gene therapy are meant to include those constructs containing sequences sufficient to express a polynucleotide that has been cloned therein. In viral expression vectors, the construct contains viral sequences sufficient to support packaging of the construct. If the polynucleotide encodes an antisense polynucleotide or a ribozyme, expression will produce the antisense polynucleotide or ribozyme. Thus in this context, expression does not require that a protein product be synthesized. In addition to the polynucleotide cloned into the expression vector, the vector also contains a promoter functional in eukaryotic cells. The cloned polynucleotide sequence is under control of this promoter. Suitable eukaryotic promoters include those described above. The expression vector may also include sequences, such as selectable markers and other sequences conventionally used.

### Antibodies to HtrA1

Antibodies to HtrA1 are described herein. Exemplary antibodies to HtrA1 according to the present description include polyclonal, monoclonal, humanized, human, and affinity matured antibodies. The HtrA1 antibodies contemplated herein also include, for example, antibody fragments, glycosylation variants, antibodies having one or more amino acid substitutions, and antibodies conjugated to one or more chemical entities that allow the antibody to achieve a desired biological activity or functional characteristic. Antibodies as described herein may be agonists, antagonists or blocking antibodies to HtrA1 or an HtrA1 variant.

In one embodiment, the antibodies to HtrA1 are polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include, for example, a HtrA1, a variant of HtrA1, or a fusion protein thereof. In addition, it may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in, the art without undue experimentation. The mammal can then be bled, and the serum assayed for HtrA1 antibody titer. If desired, the mammal can be boosted until the antibody titer increases or plateaus. In one embodiment, the immunizing agent used to generate polyclonal antibodies to HtrA1 includes a fragment of human HtrA1 according to SEQ. ID. NO.: 1, the fragment comprising amino acids 141-480 of a full length human HtrA1 molecule, or a variant of such a fragment. In an alternative embodiment, the immunizing agent used to generate polyclonal antibodies to HtrA1 includes a full-length human HtrA1 molecule according to SEQ. ID. NO. 2 or a variant thereof.

In another embodiment, the HtrA1 antibodies described herein are monoclonal antibodies. Monoclonal antibodies may be prepared using hybridoma methods, such as those described by Kohler and Milstein, Nature, 256:495 (1975), and by Harlow and Lane, Antibodies: A Laboratory Manual. 1988 Cold Spring Harbor, NY: Cold Spring Harbor Laboratory Press. In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro. The immunizing agent will typically include a desired HtrA1 or a fusion protein thereof. In one embodiment, the immunizing agent used to generate polyclonal antibodies to HtrA1 includes a fragment of human HtrA1 according to SEQ. ID. NO.: 1, the fragment comprising amino acids 141-480 of a full length human HtrA1 molecule, or a variant of such a fragment. In an alternative embodiment, the immunizing agent used to generate polyclonal antibodies to HtrA1 includes a full-length human HtrA1 molecule according to SEQ. ID. NO.: 2 or a variant thereof.

The lymphocytes that produce or are capable of producing HtrA1 antibodies are then fused with an immortalized cell line using a suitable fusing agent, such as polyethylene glycol, to form a hybridoma cell (See, Goding, Monoclonal Antibodies: Principles and Practice, Academic Press, (1986) pp. 59-103). Generally, either peripheral blood lymphocytes (PBLs) are used if cells of human origin are desired, or spleen cells or lymph node cells are used if non-human mammalian sources are desired. Immortalized cell lines are usually transformed mammalian cells, particularly myeloma cells of rodent, bovine and human origin. Typically, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

In one embodiment, immortalized cell lines are those that fuse efficiently, support stable high level expression of antibody by the selected antibody-producing cells, and are sensitive to a medium such as HAT medium are selected for preparation of hybridoma cells. For example, murine myeloma lines, which can be obtained, for instance, from the Salk Institute Cell Distribution Center, San Diego, CA, and the American Type Culture Collection, Manassas, Va. In one embodiment, as is detailed in the Examples that follow, cells from the myeloma Sp2/0-Ag14 cell line are used to form hybridomas producing the HtrA1 antibodies. Additionally, human myeloma and mouse-human heteromyeloma cell lines may also be used in the formation of hybridomas capable of producing human monoclonal antibodies (See, Kozbor, J. Immunol., 133:3001 (1984); Brodeur et al., Monoclonal Antibody Production Techniques and Applications, Marcel Dekker, Inc., New York, (1987) pp. 51-63).

Once hybridoma cells are prepared, The culture medium in which the hybridoma cells are cultured can be assayed for the presence of monoclonal antibodies directed against HtrA1. The binding specificity of monoclonal antibodies produced by the hybridoma cells can, for example, be determined by immunoprecipitation or by an in vitro binding assay, such as radioimmunoassay (RIA) or enzyme-linked immunoabsorbent assay (ELISA). Such techniques and assays are known in the art. In addition, the binding affinity of the monoclonal antibody can, for example, be determined by the Scatchard analysis of Munson and Pollard, Anal. Biochem., 107:220 (1980).

After desired hybridoma cells are identified, the clones may be subcloned by limiting dilution procedures and grown by standard methods (See, Goding, supra). Suitable culture media for this purpose include, for example, Dulbecco's Modified Eagle's Medium or RPMI-1640 medium. Alternatively, the hybridoma cells may be grown *in*-*vivo* as ascites in a mammal.

The monoclonal HtrA1 antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

Alternatively, monoclonal HtrA1 antibodies as described herein may also be made by recombinant DNA methods, such as those described in U.S. Patent No. 4,816,567. DNA encoding the monoclonal antibodies can be isolated and sequenced using conventional procedures (*e.g.,* by using oligonucleotide probes that are capable of binding specifically to genes encoding the heavy and light chains of the monoclonal antibodies). The hybridoma cells serve as a preferred source of such DNA. Once isolated, the DNA may be placed into expression vectors, which are then transfected into host cells such as, for example, E. coli cells, simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences (See, e.g., Morrison, et al., Proc. Nat. Acad. Sci. 81, 6851 (1984)), or by covalently joining to the immunoglobulin coding sequence all or part of the coding sequence for a non-immunoglobulin polypeptide. In that manner, "chimeric" or "hybrid" antibodies that have the binding specificity of an anti-HTRA1 monoclonal antibody according to the present description can be prepared.

Typically, such non-immunoglobulin polypeptides are substituted for the constant domains of an antibody of the invention, or they are substituted for the variable domains of one antigen-combining site of an antibody of the invention to create a chimeric bivalent antibody comprising one antigen-combining site having specificity for HtrA1 and another antigen-combining site having specificity for a different antigen.

Human, chimeric, hybrid or recombinant anti-HtrA1 antibodies may comprise an antibody having full length heavy and light chains or fragments thereof, such as a Fab, Fab', F(ab')₂ or Fv fragment, a monomer or dimer of such light chain or heavy chain, a single chain Fv in which such heavy or light chain(s) are joined by a linker molecule, or having variable domains (or hypervariable domains) of such light or heavy chain(s) combined with still other types of antibody domains.

Chimeric or hybrid antibodies also may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

Single chain Fv fragments may also be produced, such as described in Iliades et al., FEBS Letters, 409:437-441 (1997). Coupling of such single chain fragments using various linkers is described in Kortt et al., Protein Engineering, 10:423-433 (1997). A variety of techniques for the recombinant production and manipulation of antibodies are well known in the art.

In one embodiment, the antibodies according to the present description are humanized HtrA1 antibodies. A humanized HtrA1 antibody will typically has one or more amino acid residues introduced into it from a non-human source. Such non-human amino acid residues are often referred to as "import" residues, which are typically taken from an "import" variable domain. Often, humanized antibodies are human antibodies in which CDR residues and possibly FR residues are substituted by residues from analogous sites in rodent antibodies. Therefore, humanized HtrA1 antibodies according to the present description are typically chimeric antibodies, wherein less than an intact human variable domain has been substituted by the corresponding sequence from a non-human species. Humanized HtrA1 antibodies as contemplated herein may be produced using methods known in the art, such as, for example, the methods described in Jones et al., Nature, 321:522-525 (1986), Riechmann et al., Nature, 332:323-327 (1988), and/or Verhoeyen et al., Science, 239:1534-1536 (1988).

In another embodiment, the antibodies described herein are human HtrA1 monoclonal antibodies made by a hybridoma method. Cell lines, such as human myeloma and mouse-human heteromyeloma cell lines, suitable for production of human monoclonal antibodies are known, and are taught, for example, by Kozbor, J. Immunol. 133, 3001 (1984), and Brodeur, et al., Monoclonal Antibody Production Techniques and Applications, pp. 51-63 (Marcel Dekker, Inc., New York, 1987). In another embodiment, human HtrA1 antibodies may be produced using a transgenic animal, such as transgenic mice, capable of producing human antibodies in the absence of endogenous immunoglobulin production. Techniques and transgenic animals suitable for production of human antibodies are described, for example by Jakobovits et al., Proc. Natl. Acad. Sci. USA 90, 2551-255 (1993), Jakobovits et al., Nature 362, 255-258 (1993), and Mendez et al., Nature Genetics 15: 146-156 (1997)).

Human antibodies can additionally be produced using the phage display technology described by McCafferty et al., Nature 348, 552-553 (1990). Such technology allows production of human antibodies and human antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g. Johnson, Kevin S. and Chiswell, David J., Current Opinion in Structural Biology 3, 564-571 (1993). Several sources of V-gene segments can be used for phage display. Clackson et al., Nature 352, 624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222, 581-597 (1991), or Griffith et al., EMBO J. 12, 725-734 (1993). In a natural immune response, antibody genes accumulate mutations at a high rate (somatic hypermutation). Some of the changes introduced will confer higher affinity, and B cells displaying high-affinity surface immunoglobulin are preferentially replicated and differentiated during subsequent antigen challenge. This natural process can be mimicked by employing the technique known as "chain shuffling" (Marks et al., Bio/Technol. 10, 779-783 [1992]). In this method, the affinity of "primary" human antibodies obtained by phage display can be improved by sequentially replacing the heavy and light chain V region genes with repertoires of naturally occurring variants (repertoires) of V domain genes obtained from unimmunized donors. This techniques allows the production of antibodies and antibody fragments with affinities in the nM range. A strategy for making very large phage antibody repertoires (also known as "the mother-of-all libraries") has been described by Waterhouse et al., Nucl. Acids Res. 21, 2265-2266 (1993). Gene shuffling can also be used to derive human antibodies from rodent antibodies, where the human antibody has similar affinities and specificities to the starting rodent antibody. According to this method, which is also referred to as "epitope imprinting", the heavy or light chain V domain gene of rodent antibodies obtained by phage display technique is replaced with a repertoire of human V domain genes, creating rodent-human chimeras. Selection on antigen results in isolation of human variable capable of restoring a functional antigen-binding site, i.e. the epitope governs (imprints) the choice of partner. When the process is repeated in order to replace the remaining rodent V domain, a human antibody is obtained (see PCT patent application WO 93/06213, published 1 Apr. 1993). Unlike traditional humanization of rodent antibodies by CDR grafting, this technique provides completely human antibodies, which have no framework or CDR residues of rodent origin.

In certain embodiments, the anti-HtRA1 antibody (including murine, human and humanized antibodies, and antibody variants) is an antibody fragment. Various techniques for the production of antibody fragments are known. For example, antibody fragments can be derived via proteolytic digestion of intact antibodies (see, e.g., Morimoto et al., J. Biochem. Biophys. Methods 24:107-117 (1992) and Brennan et al., Science 229:81 (1985)). Additionally, antibody fragments can be produced directly by recombinant host cells. For example, Fab'-SH fragments can be directly recovered from E. coli and chemically coupled to form F(ab')₂ fragments (Carter et al., Bio/Technology 10:163-167 (1992)). In another embodiment, the F(ab')₂ is formed using the leucine zipper GCN4 to promote assembly of the F(ab')₂ molecule. According to another approach, Fv, Fab or F(ab')₂ fragments can be isolated directly from recombinant host cell culture.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

Amino acid sequence variants of the anti-HtrA1 antibodies are prepared by introducing appropriate nucleotide changes into the anti-HtrA1 antibody DNA, or by peptide synthesis. Such variants include, for example, deletions from, and/or insertions into and/or substitutions of, residues within the amino acid sequences of the anti-HtrA1 antibodies of the examples herein. Any combination of deletion, insertion, and substitution is made to arrive at the final construct, provided that the final construct possesses the desired characteristics. The amino acid changes also may alter post-translational processes of the humanized or variant anti-HtrA1 antibody, such as changing the number or position of glycosylation sites.

A useful method for identification of certain residues or regions of the anti-HtrA1 antibody that are preferred locations for mutagenesis is called "alanine scanning mutagenesis," as described by Cunningham and Wells Science, 244:1081-1085 (1989). According to such method, a residue or group of target residues are identified (e.g., charged residues such as arg, asp, his, lys, and glu) and replaced by a neutral or negatively charged amino acid (most preferably alanine or polyalanine) to affect the interaction of the amino acids with HtrA1 antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed anti-HtrA1 antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an anti-HtrA1 antibody with an N-terminal methionyl residue or the antibody fused to an epitope tag. Other insertional variants of the anti-HTRA1 antibody molecule include the fusion to the N- or C-terminus of the anti-HTRA1 antibody of an enzyme or a polypeptide which increases the serum half-life of the antibody.

Another type of variant is an amino acid substitution variant. These variants have at least one amino acid residue in the anti-HtrA1 antibody molecule removed and a different residue inserted in its place. The sites of greatest interest for substitutional mutagenesis include the hypervariable regions, but FR alterations are also contemplated.

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Any cysteine residue not involved in maintaining the proper conformation of the humanized or variant anti-HtrA1 antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (*e*.*g*., a humanized or human antibody). Generally, the resulting variant(s) selected for further development will have improved biological properties relative to the parent antibody from which they are generated. A convenient way for generating such substitutional variants is affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e*.*g*., binding affinity) as herein disclosed. In order to identify candidate hypervariable region sites for modification, alanine scanning mutagenesis can be performed to identify hypervariable region residues contributing significantly to antigen binding. Alternatively, or in addition, it may be beneficial to analyze a crystal structure of the antigen-antibody complex to identify contact points between the antibody and a desired HtrA1 polypeptide. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Glycosylation variants of HtrA1 antibodies are also contemplated herein. Antibodies are glycosylated at conserved positions in their constant regions (See, Jefferis and Lund, Chem. Immunol. 65:111-128 (1997); Wright and Morrison, TibTECH 15:26-32 (1997)). The oligosaccharide side chains of the immunoglobulins affect the protein's function (See, Boyd et al., Mol. Immunol. 32:1311-1318 (1996); Wittwe and Howard, Biochem. 29:4175-4180 (1990)), and the intramolecular interaction between portions of the glycoprotein which can affect the conformation and presented three-dimensional surface of the glycoprotein (See, Jefferis and Lund, supra; Wyss and Wagner, Current Opin. Biotech. 7:409-416 (1996)). Oligosaccharides may also serve to target a given glycoprotein to certain molecules based upon specific recognition structures. For example, it has been reported that in agalactosylated IgG, the oligosaccharide moiety 'flips' out of the inter-CH2 space and terminal N-acetylglucosamine residues become available to bind mannose binding protein (See, Malhotra et al., Nature Med. 1:237-243 (1995)). Removal by glycopeptidase of the oligosaccharides from CAMPATH-1H (a recombinant humanized murine monoclonal IgG1 antibody which recognizes the CDw52 antigen of human lymphocytes) produced in Chinese Hamster Ovary (CHO) cells resulted in a complete reduction in complement mediated lysis (CMCL) (See, Boyd et al., Mol. Immunol. 32:1311-1318 (1996)), while selective removal of sialic acid residues using neuraminidase resulted in no loss of DMCL. Glycosylation of antibodies has also been reported to affect antibody-dependent cellular cytotoxicity (ADCC). In particular, CHO cells with tetracycline-regulated expression of ß(1,4)-N-acetylglucosaminyltransferase III (GnTIII), a glycosyltransferase catalyzing formation of bisecting GlcNAc, was reported to have improved ADCC activity (Umana et al., Mature Biotech. 17:176-180 (1999)).

Glycosylation variants of antibodies are variants in which the glycosylation pattern of an antibody is altered. By altering is meant deleting one or more carbohydrate moieties found in the antibody, adding one or more carbohydrate moieties to the antibody, changing the composition of glycosylation (glycosylation pattern), the extent of glycosylation, etc. Glycosylation variants may, for example, be prepared by removing, changing and/or adding one or more glycosylation sites in the nucleic acid sequence encoding the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the anti-HtrA1 antibody can be prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-HtrA1 antibody.

The glycosylation (including glycosylation pattern) of antibodies may also be altered without altering the underlying nucleotide sequence. Glycosylation largely depends on the host cell used to express the antibody. Since the cell type used for expression of recombinant glycoproteins, *e.g*., antibodies, as potential therapeutics is rarely the native cell, significant variations in the glycosylation pattern of the antibodies can be expected (See, *e*.*g*., Hse et al., J. Biol. Chem. 272:9062-9070 (1997)). In addition to the choice of host cells, factors which affect glycosylation during recombinant production of antibodies include growth mode, media formulation, culture density, oxygenation, pH, purification schemes and the like. Various methods have been proposed to alter the glycosylation pattern achieved in a particular host organism including introducing or over expressing certain enzymes involved in oligosaccharide production (See, U.S. Patent Nos. 5,047,335; 5,510,261 and 5.278,299). Glycosylation, or certain types of glycosylation, can be enzymatically removed from the glycoprotein, for example using endoglycosidase H (Endo H). In addition, the recombinant host cell can be genetically engineered, e.g. make defective in processing certain types of polysaccharides. These and similar techniques are well known in the art.

The glycosylation structure of antibodies can be readily analyzed by conventional techniques of carbohydrate analysis, including lectin chromatography, NMR, Mass spectrometry, HPLC, GPC, monosaccharide compositional analysis, sequential enzymatic digestion, and HPAEC-PAD, which uses high pH anion exchange chromatography to, separate oligosaccharides based on charge. Methods for releasing oligosaccharides for analytical purposes are also known, and include, without limitation, enzymatic treatment (commonly performed using peptide-N-glycosidase F/endo-.alpha.-galactosidase), elimination using harsh alkaline environment to release mainly O-linked structures, and chemical methods using anhydrous hydrazine to release both N- and O-linked oligosaccharides.

Other modifications of the HtrA1 antibodies are contemplated herein. For example, the antibody may be linked to one of a variety of nonproteinaceous polymers, *e.g.*, polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (*e*.*g*., hydroxymethylcellulose or gelatin-microcapsules and poly-(methylmethacylate) microcapsules, respectively), in colloidal drug delivery systems (*e.g*., liposome's, albumin microspheres, microemulsions, nano-particles and nanocapsules), or in macroemulsions. Such techniques are disclosed, for example, in Remington's Pharmaceutical Sciences, 16th edition, Oslo, A., Ed., (1980). To increase the serum half life of the antibody, one may incorporate a salvage receptor binding epitope into the antibody (especially an antibody fragment) as described in U.S. Patent No. 5,739,277, for example. As used herein, the term "salvage receptor binding epitope" refers to an epitope of the Fc region of an IgG molecule (*e.g*., IgG₁, IgG₂, IgG₃, or IgG₄) that is responsible for increasing the in vivo serum half-life of the IgG molecule.

The anti-HtrA1 antibodies disclosed herein may also be formulated as immunoliposomes. Liposome's containing the antibody are prepared by methods known in the art, such as, for example, the methods described in Epstein et al., Proc. Natl. Acad. Sci. USA 82:3688 (1985); Hwang et al., Proc. Natl. Acad. Sci. USA 77:4030 (1980); and U.S. Patent Nos. 4,485,045 and 4,544,545. Liposome's with enhanced circulation time are disclosed in U.S. Patent No. 5,013,556. Liposome's can be generated by the reverse phase evaporation method with a lipid composition comprising phosphatidylcholine, cholesterol and PEG-derivatized phosphatidylethanolamine (PEG-PE). Liposome's are typically extruded through filters of defined pore size to yield liposome's with the desired diameter. Fab' fragments of the antibody of the present invention may be conjugated to the liposome's as described in Martin et al., J. Biol. Chem. 257:286-288 (1982) via a disulfide interchange reaction.

The antibodies of the invention include "cross-linked" HtrA1 antibodies. The term "cross-linked" as used herein refers to binding of at least two IgG molecules together to form one (or single) molecule. The HtrA1 antibodies may be cross-linked using various linker molecules, preferably the HtrA1 antibodies are cross-linked using an anti-IgG molecule, complement, chemical modification or molecular engineering. It is appreciated by those skilled in the art that complement has a relatively high affinity to antibody molecules once the antibodies bind to cell surface membrane. Accordingly, it is believed that complement may be used as a cross-linking molecule to link two or more anti-HtrA1 antibodies bound to cell surface membrane. Cross-linking of the human anti-HtrA1 antibodies is also described in the Examples using either goat anti-mouse IgG Fc or goat anti-human IgG Fc.

### Exemplary Antibodies

The following embodiments are offered for illustrative purposes only, and are not intended to limit the scope of the present invention in any way.

As described in the Examples below, polyclonal anti-HtrA1 antibodies have been prepared and a number of anti-HtrA1 monoclonal antibodies have been identified and prepared. In one embodiment, the monoclonal antibodies according to this description will have the same biological characteristics as the monoclonal antibodies secreted by the hybridoma cell line(s) referred to in the Examples that follow. The term "biological characteristics" is used to refer to the *in*-*vitro* and/or *in-vivo* activities or properties of the monoclonal antibody, such as the ability to specifically bind to HtrA1 or to block, inhibit, induce or enhance HtrA1 related activities. By way of example, a blocking antibody may block binding of a ligand to HtrA1 such as, for example, binding of a ligand to the IGFBP domain of the HtrA1 or any other HtrA1 domain to which a ligand may bind. Additionally, by way of example, a blocking antibody may inhibit the activity or function of any one or more of the trypsin domain, PDZ domain, SP domain, KI domain, Mac25 domain, or FS domain.

The HtrA1 antibodies, as described herein, optionally possess one or more desired biological activities or properties. Such HtrA1 antibodies may include, but are not limited to, chimeric, humanized, human, and affinity matured antibodies. As described above, the HtrA1 antibodies may be constructed or engineered using various techniques to achieve these desired activities or properties. In one embodiment, the HtrA1 antibody has an HtrA1 binding affinity of at least 10⁵ M⁻¹. In one such embodiment, the HtrA1 antibody is an isolated polyclonal antibody. In another such embodiment, the HtrA1 antibody is an isolated monoclonal antibody. In an alternative embodiment, the HtrA1 antibody has an HtrA1 binding affinity in a range selected from 10⁶ M⁻¹ to 10⁷ M⁻¹, 10⁸ M⁻¹ to 10¹² M⁻¹, and 10⁹ M⁻¹ to 10¹² M⁻¹. In yet a further alternative embodiment, an HtrA1 antibody as described herein has a binding affinity selected from at least 10⁶ M⁻¹, at least 10⁷ M⁻¹, at least 10⁸ M⁻¹, at least 10⁹ M⁻¹, at least 10¹⁰ M⁻¹, at least 10¹¹ M⁻¹, and at least 10¹² M⁻¹. In one such embodiment, the HtrA1 antibody is an isolated polyclonal antibody. In another such embodiment, the HtrA1 antibody is an isolated monoclonal antibody.

The binding affinity of the HtrA1 antibody can be determined without undue experimentation by testing the HtrA1 antibody in accordance with techniques known in the art, including Scatchard analysis (See, Munson et al., supra). Embodiments of antibodies exhibiting the binding affinities or ranges of binding affinities set forth herein may include, for example, monoclonal antibodies, polyclonal antibodies, chimeric antibodies, humanized antibodies, human antibodies, and affinity matured antibodies. Further embodiments of antibodies exhibiting the binding affinities or ranges of binding affinities set forth herein include monoclonal antibodies, polyclonal antibodies, chimeric antibodies, humanized antibodies, human antibodies, and affinity matured antibodies generated using an immunizing agent that includes a fragment of human HtrA1 according to SEQ. ID. NO.: 1, the fragment comprising amino acids 141-480 of a full length human HtrA1 molecule, or a variant of such a fragment, and monoclonal antibodies, polyclonal antibodies, chimeric antibodies, humanized antibodies, human antibodies, and affinity matured antibodies generated using an immunizing agent that includes a full-length human HtrA1 molecule according to SEQ. ID. NO.: 2 or a variant thereof.

The antibodies disclosed herein also exhibit characteristics related to their ability to immunospecifically bind to HtrA1 epitopes. For example, in one embodiment, the antibodies disclosed herein have the ability to bind to one or more specific epitopes on the HtrA1 molecule. In addition, the amino acid sequences of the antibodies described allow binding of the antibodies described herein to the one or more HtrA1 epitopes. Antibodies disclosed herein also have the ability to competitively inhibit the immunospecific binding of antibodies that recognize an identical or nearly identical epitope on an HtrA1 molecule. In a typical embodiment, as is described and illustrated in Example 5, antibody binding of HtrA1 is evaluated, for example, using an indirect ELISA.

When evaluated in an indirect ELISA for binding to HtrA1 or a variant thereof, in one embodiment, the antibodies of the present invention exhibit a titer of 1:160,000 or greater. In another embodiment, the antibodies described herein exhibit a titer selected from 1:200,000 or greater, 1:300,00 or greater, 1:400,000 or greater, 1:500,000 or greater, 1:600,000 or greater, 1:700,000 or greater, 1:800,000 or greater, 1:900,000 or greater, and 1:1,000,000 or greater. In still another embodiment, the antibodies described herein exhibit a range of titers selected from 1:200,000 to greater than 1:1,000,000, 1:300,000 to greater than 1:1,000,000, 1:400,000 to greater than 1:1,000,000, 1:500,000 to greater than 1:1,000,000, 1:600,000 to greater than 1:1,000,000, 1:700,000 to greater than 1:1,000,000, 1:800,000 to greater than 1:1,000,000, and 1:900,000 to greater than 1:1,000,000. In yet another embodiment, the antibodies described herein exhibit a range of titers selected from 1:200,000 to 1:300,000, 1:200,000 to 1:400,000, 1:200,000 to 1:500,000, 1:200,000 to 1:800,000, 1:200,000 to 1:900,000, and 1:200,000 to 1:1,000,000. In still a further embodiment, the antibodies described herein exhibit a range of titers selected from 1:200,000 to 1:400,000, 1:200,000 to 1:500,000, 1:400,000 to 1:800,000, 1:400,000 to 1:900,000, 1:400,000 to greater than 1:1,000,000, and 1:800,000 to greater than 1:1,000,000. As used herein, the "titer" of a particular antibody provides an indication of that antibody's capability to bind to HtrA1. In particular, in evaluating and comparing the titer as described herein of different antibodies, antibody solutions exhibiting substantially uniform antibody concentration (e.g., 1mg/ml) are generated for each antibody evaluated. Such solutions are then serially diluted, and the titer of a given antibody refers to the maximum dilution of the initial antibody concentration that, when evaluated in an indirect ELISA under the conditions in Example 5, exhibits an optical density at least approximately twice the optical density of a control well which includes no antibody.

Embodiments of antibodies exhibiting the titers or ranges of titers set forth herein may include, for example, monoclonal antibodies, polyclonal antibodies, chimeric antibodies, humanized antibodies, human antibodies, and affinity matured antibodies. In yet other embodiments, antibodies exhibiting the titers or ranges of titers set forth herein may include monoclonal antibodies, polyclonal antibodies, chimeric antibodies, humanized antibodies, human antibodies, and affinity matured antibodies generated using an immunizing agent that includes a fragment of human HtrA1 according to SEQ. ID. NO.: 1, the fragment comprising amino acids 141-480 of a full length human HtrA1 molecule, or a variant of such a fragment, and monoclonal antibodies, polyclonal antibodies, chimeric antibodies, humanized antibodies, human antibodies, and affinity matured antibodies generated using an immunizing agent that includes a full-length human HtrA1 molecule according to SEQ. ID. NO.: 2 or a variant thereof.

In one embodiment, the monoclonal antibodies described herein include isolated monoclonal antibodies having one or more of the same biological characteristics of a monoclonal antibody produced by a hybridoma cell selected from the 8F12, 13D4, 10E7, 9F7, 8G8, 4H5, 7G6, 25A6, 21C9, 14E6, 3E8, 5B12, and 8F11 hybridoma cell lines as produced and described in Example 4 below. Embodiments of such antibodies include, for example, isolated monoclonal antibodies generated using an immunizing agent that includes a fragment of HtrA1 according to SEQ. ID. NO.: 1. Even further, embodiments of such antibodies may exhibit the titers or range of titers and or binding affinities described herein.

The antibodies described herein have the ability to modulate certain physiological actions or processes. In one embodiment, as is shown in Examples 6-10, the antibodies described herein inhibit one or more activities of HtrA1. In one such embodiment, an antibody according to the present description is biologically active and directly or indirectly inhibits or blocks HtrA1 activity or activation, such as by, for example, directly or indirectly inhibiting or blocking the activity or activation of one or more of the HtrA1 trypsin domain, PDZ domain, a serine protease (SP) domain, insulin-like growth factor biding domain (IGFBP), Kazal-type trypsin (KI) inhibitory domain, Mac25 domain, or the follistatin (FS) domain. In another such embodiment, an antibody according to the present invention inhibits the activity or activation of one or more functions of HtrA1, as described herein, in a manner that inhibits pathologic neovascularization. In one such embodiment, the HtrA1 antibody as described herein inhibits pathologic choroidal neovascularization. In another such embodiment, the HtrA1 antibody inhibits pathologic retinal neovascularization. In another embodiment, an HtrA1 antibody as described herein inhibits choroidal neovascularization in a laser-induced choroidal neovascularization model, wherein the reduction in choroidal neovascularization is selected from at least about 30%, about 40%, about 50%, about 60%, or about 70% or greater relative to a control. In yet another embodiment, an HtrA1 antibody as described herein inhibits retinal neovascularization in an oxygen-induced retinal neovascularization model, wherein the reduction in retinal neovascularization is selected from at least about 30%, about 40%, about 50%, about 60%, or about 70% or greater relative to a control.

Antibodies to HtrA1 that inhibit neovascularization as described herein may, for example, be isolated monoclonal antibodies, polyclonal antibodies, chimeric antibodies, humanized antibodies, human antibodies, and affinity matured antibodies to HtrA1, isolated monoclonal antibodies, polyclonal antibodies, chimeric antibodies, humanized antibodies, human antibodies, and affinity matured antibodies generated using an immunizing agent that includes a fragment of human HtrA1 according to SEQ. ID. NO.: 1, the fragment comprising amino acids 141-480 of a full length human HtrA1 molecule, or a variant of such a fragment, isolated monoclonal antibodies, polyclonal antibodies, chimeric antibodies, humanized antibodies, human antibodies, and affinity matured antibodies generated using an immunizing agent that includes a full-length human HtrA1 molecule according to SEQ. ID. NO.: 2 or a variant thereof. In particular embodiments of such antibodies, the antibodies exhibit a titer and/or binding affinity according to the titers, ranges of titers, binding affinities and ranges of binding affinities described herein.

### Methods of Treating Ocular Disease or Pathologies

The biologically active agents (e.g., compounds, antibodies, nucleic acids, including, for example RNAi compounds, ribozymes, etc.) are useful for treating, for example, ocular disease and pathologic conditions of the eye. In each such embodiment, a therapeutically effective amount of the biologically active agent is administered to the subject. In one such embodiment, the pathological condition of the eye or ocular disease is selected from one of the diseases and conditions described herein. In another embodiment, therapeutically effective amounts of two or more biologically active agents are administered to a subject. In one such embodiment, at least one biologically active agent capable of inhibiting the activity of HtrA1 and at least biologically active agent capable of inhibiting the activity of CFH are administered to the subject. In addition to administering a therapeutic amount of one or more biologically active agents, a therapeutic method according to the present description may further include administration of one or more additional pharmaceutically active agents.

In one embodiment, the pathologic condition to be treated is neovascularization induced or facilitated by the presence or expression of HtrA1. In another embodiment, the pathologic condition is selected from retinal vein occlusion, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, and juxtafoveal telangiectas. In another embodiment, the ocular disease or pathologic condition is selected from AMD in its wet form, AMD in its dry form, DR, ROP, macular edema, ischemia-induced neovascularization, and a pathologic condition associated with vascular leak or edema in the eye. In one such embodiment, the biologically active agent is selected from biologically active antibodies, nucleic acids and ribozymes according to the present description, and any combination thereof, wherein such biologically active agent inhibits or blocks one or more activities of HtrA1, of CHF, or of both HtrA1 and CFH as described herein. In yet another such embodiment, the biologically active agent is selected from a molecule that inhibits or blocks HtrA1 protein binding to TGF-ß, a molecule that stimulates TGF-ß, biologically active small molecules, and any combination thereof, wherein such biologically active agent inhibits or blocks one or more activities of HtrA1 as described herein. In yet another such embodiment, the biologically active agent is selected from a molecule that inhibits or blocks CFH protein activity.

In particular embodiments, the HtrA1 antibodies described herein may be used in methods for treating one or more pathologic conditions in a subject. In each such embodiment, a therapeutically effective amount of HtrA1 antibody is administered to the subject. In another such embodiment, therapeutically effective amounts of two or more HtrA1 antibodies are administered to a subject. In addition to administering a therapeutic amount of one or more HtrA1 antibodies, a therapeutic method according to the present description may further include administration of one or more additional pharmaceutically active agents. Such agents may be chosen from, for example, from the biologically active agents disclosed herein or one or more additional agents that modulate the activity of HtrA1, work to inhibit neovascularization, or provide another other desired therapeutic benefit. In one embodiment, the pathologic condition to be treated is neovascularization induced or facilitated by the presence or expression of HtrA1. In another embodiment, the pathologic condition is selected from AMD in its wet form, AMD in its dry form, DR, ROP, macular edema, ischemia-induced neovascularization, and a pathologic condition associated with vascular leak or edema in the eye.

In addition, both *in*-*vivo* and *in-vitro* models can be used to further explore the therapeutic effects of HtrA1 antibodies according to the present description and to understand the role of the such antibodies in the development and pathogenesis of, for instance, diseases or pathologies associated with neovascularization, wet and dry forms of AMD, DR, ROP, macular edema, ischemia-induced neovascularization, vascular leak or edema in the eye

For example, as detailed in Examples 6-10, relative to a control, HtrA1 antibodies as described herein significantly reduce neovascularization in animal models of laser-induced choroidal neovascularization and oxygen-induced retinopathy (*i*.*e*., oxygen-induced retinal neovascularization). Animal models of choroidal neovascularization and oxygen-induced retinopathy are known, are accepted models of eye diseases associate with neovascularization, and the antibodies described herein are suitable for use in such models. Examples of the application of such models can be seen in, for instance, Saishin et al., J Cell Physiol., 195(2):241-8 (2003); Dejneka et al., Mol Vis., 10:964-72 (2004); and Nambu et al., Invest Ophthalmol Vis Sci., 44(8):3650-5 (2003); Smith et al., Invest Opthalmol Vis Sci, 35(1): 101-11. Additional references to animal models that may be suited for evaluation of the HtrA1 antibodies described herein include, for example, Fu et al., Hum Mol Genet, 15:16(20):3411-22 (2007); Karan et al., Proc Natl Acad Sci USA, 15;102(11):4164-9 (2005); Kim et al., Invest Ophthalmol Vis Sci, 48(10):4407-20 (2007); and Tanaka et al., Mol Ther, 13(3):609-16 (2006).

### Pharmaceutical Formulations

The biologically active agents described herein may be administered to a subject using a suitable carrier, and in one embodiment, the carrier is a pharmaceutically-acceptable carrier. Carriers that may be used to deliver the biologically active agents described herein, such as, for example, the biologically active antibodies and nucleic acids, and the formulations of such carriers are described, for example, in Remington's Pharmaceutical Sciences, 16th ed., 1980, Mack Publishing Co., edited by Oslo et al. In addition to a carrier, a pharmaceutically acceptable formulation for delivery of biologically active agent as described herein may include, for example, a desired amount a pharmaceutically-acceptable salt or other pharmaceutically acceptable tonicity modifier in order to render the formulation isotonic. Examples of carriers that may be used to provide a pharmaceutical formulation contemplated herein include, but are not limited to, saline, Ringer's solution and dextrose solution. A pharmaceutical formulation of as described herein may exhibit a pH of from about 5 to about 8, and in one such embodiment exhibits a pH of from about 7 to about 7.5. Where desired, a pharmaceutical formulation according to the present description may include a buffer to achieve and maintain a desired pH. Pharmaceutically acceptable buffers useful for achieving a formulation exhibiting a pH suitable for delivery to a subject are well known in the art. Even further, a pharmaceutical formulation as described herein may include a carrier formulated as a sustained release preparation. Sustained release preparations may be formed by, for example, semipermeable polymer matrices containing the antibody, and such matrices may be formed into articles, such as films, particles, or depots, of a desired shape, size or configuration.

The nature of the carrier, formulation constituents, and even configuration of a pharmaceutical product to be delivered may be adjusted or altered depending upon, for instance, the route of administration and nature and concentration of biologically active agent being administered. For example, where the subject to be treated is a mammal, a pharmaceutical formulation as described herein can be formulated for delivery by injection (e.g., intravenous, intraocular, intraperitoneal, subcutaneous, intramuscular, or intraportal injection), or by other methods such as infusion that ensure delivery of the biologically active agent to the bloodstream in a therapeutically effective form.

Therapeutically effective dosages and schedules for administering one or more biologically active agents as described herein may be determined based on the agent to be administered, the desired therapeutic effect, the route of administration, and the subject to which the antibody is to be administered. Guidance in selecting appropriate doses for a biologically active antibody as described herein may be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. Where an antibody is used, a typical daily dosage of the antibody used alone might range, for example, from about 1 µg/kg to up to 100 mg/kg of body weight or more per day, depending on the factors mentioned above. In specific embodiments, a biologically active agent as described herein may be administered sequentially or concurrently with the one or more other biologically active agents.

In a further embodiment of the invention, there are provided articles of manufacture and kits containing materials useful for diagnosing and/or treating ocular disease and pathological conditions as described herein. In one such embodiment, the article of manufacture comprises a container having a label. Suitable containers include, for example, bottles, vials, and test tubes. In one embodiment, the container may be formed from a variety of materials such as glass or plastic, and holds a composition having an biologically active agent which is effective for treating ocular disease or pathological conditions. In one embodiment, the active agent in the composition includes one or more HtrA1 antibodies, serine protease inhibitors, nucleic acids, such as RNAi compounds, a molecule that inhibits or blocks HtrA1 protein binding to TGF-ß, a molecule that stimulates TGF-ß, biologically active small molecules, and any combination thereof as described herein. The label on the container may, for example, indicate that the composition included in the container is used for treating one or more diseases or pathological conditions. In addition, in one embodiment, the label may also indicate directions for either *in*-*vivo* or *in-vitro* use, such as for the uses described above. In another embodiment, the container may be formed from a variety of materials such as glass or plastic, and holds a composition having an agent which is effective detecting or purifying HtrA1 polypeptide. In another embodiment, the article of manufacture is a kit that includes a container having a label as already described and, optionally, a second container comprising one or more carriers, diluents or constituents, such as, for example, a buffer or tonicity modifier. In one embodiment, such a kit further includes one or more other materials desirable from the end user's standpoint, including, for example, filters, needles, syringes, and package inserts with instructions for use.

The Examples that follow are offered for illustrative purposes only, and are not intended to limit the scope of the present invention in any way. In each instance, unless otherwise specified, standard materials and methods were used in carrying out the work described in the Examples provided.

All patent and literature references cited in the present specification are hereby incorporated by reference in their entirety.

The practice of the present invention employs, unless otherwise indicated, conventional techniques of chemistry, molecular biology, microbiology, recombinant DNA, genetics, immunology, cell biology, cell culture and transgenic biology, which are within the skill of the art (See, e.g., Maniatis, T., et al. (1982) Molecular Cloning: A Laboratory Manual (Cold Spring Harbor Laboratory, Cold Spring Harbor, N.Y.); Sambrook, J., et al. (1989) Molecular Cloning: A Laboratory Manual, 2nd Ed. (Cold Spring Harbor Laboratory, Cold Spring Harbor, N.Y.); Ausubel, F. M., et al. (1992) Current Protocols in Molecular Biology, (J. Wiley and Sons, NY); Glover, D. (1985) DNA Cloning, I and II (Oxford Press); Anand, R. (1992) Techniques for the Analysis of Complex Genomes, (Academic Press); Guthrie, G. and Fink, G. R. (1991) Guide to Yeast Genetics and Molecular Biology (Academic Press); Harlow and Lane (1988) Antibodies: A Laboratory Manual (Cold Spring Harbor Laboratory, Cold Spring Harbor, N.Y.; Jakoby, W. B. and Pastan, I. H. (eds.) (1979) Cell Culture. Methods in Enzymology, Vol. 58 (Academic Press, Inc., Harcourt Brace Jovanovich (NY); Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Hogan et al. (eds) (1994) Manipulating the Mouse Embryo. A Laboratory Manual, 2.sup.nd Edition, Cold Spring Harbor Laboratory Press, Cold Spring Harbor, N.Y. A general discussion of techniques and materials for human gene mapping, including mapping of human chromosome 1, is provided, e.g., in White and Lalouel (1988) Ann. Rev. Genet. 22:259 279. The practice of the present invention employs, unless otherwise indicated, conventional techniques of chemistry, molecular biology, microbiology, recombinant DNA, genetics, and immunology. See, e.g., Maniatis et al., 1982; Sambrook et al., 1989; Ausubel et al., 1992; Glover, 1985; Anand, 1992; Guthrie and Fink, 1991).

### EXAMPLE 1 - Expression of HtrA1 and AMD

As described herein, the SNP rs11200638 is located 512 bp upstream of the transcription start site of the HtrA1 gene (also known as *PRSS11, NM*_*002775*). Using Matlnspector to scan putative transcription factor binding sites within this region, we identified a conserved AP2/SRF binding element that is altered by the A risk allele. To investigate the functional significance of the SNP, a real-time RT-PCR was conducted to study the expression levels of HtrA1 mRNA in lymphocytes of four AMD subjects carrying the risk allele AA and three normal controls carrying the normal allele GG. The results of this study are shown in FIG. 3. The HtrA1 mRNA levels in lymphocytes from AMD subjects with the AA genotype were approximately 2.7 fold higher than those in normal controls with the GG genotype.

The mean HtrA1 protein level in RPE of four AMD donor eyes with a homozygous AA risk allele was also analyzed. As shown in FIG. 4, HtrA1 expression level in the AA genotype was 1.7 fold higher compared to that of six normal controls with a homozygous GG allele.

### EXAMPLE 2 - Production of recombinant His-tagged HtrA1 protein

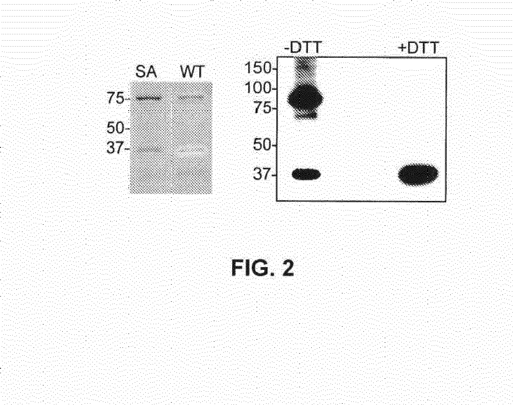

To raise antibodies against HtrA1, His-tagged wild type and His-tagged mutant protease (with a point mutation of Ser to Ala (SA) in the trypsin protease domain) proteins were produced using a bacterial expression system and Ni-NTA affinity purification. In order to produce the His-tagged wild type and His-tagged mutant protease proteins, a fragment encoding amino acids 141-480 (SEQ. ID. NO. 1) of the human HtrA1 protein was amplified from cDNA library. The fragment was amplified with the following PCR primers, HtrA1-L (5'-acgcgtcgacaaaggctgcaccg gccgccggt-3') (SEQ. ID. NO.: 3) and HtrA1-R (5'-ataagaatgcggccgcctatgggtcaatttctt cgg-3') (SEQ. ID. NO.: 4). Using standard techniques, the PCR product was cloned into a pET32a plasmid vector (Novagen) and them digested with *Sal* I and Not I restriction enzymes. The recombinant His-tagged HtrA1 construct was expressed in BL21 bacteria following the IPTG induction. The recombinant proteins were purified from bacterial lysate using a Ni-column (Qiagen) and confirmed by western blot. Purification of HtrA1 proteins indicated that both mutant HtrA1 (SA) and wild type HtrA1 (WT) produced 75 kD dimer and 37 kD monomer (FIG. 2). Moreover, zymography analysis also indicates that the SA mutant does not possess protease activity. In addition, wild type dimer was less active than monomer. Dimer formation of purified HtrA1 proteins was sensitive to reducing condition, indicating that disulfide linkage may be responsible for dimer configuration. The recombinant wild type HtrA1 protein produced according to this example was used to generate rabbit polyclonal antibodies and murine monoclonal antibodies.

### EXAMPLE 3 - Generating polyclonal antibody that recognizes HtrA1

Using the recombinant wild type HtrA1 protein of Example 1, rabbit antibodies were produced using standard methods. Western blot analysis was conducted according to the procedure described in Example 11 and confirmed that the polyclonal antibody produced specifically recognized the immunogen, and immunofluorescence conducted by the process described in Example 10 below showed that the polyclonal antibody recognized HtrA1 expressed in mammalian cells lines. Of significance, it was determined that in murine models of oxygen induced retinopathy, the polyclonal HtrA1 antibody was highly expressed in pathologic retinal vessels when compared to normal blood vessels.

With reference to FIG. 5(A), the HtrA1 polyclonal antibody was verified by Western blot. HtrA1 polyclonal antibody can recognize recombinant HtrA1 (a) and HtrA1 in human RPE (c and d). Specificity was confirmed by lack of signal following preabsorption with recombinant HtrA1 protein (e and f), (b) was a negative control consisting of a recombinant protein preparation from bacteria transfected with vector only. FIG. 5(B) shows HtrA1 polyclonal antibody stained on mammalian cells expressing recombinant human HtrA1. In FIG. 5(C-E), HtrA1 polyclonal antibody reveals HtrA1 expression in pathologic retinal endothelial cells but not normal retinal vessels in murine model of oxygen-induced retinopathy. In the normal mouse retina there is no blood vessel beyond internal limiting membrane, but in oxygen-induced retinopathy mouse model, proliferative blood vessel grows beyond internal limiting membrane into vitreous cavity (*i.e*., retinal neovascularization). FIG. 5(E) shows that HtrA1 polyclonal antibody stains pathologic endothelial cells located beyond internal limiting membrane (yellow/bottom arrows) but not intraretinal endothelial cells (white/top and middle arrows). In FIG. 5(C), isolectin staining shows blood vessels, and FIG. 5 (D) shows HtrA1 polyclonal antibody staining. FIG. 5(E) merges the images of 5(C) and 5(D) with TO-PRO3 staining to show nuclei (blue). As used in FIG. 5 (C-E), "ONL" refers to the outer nuclear layer, "INL" refers to the inner nuclear layer, "GCL" refers to the ganglion cell layer, and "ILM" refers to the internal limiting membrane.

In addition, immunohistochemistry studies of HtrA1 expression in human eyes from AMD donors were conducted. Retinal sections were taken and incubated at 37°C for 30 min. The retinal sections were then washed 3 times with PBS, and non-specific binding sites were blocked with 10% goat serum in PBS for 1 hr at room temperature. HtrA1 polyclonal antibody and biotin conjugated isolectin B4 (Vector, Burlingame, CA) diluted in PBS with 10% goat serum were applied to the sections at 4°C overnight. After washing 3 times with PBS, the retinal sections were incubated for 1 hr at room temperature with FITC conjugated goat anti mouse IgG (Jackson Immunoresearch, West Grove, PA), Texas red conjugated streptavidin (Vector, Burlingame, CA), and TO-PRO3 iodide (Molecular Probes, Carlsbad, CA). Finally, the sections were washed 3 times with PBS and mounted with Vectashield mounting media (Vector laboratories, Inc., Burlingame, CA) for microscopy. Immunolabeling was visualized using a Zeiss LSM 510 laser scanning confocal microscope (Zeiss, Thornwood, NY).

The results of the immunohistochemistry studies are shown in FIG. 6. With reference to FIG. 6(A), it was found that HtrA1 is nonuniformly expressed on drusen, the hallmark of dry AMD, and in the Bruch's membrane. In addition, as shown in FIG. 6(B), HtrA1 is not only expressed on drusen, but also on retinal blood vessels and internal limiting membrane. As is shown in FIG. 6(C), in a wet AMD donor eye, HtrA1 is expressed on choroidal vessels (white/top arrows) and in choroidal neovascularization (yellow/bottom arrows). Green channel is the staining of HtrA1 polyclonal antibody. Nuclei were counter-stained with propidium iodine (red). Scale bar shown in FIG. 6 represents a length of 20 µm.

These studies indicate that HtrA1 expression plays a role in formation of drusen and that HtrA1 is upregulated in conditions of pathologic neovascularization, potentially accelerating the invasiveness and destructive nature of the new vessels. EXAMPLE 4 - *Generation of hybridomas for production of monoclonal antibodies*

In this example, mouse hybridomas were created using standard techniques for the production of anti-HtrA1 monoclonal antibodies. More particularly, female BALB/c mice were immunized and boosted subcutaneously with 25-50 µg of the recombinant His-tagged HtrA1 protein of Example 1 according to the schedule described in Harlow, E and Lane, D, Antibodies: A laboratory Manual. 1988 Cold Spring Harbor, NY: Cold Spring Harbor Laboratory Press.

The lymphocyes from the spleens of immunized mice were recovered and fused to myeloma Sp2/0-Ag14 cells, at 1:5 ratio using 50% PEG, to form hybridomas. The supernatant form fused cells was a screened for antibody against HtrA1 by indirect ELISA using recombinant GST-tagged GST-tagged HtrA1. The hybridomas that were identified as producing antibodies for HtrA1 were cloned and cultured to establish a continuous cell line with stable genetic coding.

Hybridoma cells produced as disclosed herein were implanted via injection into the peritoneal cavity of mice, and monoclonal antibodies were isolated and purified from ascites. Mice peritoneal effusions were obtained and centrifugated at 9600 rpm for 20 minutes. Supernatant pH was measured and adjusted to 6.4-7.0. To the supernatant a two-fold volume of pH4.5, 0.06mol/L sodium acetate solution and caprylic acid 3.3% were added dropwise with agitation. The solution was then centrifuged at 9600rpm for 25 minutes and the supernatant was filtered and measured for pH. To the supernatant, a ten-percent volume of 0.1mol/L PBS was added. After, isovolumic saturated ammonium sulfate was slowly added dropwise, with agitation, to the supernatant. The resulting solution was kept in 4°C overnight, then centrifuged at 11000 rpm for 20min, and the precipitate was dissolved with normal saline. Precipitation and dissolution was repeated 3 times. The solution was then dialyzed with 0.005mol/L PBS for 24 hours and centrifugated with 11000 rpm for 20 min. The supernatant was collected and stored at -20°C.

### EXAMPLE 5 - ELISA to compare titers of monoclonal anti-HTRA1 antibodies

In this example, indirect ELISA was used to estimate and compare the titers of monoclonal antibodies produced and collected from a selection of hybridomas produced according to Example 4. Table 1 provides the measured titers for the HtrA1 monoclonal antibodies produced by the hybridoma cell lines listed therein. The titers provided in Table 1 represent the threshold dilutions at which the monoclonal antibodies produced by the specified hybridomas still provide an optical density in the well that was twice the optical density observed in the control, the control being provided by wells loaded with dilution buffer 1XPBS at pH7.4, 0.05% Tween 20, and 0.1% BSA.

To carry out the ELISA, the wells of a PVC microtiter plate (Corning, Corning, NY) were coated with 100 µl of 3 µg/ml recombinant HtrA1 protein diluted in 50 mM carbonate/bicarbonate buffer (pH 9.6) at 4°C overnight. After washing 4 times with 0.05% Tween 20/PBS, the wells were blocked with 10% goat serum in PBS at room temperature for 2 hrs. After blocking, 100 µl of a 1 mg/ml solution of each the HtrA1 monoclonal antibodies at dilutions ranging from 1:10,000 to 1:1,280,000 in PBS were added with 10% goat serum to each well in triplicate, and incubated at room temperature for 1 hr. After incubation, the plate was washed 4 times, and then each well was incubated with 100 µl of 1:1000 dilution of horseradish peroxidase (HRP) conjugated goat anti-mouse IgG antibody (Santa Cruz, Santa Cruz, CA) for 1 hr at room temperature. The wells were then washed 6 times and developed with 100 µl TMB substrate (BIO-RAD, Hercules, CA) for 10 min at 37° C and stopped with 100 µl of 2 M H₂SO₄. The absorbance (*i.e*., optical density) in each of the wells was read at 450nm by a Benchmark Plus microplate reader (BIO-RAD, Hercules, CA).

### EXAMPLE 6 - HtrA1 polyclonal antibodies inhibit neovascularization in a mouse

### model of oxygen-induced retinopathy

Intraocular injections of polyclonal HtrA1 antibodies significantly reduced pathologic neovascularization in an animal model of oxygen-induced retinopathy (OIR). More particularly, in a mouse model of OIR, administration of polyclonal HtrA1 antibody resulted in reduced neovascularization when compared to a contralateral control receiving a negative control pre-immune serum. The polyclonal antibody was delivered in solution by intraocular injection, wherein the concentration of antibody was 1 mg/ml and the dose volume delivered was 1µl.

Oxygen-induced retinopathy was induced in mice as described in Smith et al., Invest Opthalmol Vis Sci, 35(1): 101-11. More specifically, seven day postpartem (P7) pups along with nursing mothers were placed in 75% oxygen, maintained by a Pro-OX oxygen controller (BioSpherix, Redfield, NY). The pups were removed on P12 and given a 1µl intraocular injection of HtrA1 polyclonal antibody in one eye and a 1µl intraocular injection of the pre-immune serum in the contralateral eye. Mice were sacrificed on P17 and perfused via the left ventricle with 1 ml 50 mg/ml FITC-Dextran (Sigma, St. Louis, MO). The mice eyes were enucleated, fixed for 30 minutes in 4% paraformaldehyde, and retinal flat mounts were prepared. The blood vessels in the flat mounts were visualized with either FITC Dextran or lectin staining. The flat mounts were analyzed by Axiovert 200 fluorescence microscopy (Carl Zeiss, Thornwood, NY). Neovascularization was quantified using AxioVision software (Carl Zeiss, Thornwood, NY).

FIG. 7 shows a retinal flat mount of an eye injected with pre-immune serum as a negative control (A), a retinal flat mount of a contralateral eye injected with HtrA1 polyclonal antibody (B), and the results of a paired t test (C) showing that the area of retinal neovasculariztion in eyes injected with HtrA1 polyclonal antibody was significantly less than the area of neovascularization seen in contralateral eyes injected with the negative control.

### EXAMPLE 7 - HtrA1 monoclonal antibodies inhibit neovascularization in a mouse model of oxygen-induced retinopathy

The OIR model described in Example 6 was repeated. In this instance however, two monoclonal antibodies were administered. The monoclonal antibodies administered were 8F12 HtrA1 monoclonal antibodies and 9F7 monoclonal HtrA1 antibodies. The 8F12 and 9F7 monoclonal antibodies were those produced by the 8F12 and 9F7 hybridoma lines generated according to Example 4 and subject to ELISA analysis according to Example 5. The 8F12 and 9F7 monoclonal antibodies were each delivered by intraocular injection at a dose volume of 1µl were the concentration of antibody was 1 mg/ml.

FIG. 8 shows a retinal flat mount of an eye injected with 8F12 monoclonal antibody (A), a retinal flat mount of a contralateral eye injected with 9F7 monoclonal antibody (B), and the results of a paired t test (C) showing that the area of retinal neovasculariztion in eyes injected with 8F12 monoclonal antibody is significantly less than the area of neovascularization seen in contralateral eyes injected with 9F7 monoclonal antibody. These results indicate that monoclonal antibodies exhibiting relatively higher titers as evaluated according to Example 5, may provide relatively higher reduction in retinal neovascularization.

### EXAMPLE 8 - HtrA1 polyclonal antibodies inhibit laser-induced choroidal neovascularization

A mouse method for assessing choroidal neovascularization (CNV) was developed. Adult mice (2-3 months old) were subjected to laser-induced disruption of Bruch's. Before doing so, a general anesthetic was introduced in the mice via intraperitoneal injection of a mixture of ketamine hydrochloride and xylazine hydrochloride. Pupils were dilated with 1% tropicamide for photocoagulation. An Iridex OcuLight GL 532 nm laser photocoagulator (Iridex, Mountain View, CA) with slit lamp delivery system was used to disrupt Bruch's membrane at 3 spots at posterior pole of retina with the following parameters: 150mW power, 75um spot size, and 0.1 seconds duration. Production of a bubble at the time of laser treatment, which indicates rupture of Bruch's membrane, was an important factor in obtaining CNV, so only burns in which a bubble produced were included in the study. Immediately after laser treatment and 3 days later, the mice received an intraocular injection of 1µl of a 1 mg/ml solution of the HtrA1 polyclonal antibody in one eye and an intraocular injection of 1µl of a negative control pre-immune serum in the contralateral eye. One week later, the mice were sacrificed and choroidal flat mounts were prepared after fixation. Biotin conjugated isolectin (Sigma, St. Louis, MO) and Texas red conjugated streptavidin (Sigma, St. Louis, MO) were used to stain blood vessels. The flat mounts prepared in this manner were examined by Zeiss LSM 510 confocal microscope (Zeiss, Thornwood, NY) and the area of CNV was measured and quantified by ImageJ (NIH, Bethesda, MD) software.

FIG. 9 shows a choroidal flat mount of an eye injected with pre-immune serum as a negative control (A), a choroidal flat mount of a contralateral eye injected with HtrA1 polyclonal antibody (B), and the results of a paired t test (C) showing that the area of choroidal neovasculariztion in eyes injected with HtrA1 polyclonal antibody is significantly less than the area of neovascularization seen in eyes injected with the negative control.

### EXAMPLE 9 - HtrA1 monoclonal antibodies inhibit laser-induced choroidal neovascularization

The CNV model described in Example 8 was repeated. In this instance however, two monoclonal antibodies were administered. The monoclonal antibodies administered were 8F12 HtrA1 monoclonal antibodies and 9F7 monoclonal HtrA1 antibodies. The 8F12 and 9F7 monoclonal antibodies were those produced by the 8F12 and 9F7 hybridoma lines generated according to Example 4 and subject to ELISA analysis according to Example 5. The 8F12 and 9F7 monoclonal antibodies were each delivered by intraocular injection at a dose volume of 1µl where the concentration of antibody was 1 mg/ml.

FIG. 10 shows a choroidal flat mount of an eye injected with 9F7 monoclonal antibody (A), a choroidal flat mount of a contralateral eye injected with 8F12 monoclonal antibody (B), and the results of a paired t test (C) showing that the area of choroidal neovasculariztion in eyes injected with 8F12 monoclonal antibody is significantly less than the area of neovascularization seen in contralateral eyes injected with 9F7 monoclonal antibody. These results indicate that monoclonal antibodies exhibiting relatively higher titers as evaluated according to Example 5, provide relatively higher reduction in choroidal neovascularization.

The results provided by the OIR and CNV studies detailed in Examples 6-10 herein indicate that HtrA1 antibodies as provided by the present description are biologically active and work to directly or indirectly inhibit or block one or more activity of HtrA1 and, thereby, work to inhibit or block pathologic neovascularization.

### EXAMPLE 10 - Immunocytochemistry analysis of mammalian expressed HtrA1 by monoclonal antibodies

Mammalian HtrA1 was exposed to anti-HtrA1 antibodies and analyzed by immunocytochemistry. More specifically, HEK293 cells were seeded onto poly-L-lysine coated four chamber glass slides in Dulbecco's Modified Eagles Medium (DMEM) supplemented with 10% fetal bovine serum (FBS), 100 units/ml of penicillin, and 100 µg/ml of streptomycin. The cells were transfected with HTRA1-pcDNA 3.1 recombinant plasmids using fugen 6 (Roche) according to the manufacturer's protocol. Transfected cells were washed twice with phosphate buffer saline (PBS, pH 7.5), and fixed in methanol:acetone (50:50, V/V) for 5 min at 20°C. The fixed cells were washed 2-3 times for 5 min each with PBS, and once with 0.1% triton X-100 in PBS for 5 min. The fixed cells were washed again 2-3 time for 5 min each with PBS and the slides blocked with 5-10% goat serum for 30 min at room temperature. After incubation with HtrA1 antibody at 1:100 dilution in PBS at room temperature for 2 hrs, the cells were washed with PBS 2-3 times for 5 min each. The slides were exposed to secondary antibody conjugated with FITC for 1hour at room temperature and then washed with PBS 2-3 times for 5 min each. The slides were mounted with vectashield mounting media (Vector laboratories, Inc., Burlingame, CA) for microscopy.

### EXAMPLE 11 - Western blot analysis of HTRA1 monoclonal antibodies

Retinal pigment epithelium (RPE) samples were homogenized according to the method of Laemmli. Approximately 7 ug of protein lysate were resolved with 9% SDS PAGE and transferred to a PVDF membrane (Millipore, Billerica, MA). The non-specific binding sites on the blot were blocked for 2 h at room temperature with 5% skim milk in NaCl/Tris buffer containing 0.05% Tween 20 (TTBS). The membrane was incubated overnight at 4°C with anti-HtrA1 antibody at 1:2000 dilution in TTBS containing 5% milk, and then washed three times with TTBS for 5 min each time. Anti mouse antibody conjugated with HRP (Amersham Biosciences, NJ), 1:4000 dilution in TTBS, was applied to the membrane for 1 hour at room temperature, and the membrane was then washed twice for 5 min each time with TTBS, and once for 5 min with NaCl/Tris buffer. The membrane was developed with an ECL (Amersham Bioscience, NJ) detection kit.

### EXAMPLE 12 - Correlation of SNP rs11200638 to AMD

**Subjects.** This study was approved by the University of Utah Institutional Review Board. All subjects provided informed consent prior to participation in the study. AMD subjects were recruited at the Moran Eye Center (University of Utah), as were normal age-matched controls (subjects age 60 years or older with no drusen or RPE changes). Grading was carried out according to the standard grid classification system suggested by the International ARM Epidemiological Study Group for age-related maculopathy (ARM) and AMD (A. C. Bird *et at., Surv Ophthalmol 39*, 367 (1995)). All abnormalities in the macula were characterized according to 1) type, size, and number of drusen, 2) RPE hyperpigmentation or hypopigmentation, and 3) advanced AMD stages including geographic atrophy (GA, dry AMD), and choroidal neovascularization (CNV, wet AMD).

**Genotyping.** The initial Utah cohort of 442 caucasion AMD subjects including 265 wet AMD, and 177 soft confluent drusen was genotyped and allele frequencies were compared to 309 age and ethnicity matched normal controls. The expanded sample for second stage genotyping of rs10490924 and rs11200638 included 581 AMD subjects (392 wet AMD, 189 soft confluent drusen). For the rs11200638 genotype, the inventors PCR-amplified genomic DNA extracted from AMD and control subject blood samples. Oligonucleotide primers, forward 5'-ATGCCACCCACAACAACTTT-3' and reverse, 5'-CGCGTCCTTCAAACTAATGG-3' were used in PCR reactions containing 5% DMSO. DNA was denatured at 95°C-3 minutes, followed by 35 cycles, 94°C-30 seconds, 52°C-30 seconds, and 72°C-45 seconds. The PCR product was digested with *Eag* / to identify the G allele. For rs10490924, forward primer 5'-TACCCAGGACCGATGGTAAC-3' and reverse primer 5'-GAGGAAGGCTGAATTGCCTA-3' were used for PCR amplification, PVUII digestion, and identification of the G allele. The remaining 13 SNPs were genotyped using the SNaPshot method on an ABI 3130 genetic analyzer (Applied Biosystems, Foster City, CA) according to the manufacturer's instructions. CFH genotyping was performed according to published methods. (A. O. Edwards et al., Science 308, 421 (Apr 15, 2005).

**Data Analysis.** The chi-squared test for trend for the additive model over alleles was performed to assess evidence for association. Odds Ratios and 95% confidence intervals were also calculated to estimate risk size for the heterozygotes and homozygotes for the risk alleles.

**Two Locus Analyses.** Two-locus analyses were performed for the CFH Y402H variant at 1q31 and rs11200638 for 10q26. A global two locus (9x2) contingency table, enumerating all 9 two-locus genotype combinations was constructed. Odds ratios and 95% confidence intervals, comparing each genotypic combination to the baseline of homozygosity for the common allele at both loci. For the risk genotypes identified, the inventors calculated population attributable risks (PAR) using the Levin formula. (M. L. Levin, Acta *Unio Mt Contra Cancrum **9**,* 531 (1953).

### EXAMPLE 13 - HtrA1 Immunohistochemistry

AMD donor eyes were obtained from Utah Lions Eye Bank. Cryosections from paraformaldehyde-fixed eyes were incubated in 0.3% H₂O₂ in methanol to quench endogenous peroxidase activity. Immunohistochemistry was performed using 5pg/ml 5 monospecific anti-human Htral polyclonal antibody (ABGENT, San Diego, CA) which detects a single band on western blot (data not shown). The VectorStain Elite ABC kit (Vector Laboratories, Burlingame, CA) and the VIP peroxidase substrate (Vector Laboratories) were used for Hirai detection and immunolabeling was captured using Nomarski optics on a Nikon Eclipse 80i microscope. The results of this analysis are illustrated in FIG. 12.

### EXAMPLE 14 - Semiquantitative RT-PCR

A commercial real time PCR system (Opticon; MJ Research, Watertown, MA) was used for quantifying HtrA1 transcript levels from subject blood samples. Total RNA was extracted (RNeasy; Qiagen, Valencia, CA) and RT-PCR performed using the QuantiTect SYBR Green RT-PCR kit (Qiagen). HtrA1 primers (HtrA1 forward 5'-AACACCTACGCCAACCTGTG-3' (exon 2) and HtrA1 reverse 5'-GCAAACTGTTGGGATCTTCC-3' (exon 3)) and 100 nanograms (ng) total RNA from each sample were used for one step RT-PCR reactions. Standard curves were generated from 0 to 400 ng total RNA from subject samples. GAPDH was amplified in parallel reactions and used to normalize HrA1 values. HtrA1 and GAPDH standard curves showed similar amplification kinetics. Three to four subjects for GG and AA genotypes were assayed in duplicate reactions and duplicate runs. Results are presented as percent increase in HtrA1 RNA levels for AA samples relative to GG samples (Fig. 12 B).

The following numbered clauses contain further statements of various aspects of the present invention:
1. An antibody to HtrA1, wherein the antibody exhibits a binding affinity to HtrA1 selected from one of 10⁵ M⁻¹ and a range selected from 10⁶ M⁻¹ to 10⁷ M⁻¹, 10⁸ M⁻¹ to 10¹² M⁻¹, and 10⁹ M⁻¹ to 10¹² M⁻¹.
2. The antibody of clause 1, wherein the antibody is a selected from a polyclonal, monoclonal, humanized, human, mouse, and affinity matured antibody.
3. The antibody of clause 2, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof.
4. The antibody of clause 3, wherein the antibody exhibits a titer of 1:160,000 or greater.
5. The antibody of clause 3, wherein the antibody exhibits a titer of selected from a titer of 1:200,000 or greater, 1:300,00 or greater, 1:400,000 or greater, 1:500,000 or greater, 1:600,000 or greater, 1:700,000 or greater, 1:800,000 or greater, 1:900,000 or greater, and 1:1,000,000 or greater.
6. An antibody to HtrA1, wherein the antibody inhibits neovascularization selected from HtrA1-induced neovascularization and ischemia-induced neovascularization.
7. An antibody to HtrA1, wherein the antibody inhibits neovascularization in an animal model selected from an animal model of oxygen-induced retinopathy and an animal model of laser-induced choroidal neovascularization.
8. A method of treating an ocular disease selected from AMD, DR, ROP, and macular edema in a subject, the method comprising:
   administering a therapeutically effective amount of an antibody to HtrA1 to the subject, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof.
9. The method of clause 8, wherein administering the antibody of HtrA1 comprises administering an antibody selected from a polyclonal, monoclonal, humanized, human, mouse, and affinity matured antibody.
10. A method of treating an ischemia induced neovascularization in a subject, the method comprising:
   administering a therapeutically effective amount of an antibody to HtrA1 to the subject, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof.
11. The method of clause 10, wherein administering the antibody of HtrA1 comprises administering an antibody selected from a polyclonal, monoclonal, humanized, human, mouse, and affinity matured antibody.
12. The method of clause 10, wherein said pathologic neovascularization is ischemia-induced neovascularization resulting from injury.
13. The method of clause 10, wherein macular edema or vascular leak is associated with said pathologic neovascularization.
14. A method of treating AMD in its dry form in a subject, the method comprising:
   administering a therapeutically effective amount of an antibody to HtrA1 to the subject, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof.
15. The method of clause 14, wherein administering the antibody of HtrA1 comprises administering an antibody selected from a polyclonal, monoclonal, humanized, human, mouse, and affinity matured antibody.
16. A method of treating a subject suffering a disease selected from AMD in its wet form, AMD in its dry form, DR, and ROP, the method comprising:
   administering a therapeutically effective amount of an antibody to HtrA1 to the subject, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof.
17. The method of clause 16, wherein administering the antibody of HtrA1 comprises administering an antibody selected from a polyclonal, monoclonal, humanized, human, mouse, and affinity matured antibody.
18. The method of clause 16, wherein macular edema is associated with said disease.
19. A pharmaceutical formulation for the treatment of ocular disease or a pathologic condition of the eye, comprising:
   an antibody to HtrA1, wherein the immunizing agent used to generate the antibody includes an agent selected from an HtrA1 fragment according to SEQ. ID. NO.: 1, a full-length HtrA1 polypeptide according to SEQ. ID. NO.: 2, or variants thereof; and
   a pharmaceutically acceptable carrier.
20. The pharmaceutical formulation of clause 19, further comprising at least one additional formulation constituent selected from a diluent, a tonicity modifier, and a buffer.
21. A method of treating ocular disease or a pathologic condition of the eye in a subject, the method comprising:
   inhibiting at least one function of HtrA1 in said subject.
22. The method of clause 21, wherein inhibiting at least one function of HtrA1 comprises administering a therapeutically effective amount of an agent that inhibits at least one function of HtrA1.
23. The method of clause 21, wherein administering a therapeutically effective amount of an agent comprises administering a therapeutically effective amount of an antibody to HtrA1.
24. The method of clause 21, further comprising inhibiting at least one function of CFH in said subject, wherein inhibiting at least one function of CFH comprises administering a therapeutically effective amount of an agent that inhibits at least one function of CFH.
25. The method of clause 21, wherein the ocular disease or pathologic condition of the eye is selected from AMD in its wet form, AMD in its dry form, DR, ROP, macular edema, vascular leak, and ischemia induced neovascularization.
26. A method for diagnosing ocular disease or a pathologic condition of the eye in a subject comprising;
   isolating polynucleotides from at least one cell of an individual, wherein said polynucleotides comprise at least one nucleotide of an HtrA1 gene from that individuals genome;
   detecting the presence or absence of at least one SNP in the at least one nucleotide of the HtrA1 gene in said polynucleotides, wherein said at least one SNP correlates with said ocular disease; and
   identifying the presence or absence of ocular disease in said individual.
27. The method of clause 26, wherein the ocular disease is selected from AMD in both its wet and dry forms, DR, and ROP.
28. The method of clause 26, wherein the ocular disease includes a pathologic condition of the eye selected from pathologic neovascularization, ischemia-induced neovascularization, vascular leak, and edema.
29. The method of clause 26, wherein detecting the presence or absence of at least one SNP in the at least one nucleotide of the HtrA1 gene in said polynucleotides, comprises detecting the presence or absence of a SNP selected from rs11200638, rs1061170, rs10490924, rs3750848, rs3793917, rs932275, and combinations thereof.
30. The method of clause26, wherein detecting the presence or absence of at least one SNP of the HtrA1 gene in said polynucleotides, comprises contacting said polynucleotides with a polynucleotide probe that hybridizes under high stringency conditions to a nucleic acid molecule comprising SNP rs11200638.
31. The method of clause 26, further comprising:
   isolating polynucleotides from at least one cell of an individual, wherein said polynucleotides comprise a CFH gene from that individual's genome;
   detecting the presence or absence of at least one SNP of the CFH gene in said polynucleotides, wherein said at least one SNP correlates with said ocular disease; and
   identifying the presence or absence of ocular disease in said individual.
32. The method of clause 31, wherein detecting the presence or absence of at least one SNP of the CFH gene comprises detecting the presence or absence of a SNP selected from rs1061170 (Y402H variant at 1q31 chromosome locus), rs529825, rs800292, rs1061147, rs203674, rs2274700, and combinations thereof.
33. A method of detecting, in a sample obtained from a subject, a variant HtrA1 gene that is correlated with the occurrence of ocular disease, the method comprising:
   combining the sample with a polynucleotide probe that hybridizes, under stringent conditions, to a variation in the HtrA1 gene that is correlated with the ocular disease, but not to a wildtype HtrA1 gene; and
   determining whether hybridization occurs.
34. A method for determining a subject susceptibility to ocular disease or a pathologic condition of the eye in a subject, the method comprising:
   isolating polynucleotides from at least one cell of an individual, wherein said polynucleotides comprise at least one nucleotide from an HtrA1 gene from that individual's genome;
   detecting the presence or absence of at least one SNP of the HtrA1 gene in said polynucleotides, wherein said at least one SNP correlates with said ocular disease; and
   identifying the presence or absence of ocular disease in said individual.
35. The method of clause 34, wherein the ocular disease is selected from AMD in both its wet and dry forms, DR, and ROP.
36. The method of clause 34, wherein the ocular disease includes a pathologic condition of the eye selected from pathologic neovascularization, ischemia-induced neovascularization, vascular leak, and edema.
37. The method of clause34, wherein detecting the presence or absence of at least one SNP of the HtrA1 gene in said polynucleotides, comprises detecting the presence or absence of a SNP selected from rs11200638, rs1061170, rs10490924. rs3750848, rs3793917, rs932275, and combinations thereof.
38. The method of clause 34, wherein detecting the presence or absence of at least one SNP of the HtrA1 gene in said polynucleotides, comprises contacting said polynucleotides with a polynucleotide probe that hybridizes under high stringency conditions to a nucleic acid molecule comprising SNP rs11200638.
39. The method of clause 34, further comprising:
   isolating polynucleotides from at least one cell of an individual, wherein said polynucleotides comprise a CFH gene from that individual's genome;
   detecting the presence or absence of at least one SNP of the CFH gene in said polynucleotides, wherein said at least one SNP correlates with said ocular disease; and
   identifying the presence or absence of ocular disease in said individual.
40. The method of clause 34, wherein detecting the presence or absence of at least one SNP of the CFH gene comprises detecting the presence or absence of a SNP selected from rs1061170 (Y402H variant at 1q31 chromosome locus), rs529825, rs800292, rs1061147, rs203674, rs2274700, and combinations thereof.

## Claims

1. An agent capable of blocking or inhibiting at least one function of HtrA1 for use in a method of treating ocular disease in a subject, wherein said method comprises administering to said subject a therapeutically effective amount of said agent,
wherein said agent is selected from the group consisting of antibodies, serine protease inhibitors, nucleic acid molecules, RNAi compounds, molecules that inhibit or block HtrA1 protein binding to TGF-β and a molecule that stimulates TGF-β; and
wherein said ocular disease is selected from the group consisting of retinopathy of prematurity, retinal vein occlusion, diabetic retinopathy, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, juxtafoveal telangiectas and diabetic retinopathy.

2. The agent for use according to claim 1, wherein said agent is an antibody.

3. The agent for use according to claim 2, wherein said antibody has been generated using an immunizing agent selected from an HtrA1 fragment according to SEQ ID NO. 1, a full-length HtrA1 polypeptide according to SEQ ID NO. 2 or a variant thereof.

4. The agent for use according to claim 2 or claim 3, wherein said antibody of HtrA1 comprises an antibody selected from a polyclonal, monoclonal, humanized, human, mouse, and affinity matured antibody.

5. The agent for use according to any one of the preceding claims, wherein said method further comprises administering a therapeutically effective amount of an agent that inhibits at least one function of complement factor H (CFH).

6. An antibody to HtrA1 for use in a method of treating an ocular disease,
wherein said ocular disease is selected from the group consisting of retinopathy of prematurity, retinal vein occlusion, diabetic retinopathy, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, juxtafoveal telangiectas and diabetic retinopathy.

7. The antibody for use according to claim 6, wherein said antibody has been generated using an immunizing agent selected from an HtrA1 fragment according to SEQ ID NO. 1, a full-length HtrA1 polypeptide according to SEQ ID NO. 2 or a variant thereof.

8. The antibody for use according to claim 6 or claim 7, wherein said antibody of HtrA1 comprises an antibody selected from a polyclonal, monoclonal, humanized, human, mouse, and affinity matured antibody.

9. The antibody for use according to any one of claims 6 to 8, wherein the method comprises inhibiting at least one function of HtrA1 in said subject.

10. A method of diagnosing an ocular disease in a subject or diagnosing an increased risk of a subject developing an ocular disease, the method comprising isolating polynucleotides from at least one cell previously obtained from said subject,; detecting the presence or absence of at least one SNP in said polynucleotides, wherein said at least one SNP correlates with said ocular disease; and identifying the presence or absence of said ocular disease in said subject or the increased risk of said subject developing said ocular disease;
wherein said ocular disease is selected from the group consisting of retinopathy of prematurity, retinal vein occlusion, diabetic retinopathy, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, juxtafoveal telangiectas and diabetic retinopathy.

11. The method according to claim 10, wherein detecting the presence or absence of said at least one SNP in said polynucleotides, comprises detecting the presence or absence of a SNP selected from rs11200638, rs1061170, rs10490924, rs3750848, rs3793917, rs932275, and combinations thereof.

12. The method according to claim 11, wherein detecting the presence or absence of said at least one SNP in said polynucleotides, further comprises contacting said polynucleotides with a polynucleotide probe that hybridizes under high stringency conditions to a nucleic acid molecule comprising SNP rs11200638.

13. The method according to claim 10, wherein detecting the presence or absence of said at least one SNP in said polynucleotides, comprises detecting the presence or absence of a SNP selected from rs1061170 (Y402H variant at 1q31 chromosome locus), rs529825, rs800292, rs1061147, rs203674, rs2274700, and combinations thereof.

14. A variant of the HtrA1 gene for use in a method of diagnosing an ocular disease in a subject or a method of diagnosing an increased risk of a subject developing an ocular disease, wherein the variant of the HtrA1 gene comprises at least one SNP rs11200638, rs1061170, rs10490924, rs3750848, rs3793917, rs932275, and combinations thereof;
wherein said ocular disease or the increased risk of developing said ocular disease is diagnosed in said subject by detecting the presence of said at least one SNP in said subject; and
wherein said ocular disease is selected from the group consisting of retinopathy of prematurity, retinal vein occlusion, diabetic retinopathy, preretinal hemorrhage, flame-shaped hemorrhage, subretinal hemorrhage, hard exudates, central retinal vein occlusion, optociliary shunt, inferior hemicentral retinal vein occlusion, branch retinal vein occlusion, central retinal artery occlusion, sickle cell proliferative retinopathy, preretinal hemorrhage, dot and blot hemorrhage, cotton-wool spots, hollenhorst plaque, central retinal vein occlusion, superior hemicentral retinal occlusion, branch retinal vein occlusion, chronic BRVO, retinal artery macroaneurism, juxtafoveal telangiectas and diabetic retinopathy.

15. The variant of the HtrA1 gene for use according to claim 14, wherein detecting the presence of said at least one SNP in said subject comprises collecting a genetic sample from said subject and genotyping the genetic sample for the presence of said at least one SNP, wherein the presence of said at least one SNP in the sample correlates with said ocular disease or the increased risk in developing said ocular disease in said subject.

16. The variant of the HtrA1 gene for use according to claim 15, wherein detecting the presence of said at least one SNP in said subject further comprises contacting the genetic sample with a polynucleotide probe that hybridises under high stringency to a nucleic acid molecule comprising at least one of SNP rs11200638, rs1061170, rs10490924, rs3750848, rs3793917, rs932275, and combinations thereof.


SEQUENCE LISTING

<110>  University of Utah Research Foundation

<120>  METHOD OF DETECTING OCULAR DISEASES AND PATHOLOGIC CONDITIONS AND
       TREATMENT OF SAME

<130>  CMD/FP7082035

<140>  TBC
<141>  2007-12-21

<150>  07872362.4
<151>  2007-12-21

<150>  PCT/US2007/088661
<151>  2007-12-21

<150>  60/876,709
<151>  2006-12-22

<160>  10

<170>  PatentIn version 3.5

<210>  1
<211>  339
<212>  PRT
<213>  Homo sapiens

<400>  1

Leu His Arg Pro Pro Val Ile Val Leu Gln Arg Gly Ala Cys Gly Gln
1               5                   10                  15

Gly Gln Glu Asp Pro Asn Ser Leu Arg His Lys Tyr Asn Phe Ile Ala
            20                  25                  30

Asp Val Val Glu Lys Ile Ala Pro Ala Val Val His Ile Glu Leu Phe
        35                  40                  45

Arg Lys Leu Pro Phe Ser Lys Arg Glu Val Pro Val Ala Ser Gly Ser
    50                  55                  60

Gly Phe Ile Val Ser Glu Asp Gly Leu Ile Val Thr Asn Ala His Val
65                  70                  75                  80

Val Thr Asn Lys His Arg Val Lys Val Glu Leu Lys Asn Gly Ala Thr
                85                  90                  95

Tyr Glu Ala Lys Ile Lys Asp Val Asp Glu Lys Ala Asp Ile Ala Leu
            100                 105                 110

Ile Lys Ile Asp His Gln Gly Lys Leu Pro Val Leu Leu Leu Gly Arg
        115                 120                 125

Ser Ser Glu Leu Arg Pro Gly Glu Phe Val Val Ala Ile Gly Ser Pro
    130                 135                 140

Phe Ser Leu Gln Asn Thr Val Thr Thr Gly Ile Val Ser Thr Thr Gln
145                 150                 155                 160

Arg Gly Gly Lys Glu Leu Gly Leu Arg Asn Ser Asp Met Asp Tyr Ile
                165                 170                 175

Gln Thr Asp Ala Ile Ile Asn Tyr Gly Asn Ser Gly Gly Pro Leu Val
            180                 185                 190

Asn Leu Asp Gly Glu Val Ile Gly Ile Asn Thr Leu Lys Val Thr Ala
        195                 200                 205

Gly Ile Ser Phe Ala Ile Pro Ser Asp Lys Ile Lys Lys Phe Leu Thr
    210                 215                 220

Glu Ser His Asp Arg Gln Ala Lys Gly Lys Ala Ile Thr Lys Lys Lys
225                 230                 235                 240

Tyr Ile Gly Ile Arg Met Met Ser Leu Thr Ser Ser Lys Ala Lys Glu
                245                 250                 255

Leu Lys Asp Arg His Arg Asp Phe Pro Asp Val Ile Ser Gly Ala Tyr
            260                 265                 270

Ile Ile Glu Val Ile Pro Asp Thr Pro Ala Glu Ala Gly Gly Leu Lys
        275                 280                 285

Glu Asn Asp Val Ile Ile Ser Ile Asn Gly Gln Ser Val Val Ser Ala
    290                 295                 300

Asn Asp Val Ser Asp Val Ile Lys Arg Glu Ser Thr Leu Asn Met Val
305                 310                 315                 320

Val Arg Arg Gly Asn Glu Asp Ile Met Ile Thr Val Ile Pro Glu Glu
                325                 330                 335

Ile Asp Pro

<210>  2
<211>  480
<212>  PRT
<213>  Homo sapiens

<400>  2

<210> 3
<211> 32
<212> DNA
<213> Homo sapiens

<400>  3
acgcgtcgac aaaggctgca ccggccgccg gt                                    32


<210>  4
<211>  36
<212>  DNA
<213>  Homo sapiens

<400>  4
ataagaatgc ggccgcctat gggtcaattt cttcgg                                36


<210>  5
<211>  20
<212>  DNA
<213>  Artificial Sequence

<220>
<223>  Primer

<400>  5
atgccaccca caacaacttt                                                  20


<210>  6
<211>  20
<212>  DNA
<213>  Artificial Sequence

<220>
<223>  Primer

<400>  6
cgcgtccttc aaactaatgg                                                  20


<210>  7
<211>  20
<212>  DNA
<213>  Artificial Sequence

<220>
<223>  Primer

<400>  7
tacccaggac cgatggtaac                                                  20


<210>  8
<211>  20
<212>  DNA
<213>  Artificial Sequence

<220>
<223>  Primer

<400>  8
gaggaaggct gaattgccta                                                  20


<210>  9
<211>  20

<212>  DNA
<213>  Artificial Sequence

<220>
<223>  Primer

<400>  9
aacacctacg ccaacctgtg                                                   20


<210>  10
<211>  20
<212>  DNA
<213>  Artificial Sequence

<220>
<223>  Primer

<400>  10
gcaaactgtt gggatcttcc                                                   20